# EUROPEAN PATENT APPLICATION

(11) **EP 2 202 220 A1**
(43) Date of publication of application: **30.06.2010**
(21) Application number: 08832233.4
(22) Date of filing: 16.09.2008
(51) Int. Cl.: C07C 237/40, C07C 231/12, C07D 209/48, C08G 69/26, C08G 73/10

(54) **ACETYLENE COMPOUND, SALT THEREOF, CONDENSATE THEREOF, AND COMPOSITION THEREOF**

(30) Priority: 19.09.2007 JP 2007242585; 28.09.2007 JP 2007256373; 31.03.2008 JP 2008094262; 31.03.2008 JP 2008094269
(71) Applicant: Fujifilm Corporation, Tokyo 106-8620 (JP)
(72) Inventor: NAKAYAMA, Masaya, Odawara-shi Kanagawa 250-0001 (JP); YAGIHARA, Morio, Odawara-shi Kanagawa 250-0001 (JP); IMAKUNI, Akira, Odawara-shi Kanagawa 250-0001 (JP)
(74) Representative: HOFFMANN EITLE
(86) International application number: PCT/JP2008/066637
(87) International publication number: WO 2009/038038

(57) **Abstract**

[Problem to be Solved]

To provide an acetylene compound having a structure in which a unit having an amino group and a unit having an ethynyl group are bonded via a linking group, the acetylene compound being introducable to a polymer having thermal resistance.

[Means for Solving the Problem] An acetylene compound represented by the following Formula (1) and a salt thereof: 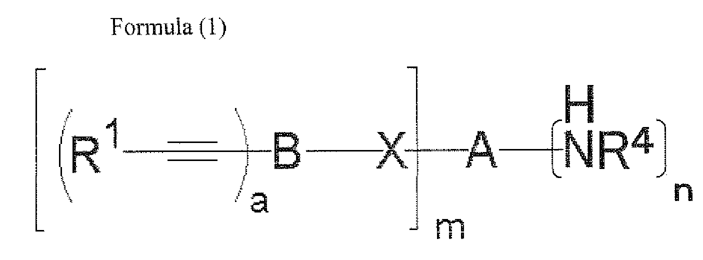 wherein in Formula (1), X represents a single bond or a divalent linking group; A represents a hydrocarbon group, a heteroaromatic ring or a heteroalicyclic compound; B represents a hydrocarbon group, a heteroaromatic ring, a heteroalicyclic compound or a single bond; R¹ represents a hydrogen atom, a hydrocarbon group, a heteroaromatic ring, a heteroalicyclic compound or a silyl group; R⁴ represents a hydrogen atom or a group that can be a substituent of an amino group; and m, n and a each independently represent an integer of 1 or greater.

## Description

### Technical Field

The present invention relates to a novel acetylene compound having one or more amino groups in the molecule thereof, the compound being usable as a raw material for functional materials such as liquid crystal materials, non-linear optical materials, electronic materials (e.g., semiconductor protection films and substrates for flexible print circuit boards), materials for adhesives, materials for lubricants, additives for photography, and materials for gas separation films; and intermediates for medicines and agricultural chemicals. The present invention also relates to a salt of the acetylene compound, a method of producing the same, a condensate of the acetylene compound, a method of producing the same, a composition including the acetylene compound, and a cured product produced by curing the acetylene compound and/or a composition including the same.

### Background Art

Aromatic compounds having an ethynyl group are usable as a raw material for functional materials such as intermediates for medicines and agricultural chemicals, liquid crystals and electronic materials. In particular, these compounds have recently attracted attention as a subject of research concerning various kinds of functional materials obtained by utilizing a carbon-carbon triple bond in the molecule. For example, an aromatic compound having an ethynyl group is used as a terminating agent that imparts thermal curability, heat resistance and antioxidization properties to a polyimide oligomer (for example, United States Patent No. 5,567,800; "Polymer" (1994), Vol. 35. pp. 4874-4880 and pp. 4857-4864; and "Kino-Zairyo (Functional Materials)" (2000), Vol. 20, No. 12, pp. 33-40).

However, as regards acetylene compounds having two or more amino groups that can be introduced in a main chain of the polymer, there are only reports on an acetylene compound having a phenyl group at an acetylene terminal thereof (for example, Japanese Patent Application Laid-Open (JP-A) Nos. 2005-320417 and 2001-056469) and an acetylene compound having a structure in which two or more aminophenol derivatives are bonded to form a ring (for example, JP-A No. 2005-272352). Moreover, there are problems in that these compounds need to be cured at high temperature, or the like.

On the other hand, no report has been made on a compound having a structure in which a unit having an amino group represented by the following Formula (1), (2) or (3) and a unit having an ethynyl group are bonded via a linking group; a polymer including this compound as a structural unit; a method of producing this polymer; a composition including this polymer; or a cured product produced by curing this composition. In particular, a compound having plural carbon-carbon triple bond structures is expected to achieve a highly efficient thermal crosslinking property.

As regards the method of synthesizing an acetylene compound having an amino croup, there are only reports on a method of synthesizing the compound by subjecting a raw material having a nitro group to condensation reaction, and then reducing the nitro group thereof; and a method of introducing an ethynyl group into the molecule of the raw material through coupling reaction at the end of synthesizing the compound. In the method of reducing a nitro group, there is a problem in that a nitrophenyl acetytlene compound, which is explosive, is formed. In the method of introducing an ethynyl group into the molecule via coupling reaction, there is a need to protect the ethynyl group when it is positioned at a terminal of the synthesized compound. Therefore, there is a problem that the intended product may hydrolyze during the deprotection reaction, thereby failing to achieve a satisfactory level of yield.

Moreover, in these methods, synthesis of the compounds is performed by isolating each compound at each process, and there has been no report on a method of performing the synthesis in a consecutive manner.

### [Disclosure of the Invention]

### [Problem to be Solved by the Invention]

The present invention provides a novel acetylene compound having one or more amino groups that can be introduced into a condensed polymer. Further, the present invention provides a salt of the acetylene compound, a method of producing the same, a condensate obtained by condensing the acetylene compound, a method of producing the same, a composition, and a cured product produced by curing the compound and/or the composition.

### [Means for Solving the Problem]

In view of the aforementioned circumstances, the present inventors have conducted intense studies, and have arrived at the present invention with the findings of a novel acetylene compound having a structure in which a unit having one amino group and a unit having one or more ethynyl groups are bonded via a linking group. Further, the present inventors have found a method of producing the aforementioned compound, the method including: protecting the amino group of a compound having one or more amino groups and one or more carboxy groups in the same molecule; converting the compound to an intermediate whose carboxylic acid moiety is highly active with respect to condensation reaction; and then reacting the intermediate with amine or alcohol having an ethynyl group.
Specifically, the following <1> to <25> are embodiments of the invention.

<1> An acetylene compound represented by the following Formula (1) and a salt thereof: 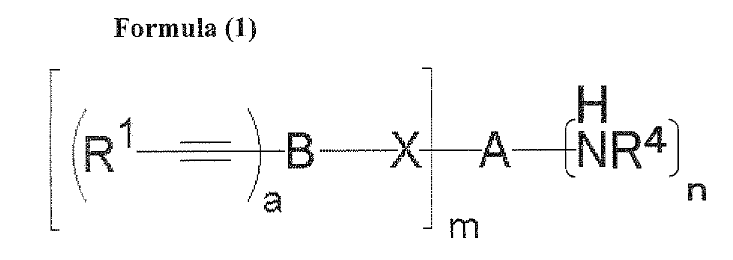 wherein in Formula (1), X represents a single bond or a divalent linking group; A represents a hydrocarbon group, a heteroaromatic ring or a heteroalicyclic compound group; B represents a hydrocarbon group, a heteroaromatic ring or a heteroalicyclic compound group or a single bond; R¹ represents a hydrogen atom, a hydrocarbon group, a heteroaromatic ring, a heteroalicyclic compound or a silyl group; R⁴ represents a hydrogen atom or a group that can be a substituent of an amino group; and m, n and a each independently represent an integer of 1 or greater.

<2> The acetylene compound and the salt thereof according to <1>, wherein in Formula (1), X is selected from the group consisting of -OCO-, -NRCO-, -NRCONR'-, -NRCOO-, -OCONR-, -OCOO-, -OCS-, -NRCS-, -NRCSNR'-, -OCSO-, -S-, -O-, -SO-, -SO₂-, -NR-, -CO-, -CS- and a single bond; R and R' each represent a hydrogen atom, a hydrocarbon group or a heterocyclic group; and R¹ represents one selected from the group consisting of a hydrogen atom, an alkyl group, an alkenyl group, a heterocyclic group and an alkylsilyl group.

<3> The acetylene compound and the salt thereof according to <1>, wherein in Formula (1), n is 1 and m is an integer of 2 or greater.
<4> The acetylene compound and the salt thereof according to <1>, wherein in Formula (1), -A- is a structure represented by the following Formula (2), b is an integer of from 0 to 4, m is an ingeter of from 1 to 4, and the sum of b and m is 5 or less.

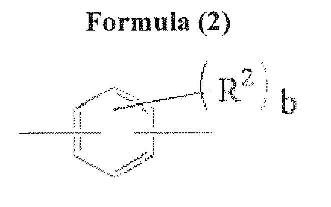

wherein in Formula (2), R² represents a hydrogen atom or a group that can be a substituent of the benzene ring.

<5> The acetylene compound and the salt thereof according to <4>, wherein -B- is a structure represented by the following Formula (3), a is an integer of from 1 to 5, and c is an integer of from 0 to 4. 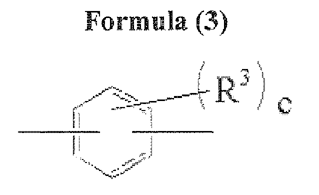 wherein in Formula (3), R³ represents a hydrogen atom or a group that can be a substituent of the benzene ring.

<6> The acetylene compound and the salt thereof according to <5>, wherein X is -OCO- or -NHCO-.

<7> The acetylene compound and the salt thereof according to <6>, wherein m is 1 or 2.
<8> The acetylene compound and the salt thereof according to <7>, wherein a is 1.
<9> The acetylene compound and the salt thereof according to <8>, wherein n is 1 or 2.
<10> The acetylene compound and the salt thereof according to <9>, wherein the ethynyl group is at a meta-position or a para-position with respect to X.

<11> An acetylene compound condensate having a structure represented by the following Formula (4) and a salt thereof, the acetylene compound condensate having a residue of the compound as described in <1> as a partial structure, and the acetylene compound condensate obtained by condensing a compound represented by Formula (1), where n is 1, with a compound having a functional group that can react with an amino group and one or more ethynyl groups in the molecule: 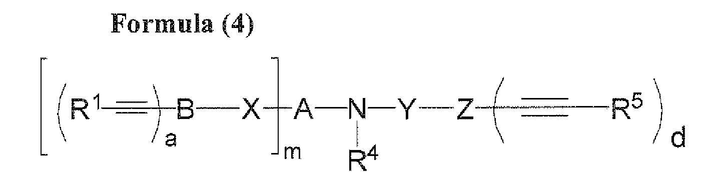 wherein in Formula (4), Z represents a hydrocarbon group; R⁴ represents a hydrogen atom or a group that can be a substituent of the amino group; R⁵ represents a hydrogen atom, a hydrocarbon grup or a silyl group; Y represents -CO-, -CS-, -O-, -CONH-, -COS-, -CH₂-, -CR"₂-CR"'₂-, =R""- or a single bond; d is an integer of 1 or greater; R", R"' and R"" each independently represent a hydrogen atom or a hydrocarbon group; and R¹, A, B, X, a and m each have the same definitions as R¹, A, B, X, a and m in <4>.

<12> The acetylene compound condensate and the salt thereof according to <11>, wherein Y is -CO-, -CS-, -CONH- or -COS-.
<13> An amide compound, an imide compound and a benzoimidazole compound, each compound being a condensate having a residue of the acetylene compound according to <1> as a partial structure,
the condensate being obtained and derived from an acetylene compound represented by Formula (1), where n is from 2 to 5, and a compound having at least one of a -COOH group, a -COOR⁰ group, a -CSOH group, a -COSH group, a -CSSH group, a -OCOL' group, a -NRCOL' group, a -OCSL' group, a -NCO group or a -NSO group; L' represnts a monovalent leaving group; R⁰ represnts a hydrocarbon group; and R has the same definitions as R in Formula (1).

<14> A condensed polymer, being a condensate including at least one acetylene compound according to any one of <1> to <10> as a structural unit thereof.
<15> The polymer according to <14>, further including at least one monomer unit selected from the group consisting of an amine compound other than that represented by Formula (1), a carboxylic acid compound, a carboxylic anhydride, a polyol compound and an aldehyde compound, as a structurral unit thereof.
<16> The polymer according to <14>, further including a diamine compound other than that represented by Formula (1) as a structural unit thereof.
<17> The polymer according to <16>, further including a tetracarboxylic anhydride as a structural unit thereof.
<18> The polymer according to <15>, having a structure of a block copolymer formed from two or more kinds of the polymer.

<19> A method of producing the polymer according to <14>, the method including using a catalyst.
<20> A composition at least including the acetylene compound according to any one of <1> to <13>, and/or a polymer including the acetylene compound according to any one of <1> to <10> as a structural unit thereof.
<21> A cured product produced by curing the composition according to <20>.
<22> A method of producing the acetylene compound or the salt thereof according to <7>, the method including:
   protecting the amino group of an acetylene compound represented by the following Formula (5) and synthesizing a compound represented by Formula (6) from the compound represented by Formula (5); and
   producing an acetylene compound represented by Formula (1) where -B- is a structure represented by Formula (3), by subjecting the compound represented by Formula (6) and a compound having an acetylene group represented by the following Formula (7) to condensation reaction.

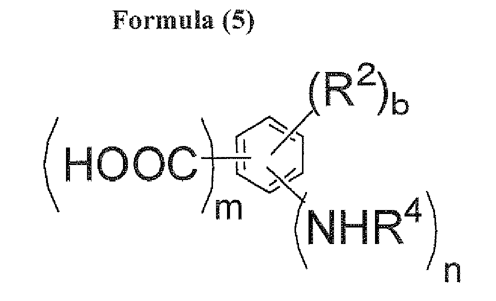

wherein in Formula (5), R², b, m and n each have the same definitions as R², b, m and n in <7>, and R⁴ represents a hydrogen a tom or a group tha can be a substituent of the amino group.

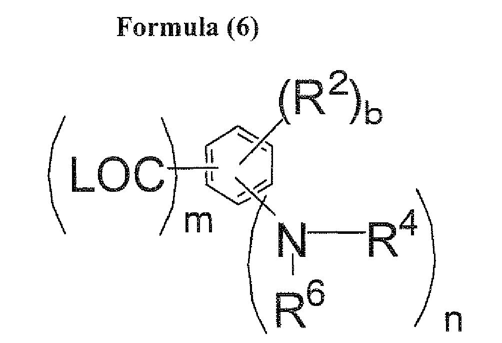

wherein in Formula (6), L is a monovalent leaving group; R⁶ represnts a functional group that can be used as a protecting group for the amino group; and R², R⁴, b, m and n each have the same definitions as R², R⁴, b, m and n in Formula (5).

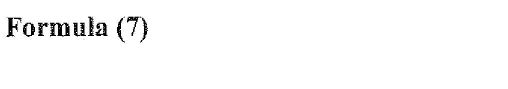

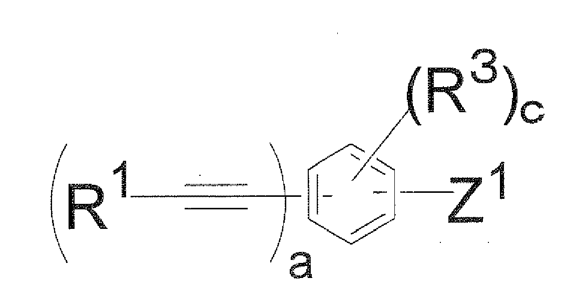

wherein in Formula (7), Z¹ represents -OH or -NHR; R has the same definitions as R in Formula (1); and R¹, R³, a and c each have the same definitions as R¹, R³, a and c in <7>.

<23> The acetylene compound or the salt thereof according to <22>, wherein the substituent L in Formula (6) is a halogen atom, a methanesulfonyl group or a phenoxycarbonyl group.
<24> The method of producing the acetylene compound and the salt thereof according to <23>, wherein isolation of the compound is not performed in each of the following steps in the method according to <22>:
   (I) protecting the amino group of a compound represented by Formula (5);
   (II) converting the compound represented by Formula (5) having the amino group protected to a compound represented by Formula (6);
   (III) reacting the compound represented by Formula (6) with an acetylene compound represented by Formula (7); and
   (IV) deprotecting the amino group.

### [Effect of the Invention]

The present invention can provide a compound having one or more amino groups that can be introduced into a condensed polymer and one or more ethynyl groups, and a salt of this compound. Further, the present invention can provide a method of producing the compound, a condensate of the same, a method of producing the condensate, a composition including the compound or the condensate, and a cured produced produced by curing the compound, the condensate and/or the composition.

### [Embodiments to Implement the Invention]

In the following, details of the present invention are described. An embodiment of the invention is a compound represented by the following Formula (1): 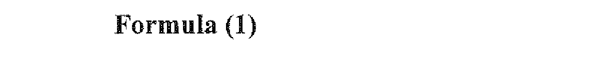 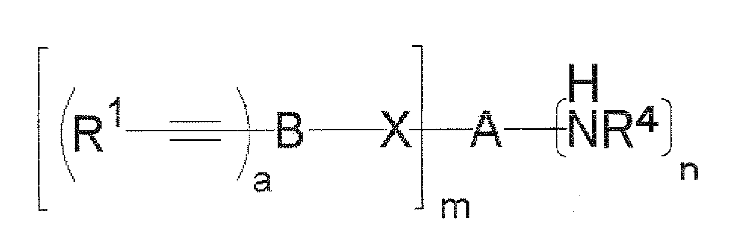 wherein in Formula (1), A represents a (m+n)-valent hydrocarbon group or a heterocyclic group (a heteroaromatic ring (heteroaryl) or a heteroalicyclic ring); B represents a single bond, an (a+1)-valent hydrocarbon group or a heterocyclic group (a heteroaromatic ring (heteroaryl) or a heteroalicyclic ring); and A and B are each optionally substituted.
Exemplary unsubstituted hydrocarbon groups include a straight chain or branched aliphatic group having 1 to 20 carbon atoms, an alicyclic group having 3 to 20 carbon atoms and an aromatic (aryl) group having 6 to 20 carbon atoms.
Exemplary straight chain or branched aliphatic groups include an alkyl group (such as a methyl group, an ethyl group, a propyl group, an 1-propyl group, a butyl group, a sec-butyl group, a t-butyl group, a neopentyl group, a hexyl group, a 2-ethylhexyl group, an octyl group or a dodecyl group) and an alkenyl group (such as a propenyl group or a butenyl group). Exemplary alicyclic groups include a cycloalkyl group (such as a cyclopentyl group, a cyclohexyl group or a menthyl group), a cycloalkenyl group (such as a cyclohexenyl group), an aliphatic polycyclic group (such as a bornyl group, a norbornyl group, a decalynyl group, an adamantyl group or a diamantyl group), and a spiro ring (such as spiro[3.4]octane, spiro[4.4]nonane or spiro[5.5]undecane).

Exemplary aromatic (aryl) rings include benzene, naphthanene, fluorene, anthracene, indene, indane and biphenyl. Exemplary heteroaromatic (heteroaryl) rings include furan, thiophene, pyridine, imidazole, pyrazole, triazole, oxasole, carbazole, indole, chromene, chromane, quinoline, dibenzofuran, phthalimide, thiophthalimide, benzoxazole, benzimidazole and benzothiazole. Exemplary heteroalicyclic compounds include oxetane, thietane, oxolane, thiolane, pyrroline, pyrrolidine, pyrazoline, imidazoline, oxane, thiane, piperidine and pyrrolidone.

Exemples of the optionally substituted hydrocarbon groups, heteroaromatic rings and heteroalicyclic compounds include hydrocarbon groups, heteroaromatic rings and heteroalicyclic compounds having a structure formed by substituting the unsubstituted hydrocarbon groups, heteroaromatic ring or heteroalicyclic compounds as illustrated above by a halogen atom (such as a fluorine atom, a chlorine atom, a bromine atom or an iodine atom), a cyano group, a nitro group, a sulfonyl group, an amido group, an alkoxy group having 1 to 20 carbon atoms (such as a methoxy group, a butoxy group or a dodecyloxy group), an aryl group (such as a phenyl grpup or a naphthyl group), a hydroxy group or a silyl group, at an arbitrary potision.

Among the above, A is preferably a (m+n)-valent, subsituted or unsubstituted, straight chain or branched aliphatic group, an alicyclic group, an aliphatic polycyclic group or an aromatic group. More preferably, A is a (m+n)-valent, subsituted or unsubstituted, straight chain or branched aliphatic group, an alicyclic group or a benzene ring. Particularly preferably, A is a (m+n)-valent, subsituted or unsubstituted, straight chain or branched aliphatic group, an alicyclic group, or an unsubstituted benzene ring. Among the above, B is preferaby a single bond, an (a+1)-valent substituted or an unsubstited benzene ring, or a heteroaromatic ring. More preferably, B is a single bond, an (a+1)-valent substituted or an unsubstited benzene ring. Particularly preferably, B is a single bond or an (a+1)-valent unsubtituted benzene ring.

In Formula (1), X represents a single bond or a divalent linking group. Specific examples of the divalent linking group include -OCO-, -NRCO-, -NRCONR'-, -NRCOO-, -OCONR-, -OCOO-, -OCS-, -NRCS-, -NRCSNR'-, -OCSO-, -O-, -SO-, -SO₂-, -S-, -NR-, -CO-, -CS-, -CRR'- and -CRR'-CR"R"'-. X and B, or X and A may form a ring together (such as an imido ring, a thioimido ring, an imidazole ring, an oxazole ring or a thiazole ring). In this case, the valency of A is m+n+1 and the valency of B is a+2. In the above specific examples of the divalent linking group, R, R', R" and R"' each independently represent a hydrogen atom, a hydrocarbon group that may be subsituted, or a heterocyclic group that may be substituted. The hydrocarbon group and the heterocyclic group may be subsituted. R or R' and B or A, R and R', R' and R", or R" and R"' may be bonded to each other to form a ring.

Exemples of R, R', R" and R"', as an unsubsituted hydrocarbon group, include a straight chain or branched aliphatic group having 1 to 20 carbon atoms, an alicyclic group having 3 to 20 carbon atoms, and an aromatic ring group having 6 to 20 carbon atoms; and as an unsubstituted heterocyclic group, a heterocyclic group having 3 to 10 carbon atoms. Exemplary straight chain or branched aliphatic groups include an alkyl group (such as a methyl gorup, an ethyl group, a propyl group, an i-propyl group, a butyl group, a sec-butyl group, a t-butyl group, a neopentyl group, a hexyl group, a 2-ethylhexyl group, an octyl group or a dodecyl group), an alkenyl group (such as a propenyl group or a butenyl group). Exemplary alicyclic groups include a cycloalkyl group (such as a cyclopentyl group, a cyclohexyl group or a menthyl group), a cycloalkenyl group (such as a cyclohexenyl group), an aliphatic polycyclic group (such as a bornyl group, a norbornyl group, a decalynyl group, an adamantyl group or a diamanthyl group), a spiro ring (such as spiro[3.4]octane, spiro[4.4]nonane or spiro[5.5]undecane), and an aromatic ring (such as benzene, naphthalene, fluorene, anthracene, indene, indane or biphenyl). Exemplary heterocyclic ring groups include a group of the aforementioned heteroaromatic ring or a heteroalicyclic compound.

Exemplary hydrocarbon groups and heterocyclic groups that may be substituted include hydrocarbon groups or heteroxyxlic groups having a structure formed by substituting the aforementioned unsubsituted hydrocarbon groups and heterocyclic groups by a halogen atom (such as a fluorine aton, a chlorine aton, a bromine atom or an iodine atom), a cyano group, a nitro group, a sulfonyl group, an amido group, an alkoxy group having 1 to 20 carbon atoms (such as a methoxy group, a butoxy group or a dodecyloxy group), an aryl group (such as a phenyl group or a naphthoyl group), a hydroxy group or a silyl group, at an arbitrary position. Among these examples, in view of availability of raw materials and ease of production, R, R', R" and R"' are preferably a hydrogen atom, a substiuted or unsubstitued cyclic or non-cyclic alkyl group, or an alkylsilyl group; more preferably a hydrocarbon group having 1 to 8 carbon atoms that is unsubstituted or substituted by a hydroxy group, a halogen atom (such as a fluorine atom or a chlorine atom), or an alkoxy group having 1 to 4 carbon atoms, an alkylsilyl group having 1 to 6 carbon atoms, or a hydrogen atom; further preferably an unsubstituted hydrocarbon group having 1 to 8 carbon atoms, an alkylsilyl group having 1 to 6 carbon atoms, or a hydogen atom; and particularly preferably a hydrogen atom.

In view of availability of raw materials or ease of production, the substituent X is preferably a single bond, -OCO-, -NRCO-, -NRCONR'-, -NRCOO-, -OCONR-, -OCOO-, -O- and -NR-; and particularly preferably a single bond, -OCO- or -NRCO-.

R¹ represents a hydrogen atom or a monovalent hydrocarbon group, a heteroaromatic ring, a heteroalicyclic compound, or a silyl group. When n is 2 or greater, R¹ preferably represents a hydrogen atom, an alkyl group, an alkenyl group, a heterocyclic group (a heteroaromatic ring or a heteroalicyclic compound), or an alkylsilyl group. When R¹ is not a hydrogen atom, R¹ may be substituted. Exemplary unsubstituted hydrocarbon groups include a straight chain or branched aliphatic group having 1 to 20 carbon atoms, an alicyclic group having 3 to 20 carbon atoms, or an aromatic ring group having 6 to 20 carbon atoms. Exemplary straight chain or branched aliphatic groups include an alkyl group (such as a methyl group, an ethyl group, a propyl group, an i-propyl group, a butyl group, a sec-butyl group, a t-butyl group, a neopentyl group, a hexyl group, a 2-ethylhexyl group, an octyl group or a dodecyl group) and an alkenyl group (such as a propenyl group and a butenyl group). Exemplary alicyclic groups include a cycloalkyl group (such as a cyclopentyl group, a cyclohexyl group or a methyl group), a cycloalkenyl group (such as a cyclohexenyl group), an aliphatic polycyclic group (such as a bornyl group, a norbornyl group, a decalynyl group, an adamanthyl group or a diamanthyl group), a spiro ring (such as spiro[3.4]octane, spiro[4.4]nonane or spiro[5.5]undecane), and an aromatic ring (such as benzene, naphthalene, fluorene, anthracene, indene, indane or biphenyl).

Exemplary heteroaromatic rings include furan, thiophene, pyridine, imidazole, pyrazole, triazole, oxazole, carbazole, indole, chromene, chromane, quinoline and dibenzofuran. Exemplary heteroalicyclic compounds include oxetane, thietane, oxolane, thiolane, pyrroline, pyrrolidine, pyrazoline, imidazoline, oxane, thian, piperidine and pyrrolidone.

Examples of the hydrocarbon groups, heteroaromatic rings, heteroalicyclic compounds and silyl groups that may be substituted include compounds having a structure formed by substiting the aforementioend unsubstituted hydrocarbon groups, heteroaromatic rings, hereroalicyclcic compounds or silyl groups by a halogen atom (such as a fluorine atom, a chlorine atom, a bromine atom or an iodine atom), a cyano group, a nitro group, a sulfonyl group, an amido group, an alkoxy group having 1 to 20 carbon atoms (such as a methoxy group, a butoxy group or a dodecyloxy group), an aryl group (such as a phenyl grpup or a naphthyl group), a hydroxy group, or a silyl group, at an arbitrary position. Among these, in view of availability of raw materials and ease of production, R¹ is preferably a hydrogen atom, an alkyl group that may be substituted, a cycloalkyl group, or an alkylsilyl group; more preferably a hydrocarbon group having 1 to 6 carbon atoms that is unsubstituted or substituted by a hydroxy group, a halogen atom (such as a fluorine atom or a chlorine atom) or an alkoxy group having 1 to 4 carbon atoms, an alkylsilyl group having 1 to 6 carbon atoms, or a hydrogen atom; further preferably an unsubstituted hydrocarbon group having 1 to 6 carbon atoms, an alkylsilyl group having 1 to 6 carbon atoms or a hydrogen atom; and particularly preferably a hydrogen atom.

R⁴ represents a hydrogen atom or a group that can be a substituent of the amino group. Examples of the group that can be a substituent of the amino group include substituted or unsubstituted hydrocarbon groups represented by R in the aforementioned Formula (1), a heterocyclic group that may be substituted, or an acyl group having 1. to 20 carbon atoms (such as a formyl group, an acetyl group, a propanoyl group, an octanoyl group, a benzoyl group, a naphthoyl group or a cinnamoyl group), a hydroxy group, a cyano group, and an alkoxy group having 1 to 20 carbon atoms (such as a methoxy group, an ethoxy group, a propoxy group, a butoxy group or a dodecyloxy group). Among these, R⁴ is preferably a hydrogen atom, an alkyl group having 1 to 10 carbon atoms or a phenyl group; more preferably a hydrogen atom or an alkyl group having 1 to 6 carbon atoms; further preferably a hydrogen atom or an alkyl group having 1 to 4 carbon atoms; and particularly preferably a hydrogen atom.

a represents an integer of 1 or greater, and in view of availability of raw materials and ease of production, a is preferably an integer of from 1 to 5, more preferably from 1 to 3, and particularly preferably 1. m represents an integer of 1 or greater, and in view of availability of raw materials and ease of production, m is preferably an integer of from 1 to 3, more preferably 1 or 2. When a or m is 2 or greater, two or more of R¹ and a group including an ethynyl group represented by B-X in the square brackets may be the same or different.

The salt of the compound represented by Formula (1) is a salt formed from an amino group and an acid that can form a salt, and the acid may be an inorganic acid or an organic acid. Exemplary inorganic acids include hydrochloric acid, sulfuric acid, sulfurous acid, nitric acid, nitrous acid, carbonic acid, bicarbonic acid, hydrofluoric acid, bromic acid, phosphoric acid, phosphorous acid, silicic acid and boric acid. Exemplary organic acids include sulfonic acids, sulfinic acids, alkylsulfonic acids, phosphonic acids, carboxylic acids, phosphates and phenols. Organic acids described in JP-A Nos. 60-88942 and 2-96755 may also be mentioned. Specific examples of the organic acid include methanesulfonic acid, trifluoromethane sulfonic acid, p-toluene sulfonic acid, dodecylbenzene sulfonic acid, p-toluenesulfinic acid, ethanesulfinic acid, phenylphosphonic acid, phenylphosphinic acid, phenyl phosphate, diphenyl phosphate, formic acid, acetic acid, propionic acid, butyric acid, glycolic acid, chloroacetic acid, dichloroacetic acid, trichloroacetic acid, fluoroacetic acid, trifluoroacetic acid, bromoacetic acid, methoxyacetic acid, oxaloacetic acid, citric acid, oxalic acid, succinic acid, malic acid, tartaric acid, fumaric acid, maleic acid, malonic acid, ascorbic acid, benzoic acid, substituted benzoic acids such as 3,4-dimethoxy benzoic acid, phenoxyacetic acid, phthalic acid, picric acid, nicotinic acid, picolinic acid, dipicolinic acid, adipic acid, p-toluic acid, terephthalic acid, 1,4-cyclohexen-2,2-dicarboxylic acid, eruic acid, lauric acid, n-undecanoic acid, ascorbic acid, phenol, and p-chlorophenol.

Among these, in view of availability of raw materials and ease of production, the acid is preferably inorganic acids, sulfonic acids and carboxylic acids; more preferably hydrochloric acid, sulfuric acid, sulfurous acid, nitric acid, carbonic acid, phosphoric acid, sulfonic acids, posphonic acids and carboxylic acids; further preferably hydrochloric acid, sulfuric acid, nitric acid, methanesulfonic acid, trifluoromethanesulfonic acid, p-totuenesulfonic acid, phenylphosphonic acid, chloroacetic acid, dichloroacetic acid, trichloroacetic acid, fluoroacetic acid, trifluoroacetic acid, formic acid and oxalic acid; and particularly preferably hydrochloric acid, sulfonic acid, methanesulfonic acid, trifluoromethanesulfonic acid, chloroacetic acid, fluoroacetic acid, trifluoroacetic acid and oxalic acid.

A further exemplary embodiment of the invention is a compound represented by Formula (1) where -A- is a structure represented by the following Formula (2), b is an integer of from 0 to 4, m is an integer of from 1 to 4, and the sum of b and m is 5 or less; and a salt of this compound.

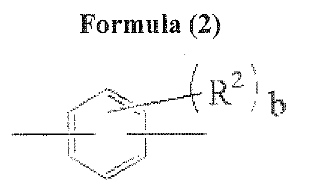

In Formula (2), R² represents a hydrogen atom or a group that can be a substituent of the benzene ring. Specific examples of the group that can be a substituent of the benzene ring include a substituted or unsubstituted hydrocarbon group, a halogen atom, a cyano group, a nitro group, a sulfonyl group, an acylamino group having 1 to 20 carbon atoms (such as a formylamino group, an acetylamino group, a propanoylamino group, an octanoylamino group, a benzoylamino group and a naphthoylamino group), an alkoxycarbonyl goup having 1 to 20 carbon atoms (such as a methoxycarbonyl group, an ethoxycarbonyl group, a propoxycarbonyl group, an iso-propoxycarbonyl group, a butoxycarbonyl group, a sec-butoxycarbonyl group, an iso-butoxycarbonyl group, an octoxycarbonyl group and a dodecyloxycarbonyl group), an acyl group having 1 to 20 carbon atoms (such as a formyl group, an acetyl group, a propanoyl group, an octanoyl group, a benzoyl group, a naphthoyl group and a cinnamoyl group), an acyloxy group having 1 to 20 carbon atoms (such as a fornyloxy group, an acetyloxy group, a propanoyloxy group, an octanoyloxy group, a benzoyloxy group, a naphthoyloxy group, or a cinnamoyloxy group ), an alkoxy group having 1 to 20 carbon atoms (such as a methoxy group, an ethoxy group, a propoxy group, an iso-propoxy group, a butoxy group, a sec-butoxy group, an iso-butoxy group, an octoxy group and a dodecyloxy group), an aryl group (such as a phenyl group, a naphthyl group, a fluorenyl group, an anthracenyl group, an indenyl group, an indanyl group and a biphenyl group), a hydroxy group, and a silyl group.

Exemplary unsubstituted hydrocarbon groups include a straight chain or branched aliphatic group having 1 to 20 carbon atoms, an alicyclic group having 3 to 20 carbon atoms, and an aromatic ring group having 6 to 20 carbon groups. Examples of the straight chain or branched aliphatic group include an alkyl group (such as a methyl group, an ethyl group, a propyl group, an i-propyl group, a butyl group, a sec-butyl group, a t-butyl group, a neopentyl group, a hexyl froup, a 2-ethylhexyl group, an octyl group and a dodecyl group), and an alkenyl group (such as a propenyl group and a butenyl group). Examples of the alicyclic group include a cycloalkyl grop (such as a cyclopentyl group, a cyclohexyl group and a methyl group), a cycloalkenyl group (such as a cyclohexenyl group), an aliphatic polycyclic group (such as a bornyl group, a norbornyl group, a decalinyl group, an adamanthyl group and a diamanthyl group), a monovalent spiro ring (such as spiro[3.4]octane, spiro[4.4]nonane and spiro[5.5]undecane), and a monovalent aromatic ring (such as benzene, naphthalene, fluorene, anthracene, indene, indane and biphenyl).

Examples of the hydrocarbon group that may be substituted include a hydrocarbon group having a structure formed by substituting the unsubstituted hydrocarbon group as illustrated above by a halogen atom (such as a fluorine atom, a chlorine atom, a bromine atom or an inodine atom), a cyano group, a nitro group, a sulfonyl group, an acylamino group having 1 to 20 carbon groups (such as a formylamino group, an acetylamino group, a propanoylamino group, an octanoylamino group, a benzoylamino group or a naphthoyl group), an alkoxy group having 1 to 20 carbon atoms (such as a methoxy group, a butoxy group or a dodecyloxy group), an aryl group (such as a phenyl group or a naphthyl group), a hydroxy gorup, or a silyl group, at an artibtrary position.

Among the above, in view of availability of raw materials and ease of production, R² is preferably a hydrogen atom, a halogen atom, a cyano group, a nitro group, a sulfonyl group, an acylamino group having 1 to 20 carbon atoms, an alkyl group having 1 to 20 carbon atoms, an alkoxy group having 1 to 20 carbon atoms, an aryl group, or a hydroxy group; more preferably a hydrogen atom, a halogen atom, an alkyl group having 1 to 8 carbon atoms, or an alkoxy group having 1 to 8 carbon atoms; further preferably a hydrogen atom, a chlorine atom, a fluorine atom, an alkyl group having 1 to 4 carbon atoms or an alkoxy group having 1 to 4 carbon atoms; and particularly preferably a hydrogen atom.

b represents an integer of from 0 to 3, and in view of availability of raw materials and ease of production, b is preferably from 0 to 2, more preferably 0 or 1. When -A- in Formula (1) is a structure represented by Formula (2), m in Formula (1) represents an integer of from 1 to 5, preferably from 1 ta 4, more preferably from 1 to 3. The sum of b and m is 5 or less. When b is 2 or greater, two or more of R² may be the same or different, and may be bonded to each other to form a ring.

A further embodiment of the invention is a compound represented by Formula (1) where -A- is a structure represented by Formula (2), -B- is a structure represented by the following Formula (3), a is an integer of from 1 to 5, b is an integer of from 0 to 4, c is an integer of from 0 to 4, m is an integer of from 1 to 4, and the sum of b and m is 5 or less; and a salt of this compound.

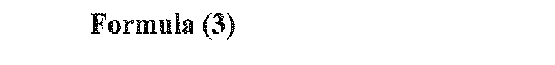

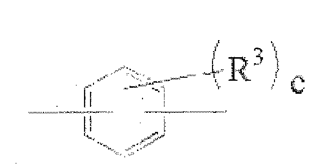

In Formula (3), R³ represents a hydrogen or a group that can be a substituent of the benzene ring. Specific examples of the group that can be a substituent of the benzene ring include a substituted or unsubstituted hydrocarbon group, a halogen atom, a cyano group, a nitro group, a sulfonyl group, an acylamino group having 1 to 20 carbon atoms (such as a formylamino group, an acetylamino group, a propanoylamino group, an octanoylamino group, a benzoylamino group and a naphthoylamino group), an alkoxycarbonyl goup having 1 to 20 carbon atoms (such as a methoxycarbonyl group, an ethoxycarbonyl group, a propoxycarbonyl group, an iso-propoxycarbonyl group, a butoxycarbonyl group, a sec-butoxycarbonyl group, an iso-butoxycarbonyl group, an octoxycarbonyl group and a dodecyloxycarbonyl group), an acyl group having 1 to 20 carbon atoms (such as a formyl group, an acetyl group, a propanoyl group, an octanoyl group, a benzoyl group, a naphthoyl group and a cinnamoyl group), an acyloxy group having 1 to 20 carbon atoms (such as a formyloxy group, an acetyloxy group, a propanoyloxy group, an octanoyloxy group, a benzoyloxy group, a naphthoyloxy group, or a cinnamoyloxy group ), an alkoxyl group having 1 to 20 carbon atoms (such as a methoxy group, an ethoxy group, a propoxy group, an iso-propoxy group, a butoxy group, a sec-butoxy group, an iso-butoxy group, an octoxy group and a dodecyloxy group), an aryl group (such as a phenyl group, a naphthyl group, a fluorenyl group, an anthracenyl group, an indenyl group, an indanyl group and a biphenyl group), a hydroxy group, and a silyl group.

Exemplary unsubstituted hydrocarbon groups include a straight chain or branched aliphatic group having 1 to 20 carbon atoms, an alicyclic group having 3 to 20 carbon atoms, and an aromatic ring group having 6 to 20 carbon groups. Examples of the straight chain or branched aliphatic group include an alkyl group (such as a methyl group, an ethyl group, a propyl group, an i-propyl group, a butyl group, a sec-butyl group, a t-butyl group, a neopentyl group, a hexyl group, a 2-ethylhexyl group, an octyl group and a dodecyl group), and an alkenyl group (such as a propenyl group and a butenyl group). Examples of the alicyclic group include a cycloalkyl grop (such as a cyclopentyl group, a cyclohexyl group and a menthyl group), a cycloalkenyl group (such as a cyclohexenyl group), an aliphatic polycyclic group (such as a bornyl group, a norbornyl group, a decalinyl group, an adamanthyl group and a diamanthyl group), a monovalent spiro ring (such as spiro[3.4]octane, spiro[4.4]nonane and spiro[5.5]undecane), and a monovalent aromatic ring (such as benzene, naphthalene, fluorene, anthracene, indene, indane and biphenyl).

Examples of the hydrocarbon group that may be substituted include a hydrocarbon group having a structure formed by substituting the unsubstituted hydrocarbon group as illustrated above by a halogen atom (such as a fluorine atom, a chlorine atom, a bromine atom or an inodine atom), a cyano group, a nitro group, a sulfonyl group, an acylamino group having 1 to 20 carbon groups (such as a formylamino group, an acetylamino group, a propanoylamino group, an octanoylamino group, a benzoylamino group or a naphthoylamino group), an alkoxy group having 1 to 20 carbon atoms (such as a methoxy group, a butoxy group or a dodecyloxy group), an aryl group (such as a phenyl group or a naphthyl group), a hydroxy group, or a silyl group, at an artibtrary position. Among the above, in view of availability of raw materials and ease of production, R³ is preferably a hydrogen atom, a halogen atom, a cyano group, a nitro group, a sulfonyl group, an acylamino group having 1 to 20 carbon atoms (such as a formylamino group, an acetylamino group, a propanoylamino group, an octanoylamino group, a benzoylamino group or a naphthoylamino group), an alkyl group having 1 to 20 carbon atoms, an alkoxy group having 1 to 20 carbon atoms, an aryl group, or a hydroxy group; more preferably a hydrogen atom, a halogen atom, an alkyl group having 1 to 8 carbon atoms, or an alkoxy group having 1 to 8 carbon atoms; further preferably a hydrogen atom, a chlorine atom, a fluorine atom, an alkyl group having 1 to 4 carbon atoms or an alkoxy group having 1 to 4 carbon atoms; and particularly preferably a hydrogen atom.

c represents an integer of from 0 to 4, and in view of availability of raw materials and ease of production, c is preferably from 0 to 3, more preferably 0 or 1. When -B- in Formula (1) is a structure represented by Formula (3), a in Formula (1) represents an integer of from 1 to 5, and in view of availability of raw materials and ease of production, a is preferably from 1 to 3, more preferably from 1 or 2. The sum of a and c is 5 or less. When c is 2 or greater, two or more of R³ may be the same or different, and may be bonded to each other to form a ring.

A further embodiment of the invention is a condensed polymer, which is a condensate that includes at least one acetylene compound selected from those described in <1> to <10> as mentioned above, as a structural unit. Exemplary polymers including the aforementioned acetylene compound as a structural unit include polyamine, polyimide, polyisoimide, polyesterimide, polyetherimide, polyamideimide, polyamic acid, polyamic acid ester, polyamide, polythioamide, polyurethane, polyurea and polyazomethine. Among these, polyimide, polyisoimide, polyesterimide, polyetherimide, polyamidoimide, polyamic acid, polyamic acid ester and polyamide are preferred; and polyimide, polyisoimide, polyesterimide, polyetherimide, polyamideimide and polyamic acid are more preferred.
The aforementioned polymer may be a homopolymer or a block copolymer. The basic skeleton of the polymer may be an aromatic skeletone or an aliphatic skeletone, and silicone, fluorene or the like may be included in the main chain or side chain thereof; but the skeleton is preferably an aromatic skeleton.

### <Compound derived from acetylene compound>

A further embodiment of the invention is a derivative condensate having a residue of the acetylene compound represented by Formula (1), which is obtained and derived from the acetylene compound represented by Formula (1) and a compound having at least one of-CHO group, a -COOH group, a -COOR⁰ group, a -CSOH group, a -COSH group, a -CSSH group, a -OCOL' group, a -NRCOL' group, a -OCSL' group, a -NCO group or a -NSO group in the molecule thereof. L' represents a monovalent leaving group and R⁰ represents a hydrocarbon group. The hydrocarbon group represented by R⁰ may be the same as those represented by R in Formula (1). Among these, R⁰ is preferably an unsubstituted, halogen-substituted or alkoxy-substituted alkyl group, a cycloalkyl group, an aliphatic polycyclic gorup, or an aromatic ring group; more preferably an unsubstituted, halogen-substituted or alkoxy-substituted alkyl group having 1 to 10 carbon atoms, a cycloalkyl group or a phenyl group; further preferably an unsubstituted, halogen-substituted or alkoxy-substituted alkyl group having 1 to 6 carbon atoms, a cycloalkyl group or a phenyl group; and particularly preferably an unsubstituted, halogen-substituted or alkoxy-substituted alkyl group having 1 to 4 carbon atoms, or a phenyl group. R has the same definitions as R in Formula (1).

Examples of the compound having at least one of a -CHO group, a -COOH group, a -COOR⁰ group, a -CSOH group, a -COSH group, a -CSSH group, a -OCOL' group, a -NRCOL' group, a -OCSL' group, a -NCO group or a -NSO group in the molecule thereof include aldehydes (such as benzaldehyde and 3-fluorobenzaldehyde), carboxylic acids (such as aliphatic carboxylic acids including acetic acid, propionic acid, pivalic acid and cyclohexanecarboxylic acid, aromatic carboxylic acids including benzoic acid, 4-fluorobenzoic acid, 3,5-dimethylbenzoic acid and naphthalene carboxylic acid, and heterocyclic carboxylic acids including 3-furancarboxylic acid, 2-thiophenecarboxylic acid, pyridine-3-carboxylic acid and pyrrole-1-carboxylic acid), esters (such as ethyl acetate and methyl benzoate), thiocarboxylic acids (such as hexanethio-S-acid, thio-O-acetic acid, cyclohexanecarbothio-O-acid and heptanedithioic acid), carbamates (such as N-phenoxycarbonylaniline, N-p-nitrophenoxycarbonylaniline, N-methoxycarbonylaniline, N-isopropoxycarbonylaniline, N-t-butoxycarbonylaniline and N-phenoxycarbonylcyclohexylamine), acid halides (such as phosgene, phenyl chlorocarbonate, methyl chloroformate and phenyl bromocarbonate), thiocarbamates (such as N-phenoxythiocarbonylaniline, N-p-nitrophenoxythiocarbonylaniline, N-methoxythiocarbonylaniline, N-t-butoxythiocarbonylaniline and N-phenoxythiocarbonylcyclohexylamine), isocyanates (such as phenylisocyanate, pentylisocyanate, 4-methylphenylisocyanate, 4-fluoroisocyanate and cyclohexylisocyanate), and thioisocyanates (such as phenylthioisocyanate, butylthioisocyanate, p-toluylisothiocyanate, cyclohexylisothiocyanate and 2,4,6-tetramethylisothiocyanate).

Among these, in veiw of reactivity with respect to an amino group and availability of raw materials, carboxylic acids, acid halides, thiocarbamates, isocyanates and thioisocyanates are preferred, and carboxylic acids are more preferred.

L' represents a monovalent leaving group, which is not limited as long as it can be a substituent of the nitrogen atom or an oxygen atom by reaction with an amino group or a hydroxy group. Preferred examples thereof inlcude a halogen atom (such as a fluorine atom, a chlorine atom, a bromine atom or an iodine atom), a sulfonate group (such as a mesylate group, a tosylate group and a triflate group), a methanesulfonyl group, an alkoxyl group, an alkoxycarbonyl group, a diazonium group, and a trialkylammonium group (such as trimethylammonium). Among these, a halogen atom, a methanesulfonyl group, a sulfonate group, an alkoxyl group and an alkoxycarbonyl group are preferred, and a halogen atom, an alkoxyl group and an alkoxycarbonyl group are more preferred.

Exemplary methods of preparing and deriving a derivative compound having a residue of the acetylene compound represented by Formula (1) as a partial structure from an acetylene compound represented by Formula (1) with a compound having at least one of a -CHO group, a -COOH group, a -COOR⁰ group, a -CSOH group, a -COSH group, a -CSSH group, a -OCOL' group, a -NRCOL' group, a -OCSL' group, a -NCO group or a -NSO group in the molecule thereof include, when an acetylene compound represented by Formula (1) is reacted with a compound having a -COOH group, -CSOH group, -COSH group or a -CSSH group, a method of converting this compound to an intermediate that is highly active with respect to condesation reaction, and then reacting the intermediate with the compound represented by Formula (1); and a method of directly condensing these compounds under the presence of a catalyst.

Among these, in view of reactivity and prevention of decomposition or reaction of ethynyl groups of the intended product, a method of converting the compound to an intermediate that is highly active with respect to condesation reaction, and then reacting the intermediate with the compound represented by Formula (1) is preferred.

When an acetylene compound represented by Formula (1) is reacted with a compound having at least one of a -CHO group, a -OCOL' group, a -NRCOL' group, a -OCSL' group, a -NCO group or a -NSO group in the molecule thereof, this compound can be directly reacted with the compound represented by Formula (1).

A further embodiment of the invention is a compound represented by the following Formula (4), the compound having a residue of the compound represented by Formula (1) as a partial structure, and being produced by reaction of a compound represented by Formula (1) with a compound having a functional group that can react with an amino group and one or more ethynyl groups in the molecule thereof; and a salt of this compound.

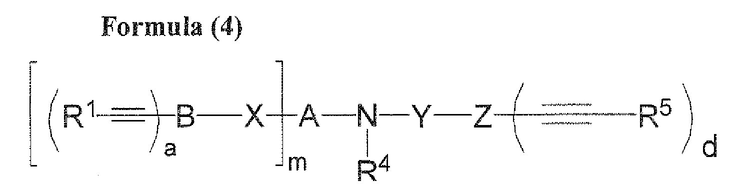

In Formula (4), Y represents -CO-, -CS-, -CONH-, -COS-, -CH₂-, -CR"₂-CR"'₂-, =CR""- or a single bond. Among these, Y is preferably -CO-, -CS-, -CONH-, -COS-, -CH₂- or a single bond; more preferably -CO-, -CS-, -CONH- or -COS-; particularly preferably -CO- or -CONH-.

R", R"' and R"" each independently represent a hydrogen atom or a hydrocarbon group that may be substitued or may not substituted. Exemplary hydrocarbon groups that are not substituted include a straight chain hydrocarbon group having 1 to 20 carbon atoms (such as a methyl group, an ethyl group, a butyl group, an octyl gorup, a dodecyl group, a methylene group and an ethylene group), a cyclic hydrocarbon group (such as a cyclopentyl group, a cyclohexyl group, a cyclopentenyl group or a cyclohexenyl group), a monovalent aromatic ring (such as benzene, naphthalene and fluorene), a monovalent hetero ring (such as furan, thiophene, pyridine, imidazole, pyrazole, triazole, oxazole, thiazole, indole and carbazole), a monovalent condensed polycyclic group (such as a norbonane group, a norbornene group, a norbornylane group, an adamantane group and a diamantane group), and a monovalent spiro ring (such as spiro[3.4]octane, spiro[4.4]nonane and spiro[5.5]undecane).

Exemplary hydrocarbon groups that may be substituted include a hydrocarbon group having a structure formed by substituting the aforementioned unsubstituted hydrocarbon group by a halogen atom (such as a fluorine atom, a chlorine atom, a bromine atom or an iodine atom), a cyano group, a nitro group, a sulfonyl group, an acylamino group having 1 to 20 carbon atoms (such as a formylamino group, an acetylamino group, a propanoylamino group, an octanoylamino group, a benzoylamino group and a naphthoylamino group), an alkoxy group having 1 to 20 carbon atoms (such as a methoxy group, a butoxy group and a dodecyloxy group), an aryl group (such as a phenyl group and a naphthyl group), a hydroxy group, or a silyl group, at an arbitrary position.

Among the above examples, R", R"' and R"" are each independently preferably a hydrogen agom, a straight chain or cyclic hydrocarbon group that may be substituted, or an alkylsilyl group; more preferably a hydrocarbon group having 1 to 6 carbon atoms that is ubsubstituted or substitued by a hydroxy group, a halogen atom (such as a fluorine atom or a chlorine atom) or an alkoxy group having 1 to 4 carbon atoms, or a hydrogen atom; further preferably an unsubstituted hydrocarbon group having 1 to 6 carbon atoms or a hydrogen atom; particularly preferably a hydrogen atom.

Z represents a (d+1)-valent, optionally-substituted hydrocarbon group. Exemplary unsubstituted hydrocarbon groups include a (d+1)-valent hydrocarbon group having 1 to 20 carbon atoms (such as a methylene group, an ethylene group, a propylene group, a butylene group and an octylene group), a cyclic hydrocarbon group (such as a cyclopentylene group and a cyclohexylene group), a cycloalkenylene group (such as a cyclopentenylene group, a cyclphexenylene group and a cyclodecenylene group), a (d+1)-valent aromatic ring (such as benzene, naphthalene, fluorene and anthracene), a (d+1)-valent herero ring (such as furan, thiopehne, pyridine, imidazole, pyrazole, triazole, oxazole, carbazole and indole), a (d+1)-valent condensed polycyclic group (such as a norbornane group, a norbornene group, a norbornylane group, an adamantane group and a diamantane group), a (d-1)-valent spiro ring (such as spiro[3.4]octane, spiro[4.4]nonane and spiro[5.5]undecane).

Exemplary hydrocarbon groups that may be substituted include a hydrocarbon group having a structure formed by substituting the aforementioned unsubstituted hydrocarbon group by a halogen atom (such as a fluorine atom, a chlorine atom, a bromine atom or an iodine atom), a cyano group, a nitro group, a sulfonyl group, an acylamino group having 1 to 20 carbon atoms (such as a formylamino group, an acetylamino group, a propanoylamino group, an octanoylamino group, a benzoylamino gorup and a naphthoylamino group), an alkoxy group having 1 to 20 carbon atoms (such as a methoxy group, a butoxy group and a dodecyloxy group), an aryl group (such as a phenyl group and a naphthyl group), a hydroxy group, or a silyl group, at an arbitrary position. Among these, Z is preferably a (d+1)-valent unsubstituted or substituted aromatic ring or a hetero ring; more preferably a (d+1)-valent unsubstituted or substituted benzene ring; particularly preferably a (d+1)-valent unsubstituted benzene ring.

R⁴ represents a hydrogen atom or a group that can be a substituent of an amino group. Among these,R⁴ is preferably a hydrogen atom, a hydrocarbon group, a hydroxy group, an alkoxy group or a cyano group. When R⁴ is bonded to Z to form a ring, R⁴ is preferably a carbonyl group or a thiocarbonyl group. The hydrocarbon group represented by R⁴ may be substituted. R⁴ and Z may be bonded to form a ring (such as an imide ring or a thioimide ring), and the valency of Z of this case is (d+2). Further, R⁴ may be a group represented by the following formula. 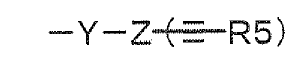 In the above formula, Y, Z and R⁵ have the same definitions as that in Formula (4).
Exemplary unsubstituted hydrocarbon groups represetned by R⁴ include an alkyl group having 1 to 20 carbon atoms (such as a menthyl group, an ethyl group, a butyl group, an octyl group and a dodecyl group), an alkenyl group (such as an ethenyl group, a propenyl group and a butenyl group), a cycloalkyl group (such as a cyclopentyl group anc a cyclohexyl group), a cycloalkenyl group (such as a cyclopentenyl group, a cyclohexenyl, group and a cyclodecenyl group), a monovalent aromatic ring (such as benzene, naphthalene and fluorene), a monovalent condensed polycyclic group (such as a norbornane group, a norbornene group, a norbornylane group, an adamantane group and a diamantane group), and a monovalent spiro ring (such as spiro[3.4]octane, spiro[4.4]nonane and spiro[5.5]undecane).

Exempalry hydrocarbon groups that may be substituted include a hydrocarbon group having a structure formed by substituting the aforementioned hydrocarbon group by a halogen atom (such as a fluorine atom, a chlorine atom, a bromine atom or an iodine atom), a cyano group, a nitro group, a sulfonyl group, an acylamino group having 1 to 20 carbon atoms (such as a formylamino group, an acetylamino group, a propanoylamino group, an octanoylamino group, a benzoylamino group and a naphthoylamino group), an alkoxy group having 1 to 20 carbon atoms (such as a methoxy group, a butoxy group and a dodecyloxy group), an aryl group (such as a phenyl group or a naphthyl group), a hydroxy group, or a silyl group, at an arbitrary position.

Among these, R⁴ is preferably a hydrogen atom, an unsubstituted or substituted alkyl group or a carbonyl group; more preferably a hydogen atom, a hydrocarbon group having 1 to 6 carbon atoms that is unsubstituted or substituted by a hydroxy group, a halogen atom (such as a fluorine atom or a chlorine atom) or an alkoxy group having 1 to 4 carbon atoms, or a carbonyl group; further preferably a hydrogen atom, an unsubstituted hydrocarbon group having 1 to 6 carbon atoms, or a carbonyl group; particularly preferably a hydrogen atom.

R⁵ represents a hydrogen atom, a hydrocarbon group, a hetero ring (a heteroaromatic ring or a heteroalicyclic compound) group, or a silyl group. When R⁵ is not a hydrogen atom, it may be substituted by an arbitrarily selected substituent.
Exemplary unsubstituted hydrocarbon groups include an alkyl group having 1 to 20 carbon atoms (such as a methyl group, a butyl group, an octyl group and an isopropyl group), an alkenyl group (such as an ethenyl group and a butenyl group), a cycloalkyl group (such as a cyclopentyl group and a cyclohexyl group), a cycloalkenyl group (such as a cyclopentenyl group, a cyclohexenyl group and a cyclodecenyl group), a monovalent aromatic ring (such as benzene, naphthalene and fluorene), a monovalent condensed polycyclic group (such as a norbomane group, a norbornene group, a norbornylane group, an adamantane group and a diamantane group), and a monovalent spiro ring (such as spiro[3.4]octane, spiro[4.4nonane and spiro[5.5]undecane).

Exempary heteroaromatic rings include furan, thiophene, pyridine, imidazole, pyrazole, triazole, oxazole, carbazole, indole, chromene, chromane, quinoline and dibenzofuran. Exemplary heteroalicyclic compounds include oxetane, thietane, oxolane, thiolane, pyrroline, pyrrolidine, pyrazoline, imidazotine, oxane, thian, piperidine and pyrrolidone.

The optionally substituted hydrocarbon group, the hetero ring (a heteroaromatic ring or a heteroalicyclic compound) or the silyl group include those having a structure formed by substituting the aforementioned unsubstituted hydrocarbon group, the hetero ring (a heteroaromatic ring or a heteroalicyclic compound) or the silyl group by a halogen atom (such as a flurorine atom, a chlorine atom, a bromine atom or an iodine atom), a cyano group, a nitro group, a sulfonyl group, an acylamino group having 1 to 20 carbon atoms (such as a formylamino group, an acetylamino group, a propanoylamino group, an octanoylamino group, a benzoylamino group or a naphthoylamino group), an alkoxy group having 1 to 20 carbon atoms (such as a methoxy group, a butoxy group or a dodecyloxy group), an aryl group (such as a phenyl group or a naphthyl group), a hydroxy group, or a silyl group, at an arbitrary position.

Among these, R⁵ is preferably a hydrogen atom, an unsubstituted or substituted alkyl group or an alkylsilyl group: more preferably a hydrocarbon group that is unsubstituted or substituted by a hydroxy group, a halogen atom (such as a fluorine atom or a chlorine atom) or an alkoxy group having 1 to 4 carbon atoms, an alkylsilyl group having 1 to 6 carbon atoms, or a hydrogen atom; further preferably an unsubstituted hydrocarbon group having 1 to 6 carbon atoms, an alkylsilyl group having 1 to 6 carbon atoms, or a hydrogen atom; particularly preferably a hydrogen atom.

d is an integer of 1 or greater, and in view of availability of raw materials and ease of production, d is preferably from 1 to 5, more preferably from 1 to 3, further preferably 1 or 2. R¹, A, B, X, a and m each have the same definitions as those in Formula (1) when -A- is a structure represented by Formula (2), and preferred ranges thereof are also the same. When a, d and m are 2 or greater, the two or more of R¹, R⁵ or the group including an ethynyl group represented by B-X in the square brackets may be the same or different, respectively.

A further embodiment of the invention is a condensed polymer, which is a condensate including the acetylene compound according to any of the aforementioned <1> to <10> as a structural unit, and a method of producing the same.
The types of the polymer including the acetylene compound according to any of the aforementioned <1> to <10> as a structural unit include polyamide, polyimide, polyisoimide, polyesterimide, polyetherimide, polyamidoimide, polyamic acid, polyamic acid ester, polyamide, polythioamide, polyurethane, polyurea and polyazomethine; preferably polyimide, polyisoimide, polyesterimide, polyetherimide, polyamidoimide, polyamic acid, polyamic acid ester and polyamide; further preferably polyimide, polyisoimide, polyesterimide, polyetherimide, polyamidoimide and polyamic acid. These polymers may be a homopolymer or a block copolymer. The basic skeleton of the polymer may be an aromatic skeletone or an aliphatic skeleton, and silicone, fluorine or the like may be included in amain chain or a side chain thereof, but the basic skeleton is preferably an aromatic skeleton.

The polymer having the acetylene compound as a strucrural unit can be prepared by causing reaction of the acetylene compound according to any of the aforementioned <1> to <10>; a compound having two of a -CHO group, a -COOH group, a -COOR"" group, a -CSOH group, a -COSH group, a -CSSH group, a -NCO group or a -NSO group; a tetracarboxylic dianhydride; a substituted or unsubstituted hydrocarbon compound having two or more amino groups in the molecule thereof (diamine compound) other than the compound represented by Formula (1); and/or a diol compound. As necessary, a monoaldehyde compound may be used in the reaction. R"" have the same definitions as R' as mentioned above, but is preferably not a hydrogen atom. R"" is preferably a substituted or unsubstituted hydrocarbon group having 1 to 12 carbon atoms, more prefarably an unsubstituted hydrocarbon group having 1 to 8 carbon atoms, further preferably an unsubstituted hydrocarbon group having 1 to 4 carbon atoms.

Examples of the compound having any two of a -CHO group, a -COO group, a -COOR"" group, a -CSOH group, a -COSH group, a -CSSH group, a -NCO group or a -NSO group in the molecule thereof include dialdehydes (such as terephthalaldehyde, isophthalaldehyde, phthalaldehyde, 4-methylphthalaldehyde, 4-methylisophthalaldehyde, 2,5-dimethylterephthalaldehyde, 1,4-cyclohexanedialdehyde, 2-fluoro-1,4-benzenedialdehyde, 3-methoxy-1,4-benzenedialdehyde, 1,6-hexanedialdehyde, 4,4'-dialdehydobiphenyl, 2,2-bis(4-aldehydophenyl)propane, 1,3-diacetylbenzene, 1,4-diacetylcyclohexane); dicarboxylic acids (such as terephthalic acid, isophthalic acid, phthalic acid, 4-methylphthalic acid, 4-methylisophthalic acid, 2,5-dimethylterephthalic acid, 1,4-cyclohexanedicarboxylic acid, 2-fluoro-1,4-benzenedicarboxylic acid, 3-methoxy-1,4-benzenedicarboxylic acid, 1,6-hexanedicarboxylic acid, 4,4'-dicarboxybiphenyl, 2,2-bis(4-carboxyphenyl)propane, bis(4-carboxyphenyl)sulfone, 4,4'-dicarboxybenzophenone, 4,4'-dicarboxybiphenyl ether, 3,3'-dicarboxybiphenyl, 2,2-bis(3-carboxyphenyl)propane, bis(3-carboxyphenyl)sulfone; 3,3'-dicarboxybenzophenone, 4,4'-dicarboxy-3,3'-dimethylbiphenyl ether, 4,4'-dicarboxy-3,3'-dimethylbiphenyl, 2,2-bis(4-carboxy-3-methylphenyl)propane, bis(4-carboxy-3-methylphenyl)sulfone, 4,4'-dicarboxy-3,3'-dimethylbenzophenone, 4,4'-dicarboxy-3,3'-dichlorobiphenyl, 2,2-bis(4-carboxy-3-chlorophenyl)propane, bis(4-carboxy-3-chlorophenyl)sulfone, 4,4'-dicarboxy-3,3'-dichlorobenzophenone, malonic acid, ethylmalonic acid, maleic acid, succinic acid, 2,2-diethyl succinic acid, 2,3-dimethyl succinic acid, adipic acid, azelaic acid, sebacic acid, lutidinic acid, and dipicolinic acid);

diesters (such as dimethyl isophthalate, diethyl isophthalate, dimethyl terephthalate, dimethyl phthalate, diethyl 4-methylphthalate, dimethyl 4-menthyl isophthalate, dimethyl 2,5-dimethylterephthalate, dimethyl 1,4-cyclohexanedicarboxylate, diethyl 1,4-cyclohexanedicarboxylate, dimethyl 2-fluoro-1,4-benzenedicarboxylate, dimethyl 3-methoxy-1,4-benzenedicarboxylate, dimethyl 1,6-hexanedicarboylate, 4,4'-dimethoxycarbonylbiphenyl, 2,2-bis(4-methoxycarbonylphenyl)propane, 2,2-bis(4-ethoxycarbonylphenyl)propane, bis(4-methoxycarbonylphenyl)sulfone, 4,4'-dimethoxycarbonylbenzophenone, 4,4'-dimethoxycarbonylbiphenyl ether, 3,3'-dimethoxycarbonylbiphenyl, 2,2-bis(3-methoxycarbonylphenyl)propane, bis(3-methoxycarbonylphenyl)sutfone, 3,3'-dimethoxycarbonylbenzophenone, 4,4-dimethoxycarbonyl-3,3'-dimethylbiphenyl ether, 4,4'-dimethoxycarbonyl-3,3'-dimethylbiphenyl, 2,2-bis(4-methoxycarbonyl-3-methylphenyl)propane, bis(4-methoxycarbonyl-3-methylphenyl)sulfone, 4,4'-dimethoxycarbonyl-3,3'-dimethylbenzophenone, 4,4'-dimethoxycarbonyl-3,3'-dichlorobiphenyl, 2,2-bis(4-methoxycarbonyl-3-chlorophenyl)propane, bis(4-methoxycarbony)-3-chlorophenyl)sulfone, 4,4'-dimethoxycarbonyl-3,3'-dichiorobenzophenone, dimethyl malonate, diethyl malonate, dimethyl ethylmalonate, dimethyl maleate, dimethyl succinate, diethyl succinate, dimethyl 2,2-dimethylsuccinate, dimethyl 2,3-dimethylsuccinate, dimethyl adipate, diethyl adipate, dimethyl azelate, dimethyl sebacate, dimethyl lutidinate and dimethyl dipicolinate);

dithiocarboxylic acids (such as hexanedithiocarboxylic-s-acid and hexane dithiocarboxylic acid); diisocyanates (such as trilene diisocyanate, diphenylmethane diisocyanate, hexamethylene diisocyanate and 1,4-cyclohexane dicyanate); dithioisocyanates (such as 1,4-phenylene dithioisocyanate, 1,3-phenylene dithioisocyanate, 1,4-cyclohexyl dithioisocyanate and 5-methyl-1.3-phenylene dithioisocyanate).

Examples of the diamine compound other than the compound represented by Formula (1) that can be used for the polymer according to the invention are not particularly limited, but include the following diamine compounds.
p-phenylenediamine, m-phenylenediamine, o-phenylenediamine, 1,4-diamino-2-methylbenzene, 1,3-diamino-4-methyl-benzene, 1,3-diamino-4-chloro-benzene, 1,3-diamino-4-acetylamino-benzene, 1,3-bisaminoethyl-benzene, hexamethylenediamine, 3,3'-diaminobiphenyl, 4,4'-diamino-3,3'-dimethylbipheyl, 4,4'-diamino-3,3'-dichlorobiphenyl, 2,2'-difluoro-4,4'-diaminobiphenyl, 3,3'-difluoro-4,4'-diaminobiphenyl, 2,2'-difluoro-5,5'-diaminobiphenyl; 3,3'-difluoro-5,5'-diaminobiphenyl, 2,2'-dichloro-4,4'-diaminobiphenyl, 3,3'-dichloro-4,4'-diaminobiphenyl, 2,2'-dichloro-5,5'-diaminobiphenyl, 3,3'-dichloro-5,5'-diaininobiphenyl , 2,2-dibromo-4,4'-diamanobiphenyl, 3,3'-dibromo-4,4'-diaminobiphenyl, 2,2'-dibromo-5,5'-diaminobiphenyl, 3,3'-dibromo-5,5'-diaminobiphenyl, 2,2-bis(trifluoromethyl)-4,4'-diaininobiphenyl, 3,3'-bis(trifluoromethyl)-4,4'-diaminobiphenyl, 2,2'-bis(trifluoromethyl)-5,5'-diaminobiphenyl, 3,3'-bis(trifluoromethyl)-5,5'-diaminobiphenyl, 2,2'-bis(trichloroinethyl)-4,4'-diaminobiphenyl, 3,3'-bis(trichloromethyl)-4,4'-diaminobiphenyl, 2,2'-bis(trichloromethyl)-5,5'-diaminobiphenyl, 3.3'-bis(trichloromethyl)-5,5'-diaminobiphenyl, 2;2'-bis(tribromomethyl)-4,4'-diaminobiphenyl, 3,3'-bis(tribromomethyl)-4,4'-diaminobiphenyl, 2,2'-bis(tribroinomethyl)-5,5'-diaminobiphenyl, 3,3'-bis(tribromoinethyl)-5,5'-dianlinobiphenyl, 3,3'-diaminodiphenyl ether, 3,4'-diaminodiphenyl ether, 4,4'-diaminodiphenyl ether, 4,4'-diamino-3,3'-dimethylbiphenyl ether, 3,3'-diaminodiphenylsulfide, 3,4'-diaminodiphenylsulfide, 4,4'-diaminodiphenylsulfide, 3,3'-diaminodiphenylsulfone; 3,4'-diaminodiphenylsulfone, 4,4'-diaminodiphenylsulfone, bis(4-amino-3-methylphenyl)sulfone, bis(4-amino-3-chlorophenyl)sulfone, bis(4-aminophenyl)sulfone, bis(3-aminophenyl)sulfone, bis(5-fluoro-4-aminoplienyl)sulfone, bis(5-fluoro-3-aininophenyl)sulfone, bis(5-chloro-4-aminophenyl)sulfone, bis(5-chloro-3-aminophenyl)sulfone, bis(5-bromo-4-aminophenyl)sulfone, bis(5-bromo-3-aminophenyl)sulfone, bis(5-trifluoromethyl-4-aminophenyl)sulfone, bis(5-trifluoromethyl-3-aminophenyl)sulfone, bis(5-trichloromethyl-4-aminophenyl)sulfone, bis(5-trichloromethyl-3-aminophenyl)sulfone, bis(5-tribromomethyl-4-aminophenyl)sulfone, bis(5-tribromomethyl-3-aminophenyl)sulfone, 3,3'-diaminobenzophenone, 4,4'-diaminobenzophenone, 3,4'-diaminobenzophenone, 4,4'-diamino-3,3'-dimethylbenzophenone, 4,4'-diamino-3,3'-dichlorobenzophenone, 3,3'-diaminodiphenylmethane, 4,4'-diaminodiphenylmethane, 3,4'-diaminodiphenylmethane, 2,2-di(3-aminophenyl)propane, 2,2-di(4-aminophenyl)propane, 2-(3-aminophenyl)-2-(4-aminophenyl)propane, 2,2-di(3-aminophenyl)-1,1,1,3,3,3-hexafluoropropane, 2,2-di(4-aminophenyl)-1,1,1,3,3,3-hexafluoropropane, 2-(3-aminophenyl)-2-(4-aminophenyl)-1,1,1,3,3,3-hexafluoropropane, 2,2-bis(4-amino-3-methylphenyl)propane, 2,2-bis(4-amino-3-chlorophenyl)propane,

1,1-di(3-aminophenyl)-1-phenylethane; 1,1-di(4-aminophenyl)-1-phenylethane,1-(3-aminophenyl)-1-(4-aminophenyl)-1-phenylethane, 1,3-bis(3-aminophenoxy)benzene, 1,3-bis(4-aminophenoxy)benzene, 1,4-bis(3-aminophenoxy)benzene, 1,4-bis(4-aminophenoxy)benzene, 1,3-bis(3-aminobenzoyl)benzene, 1,3-bis(4-aminobenzoyl)benzene, 1,4-bis(3-aminobenzoyl)benzene, 1,4-bis(4-aminobenzolyl)benzene, 1,3-bis(3-amino-α,α-dimethylbenzyl)benzene,11,3-bis(4-amino-α,α-dimethylbenzyl)benzene, 1,4-bis(3-amino-α,α-dimethylbenzyl)benzene, 1,4-bis(4-ainino-α,α-dimethylbenzyl)benzene, 1,3-bis(3-amino-α,α-ditrifluoromethylbenzyl)benzene, 1,3-bis(4-amino-α,α-ditrifluoromethylbenzyl)benzene, 1,3-bis(4-amino-α,α-ditrifluoromethylbenzyl)benzene, 1,4-bis(4-amino-α,α-ditrifluoromethylbenzyl)benzene, 2,6-bis(3-aminophenoxy)benzonitrile, 2,6-bis(3-aminophenoxy)pyridine,

4,4'-bis(3-aminophenoxy)biphenyl, 4.4'-bis(4-aminophenoxy)biphenyl, bis[4-(3-aminaphenoxy)phenyl]ketone, bis[4-(4-aminophemoxy)phenyl]ketone, bis[4-(3-aminophenoxy)phenyl]sulfide, bis[4-(4-aminophenoxy)phenyl]sulfide, bis[4-(4-aminophenoxy)phenyl]sulfone, bis[4-(3-aminophenoxy)phenyl]sulfone, bis[4-(5-fluoro-4-aminophenoxy)phenyl]sulfone, bis[4-(5-fluoro-3-aminophenoxy)phenyl]sulfone, bis[4-(5-chloro-4-aminophenoxy)phenyl]sulfone, bis[4-(5-chloro-3-aminophenoxy)phenyl]sulfone, bis[4-(5-bromo-4-aminophenoxy)phenyl]sulfone, bis[4-(5-bromo-3-aminophenoxy)phenyl]sulfone, bis[4-(5-trifluoromethyl-4-aminophenoxy)phenyl]sulfone, bis[4-(5-trifluoromethyl-3-aminophenoxy)phenyl]sulfone, bis[4-(5-trichloromethyl-4-aminophenoxy)phenyl]sulfane, bis[4-(5-trichloromethy1-3-aminophenoxy)phenyl]sulfone, bis[4-(5-tribromomethyl-4-aminophenoxy)phenyl]sulfone, bis[4-(5-tribi-omomethyl-3-aminophenoxy)pheulfone, bis[4-(3-aminophenoxy)phenyl] ether, bis[4-(4-aminophenaxy)phenyl] ether, bis[4-(4-aminophenoxy)phenyl]methane, 2,2-bis[4-(3-aminophenoxy)phenyl]propane, 2,2-bis[4-(4-aminophenoxy)phenyl]propane, 2,2-bis[3-(3-aminophenoxy)phenyl]-1,1,1,3,3,3-hexafluoropropane, 2,2-bis[4-(4-aminophenoxy)phenyl]-1,1,1,3,3,3-hexafluoropropane,

1,3-bis[4-(3-aminophenoxy)benzoyl]benzene, 1,3-bis[4-(4-aminophenoxy)benzoyl]benzene, 1,4-bis[4-(3-aminophenoxy)benzoyl]benzene, 1,4-bis[4-(4-aminophenoxy)benzoyl]benzene; 1,3-bis[4-(3-aminophenoxy)-α,α-dimethylbenzyl]benzene, 1,3-bis[4-(4-aminophenoxy)-α,α-dimethylbenzyl]benzene, 1,4-bis[4-(3-aminophenoxy)-α,α-dimethylbenzyl]benzene, 1,4-bis[4-(4-aminophenoxy)-α,α-dimethylbenzyl]benzene,

4,4-bis[4-(4-aminophenoxy)benzoyl]diphenyl ether, 4,4'-bis[4-(4-amino-α,α-dimethylbenzyl)phenoxy]benzophenone, 4,4'-bis[4-(4-amino-α,α-dimethylbenzyl)phenoxy]diphenylsulfone, 4,4'-bis[4-(4-aminophenoxy)phenoxy]diphenylsulfone, 3,3'-diamino-4,4'-diphenoxybenzophenone, 3,3'-diamino-4,4'-dibiphenoxybenzophenone, 3,3'-diamino-4-phenoxyhenzophenone, 3,3'-diamino-4-biphenaxybenzophenone.

6,6'-bis(3-aminophenoxy)3,3,3',3'-tetramethy1-1'-spirobiindane, 6,6'-bis(4-aminophenoxy)3,3,3',3'-tetramethyl-1,1'-spirobiindane; 1,3-bis(3-aminopropyl)tetramethyldisiloxane, 1,3-bis(4-aminobutyl)tetramethyldisiloxane, α,ω-bis(3-aminopropyl)polydimthylsiloxane, α,ω-bis(3-aminobutyl)polydimethylsiloxane, and diaminopolysiloxane.

These diamine compounds as illustrated above may be used alone or as a mixture. Further, the hydrogen atoms on the aromatic ring of these diamine compounds may be partly or totally substituted by a substituent selected from a fluorine atom, a methyl group, a methoxy group, a trifluoromethyl group, or a trifluoromethoxy group. In order to introduce a branched structure, part of the diamine compound may be changed to triamines or tetraamines. Specific examples of the triamines include pararosaniline.

Examples of the tetracarboxylic dianhydride that can be used in the polymer according to the invention is not particularly limited, but include the following compounds.
Pyromellitic dianhydride, 3-filuoropyromellitic dianhydride, 3-chloropyromellitic dianhydride, 3-bromopyromellitic dianhydride, 3-trifluoromethylpyromellitic dianhydride, 3-trichloromethylpyromellitic dianhydride. 3-tribromomethylpyromellitic dianhydride, 3,6-difluoropyromellitic dianhydride, 3,6-dichloropyromellitic dianhydride, 3,6-dibromopyromellitic dianhydride, 3,6-bistrifluoromethylpyrornellitic dianhydride, 3,6-bistrichloromethylpyroincllitic dianhydride, 3,6-bistribromoinethylpyromellitic dianhydride, 2,2',3,3'-biphenyltetracarboxylic dianhydride, 3,3',4,4'-biphenyltetracarboxylic dianhydride, 2,2',3,3'-benzophenonetetracarboxylic dianhydride, 3,3',4,4'-benzophenonetetracarboxylic dianhydride, bis(2,3-dicarboxyphenyl) ether dianhydride, bis(3,4-dicarboxyphenyl) ether dianhydride, bis(2,3-dicarboxyphenyl)sulfide dianhydride, bis(3,4-dicarboxyphenyl)sulfide dianhydride, bis(2,3-dicarboxyphenyl)sulfone dianhydride, bis(3,4-dicarboxyphenyl)sulfone dianhydride, 2,2-bis(2,3-dicarboxyphenyl)propane dianhydride, 2,2-bis(3,4-dicarboxyphenyl)propane dianhydride, 2,2-(bis(2,3-dicarboxyphenyl)-1,1,1,3,3,3-hexafluoropropane dianhydride, 2,2-bis(3,4-diearboxyphenyl)-1,1,1,3,3,3-hexafluoropropane dianhydride, 1,3-bis(3,4-dicarboxyphenoxy)benzene dianhydride, 1,3-bis(2,3-dicarboxyphenoxy)benzene dianhydride, 1,4-bis(2.3-diearboxyphenoxy)benzene dianhydride, 1,4-bis(3,4-dicarboxyphenoxy)benzene dianhydride, 4,4'-bis(3,4-dicarboxyphenoxy)biphenyl dianhydride, 2,2-bis[(3,4-dicarboxyphenoxy)phenyl]propane dianhydride, 9,9-bis(3,4-dicarboxyphenyl)fluorene dianhydride, 9,9-bis[4-(3,4-dicarboxyphenoxy)phenyl]fluorene dianhydride, 4,4'-biphenylenebis(trimellitic acid anhydride monoester), p-phenylenebis(trimellitic acid anhydride monoester), p-methylphenylenebis(trimellitic acid anhydride monoester), p-(2,3-dimethylphenylene)bis(trimellitic acid monoester acid anhydride), 1,4-naphthalenebis(trimellititc acid monoester acid anhydride), 2,6-naphthalenebis(trimellitic acid anhydride monoester), 2,2-bis[4-(trimellitic acid anhydride monoester)phenyl]propane, 2,2-bis[4-(trimellitic acid anhydride monoester)phenyl]hexafluoropropane, 1,2,5,6-naphthalenetetracarboxylic dianhydride, 2,3,5,6-pyridinetetracarboxylic dianhydride, 3,4,9,10-perylenetetracarboxylic dianhydride, 1,3-bis(3,4-dicarboxyphenyl)-1,1,3,3-tetramethyldisiloxane dianhydride, 1-(2,3-dicarboxyphenyl)-3-(3,4-dicarboxyphenyl)-1,1,3,3-tetramethyldisiloxane dianhydride, 1,2,5,6-naphthalenetetracarboxylic dianhydride, 2,3,6,7-naphthalenetetracarboxylic dianhydride, 1,4,5,8-naphthalenetetracarboxylic dianhydride, ethyelnetetracarboxylic dianhydride, butanetetracarboxylic dianhydride, and cyclopentanetetracarboxylic dianhydride.

These tetracarboxylic dianhydrides as illustrated above may be used alone or as a mixture. Further, the hydrogen atoms on the aromatic ring of these tetracarboxylic dianhydrides may be partly or totally substituted by a substituent selected from a fluorine atom, a methyl group, a methoxy group, a trifluoromethyl group, or a trifluoromethoxy group.
In order to introduce a branched structure, part of the tetracarboxylic anhydride may be changed to h.exacas-boxylic trianhydrides or octacarboxylic tetraanhydrides.

Examples of the diol compound that can be used in the polymer according to the invention include, but not limited thereto, the following compounds.
4,4'-dihydroxybiphenyl, 2,2-bis(4-hydroxyphenyl)propane, 2,2-bis(4-hydroxyphenyl)-1-phenylpropane, bis(4-hydroxyphenyl)sulfone, 4,4'-dihydroxybenzophenone, 4,4'-dihydroxybiphenyl ether, 3,3'-dihydroxybiphenyl, 2,2-bis(3-hydroxyphenyl)propane, bis(3-hydroxyphenyl)sulfone, 3,3'-dihydroxybenzophenone, 4,4'-dihydroxy-3,3'-dimethylbiphenyl ether, 4,4'-dihydroxy-3,3'-dimethylbiphenyl, 2,2-bis(4-hydroxy-3-methylphenyl)propane, bis(4-hydroxy-3-methylphenyl)sulfone, 4,4'-dihydroxy-3,3'-dimethylbenzophenone, 4,4'-dihydroxy-3,3'-dichlarobiphenyl; 2,2-bis(4-hydroxy-3-clalorophenyl)propane, bis(4-amino-3-chlorophenyl)sulfone, 4,4'-diamino-3,3'-dichlorobenzophenone, 1,4-dihydroxybenzene, 1,3-dihydroxybenzene, 1,4-dihydroxy-2-methylbenzene, 1,3-dihydroxy-4-methyl-benzene, 1,3-dihydroxy-4-chloro-benzene, 1,3-dihydroxy-4-acetoxy-benzene, 1,3-bishydroxyethyl-benzene, ethylene glycol, diethylene glycol, propylene glycol, dipropylene glycol, butanediol, hexanediol, cyclohexanediol, 1,6-bishydroxymethylcyclohexane, and neopentyl glycol.

Example of the monoaldehyde compound that can be used in the polymer according to the invention include, but not limited thereto, formaldehyde, acetaldehyde, trioxane, propionaldehyde and benzaldehyde. Among these, formaldehyde and acetaldehyde are preferred.

Among the above compounds, in view of availability of raw materials and the wide application thereof, the structural unit used in the polymer including an acetylene compound according to the invention is preferably dicarboxylic acids, diesters, diisocyantes, tetracarboxylic dianhydrides, diamines or diols; more preferably dicarboxylic acids, diesters, tetracarboxylic dianhydrides, diamines or diols; further preferably dicarboxylic aids, diesters, tetracarboxylic dianhydrides, diamines or diols; and particularly preferably tetracarboxylic dianhydrides, diamines, diols or diesters.

When the condensed polymer including an acetylene compound as a structural unit according to the invention is prepared and derived by reacing the acetylene compound according to any of the aforementioned <1> to <10> with a compound having a -COOH group, a -CSOH group, a -COSH group or a -CSSH group in the molecule thereof, exemplay methods of producing the same include a method of convering the above compound to an intermediate that is highly active with respect to condensation reaction, and then reacting this intermediate with the acetylene compound according to any of the aforementioned <1> to <10>; and a method of directly performing condensation or addition of the acetylene compound according to any of the aforementioned <1> to <10>, a compound having two groups selected from a -CHO group, a -COOH group, a -COOR"" group, a -CSOH group, a -COSH group, a -CSSH group, a -NCO group or a -NSO group, a tetracarboxylic dianhydride, and a substituted or unsubstituted hydrocarbon compound having two or more amino groups in the molecule thereof, and/or a diol compound, under the presence of a catalyst. As nececessary, a monoaldehyde compound is used in the reaction.

The method of producing the polymer according to the invention is not particularly limited, but can be prepared by using the aforementioned acetylene compound, and the aforementioned monomer or a mixture thereof.
For example, the polyimide-based polymer according to the invention can be produced by a mehtod of imidizing the raw material via a polyamic acid, and closing the ring thereof; a method via a polyisoimide, or a method of partially imidizing the raw material and forming a block polyimide via a polyamic acid. However, the invention is not particularly limited to these methods. Known polymerization methods that can be used include a method of dispersing an acid anhydride in an organic solvent in which an amine compound such as a diamine is dissolved, stirring the same to completely dissolve these components, and then polymerizing the same; a method of dissolving or dispersing an acid anhydride in an organic solvent, and then polymerizing the same using an amine compound; and a method of reacting a mixture of an acid anhydride and an amine compound in an organic solvent, and then polymerizing the same.
In the process of imidization, water is generated as a result of cyclization of the polyamic acid. This water is preferably removed from the reaction system by forming an azeotrope with benzene, xylene, tetralin or the like, in order to promote the imidization. The imidization reaction can be further promoted by using a dehydration agent such as an aliphatic acid anhydride (such as acetic anhydride) or an aromatic acid anhydride.

As necessary, a poly condensation promoter can be added to the reaction system in order to rapidly bring the reaction to completion. Examples of the polycondensation promotor catalyst include a basic polycondensation promotor and an acidic polycondensation promoter, which may be used in combintaion. Exemplary basic polyconensation promotors include N,N-dimethylaniline, N.N-diethylamline, pyridine, quinoline, isoquinoline, α-picoline, β-picoline, γ-picoline, 2,4-lutidine, triethylamine, tributylamine, tripentylamine, N-methylmorpholine, diazabicycloundecene, and diazabicyclononene. Among these, in view of availability and the ability of promoting reaction, diazabicylcoundecene, diazabicyclononene, methylpyridine, pyridine and triethylamine are preferred, and pyridine, and triethylamine are more preferred. Exemplary acidic polycondensation promotors include benzoic acid, o-hydroxybenzoic acid, m-hydroxybenzoic acid, p-hydroxybenzoic acid, 2,4-dihydroxybenzoic acid, p-hydroxyphenylacetic acid, 4-hydroxyphenylpropionic acid, phosphoric acid, p-phenylsulfonic acid, p-toluenesulfonic acid, and crotonic acid.

The amount of the polycondensation promotor to be used is from 1 to 50 mol%, preferably from 5 to 35 mol%, with respect to the amine or diamine component including the compound represented by Formula (1). By using the polycondensation promotor, the reaction temperature can be lowered. As a result, side reaction due to heating, which is regarded as a cause of coloration, can be prevented, and the reaction time can be significantly reduced to improve cost efficiency.
The temperature for the polymerization of polyamic acid is preferably 60°C or less, more preferably 40°C or less, in view of efficient reaction and ease of increasing the viscosity of polyamic acid.
The molecular weight of the polymer including an acetylene compound as a structural unit according to the invention is not particulalry limited, but is from 300 to 1.000,000, preferably from 500 to 200,000, further preferably from 1,000 to 50,000 in terms of weight average molecular weight, in view of handleability, curability and the like. In the present specification, a polymer having a molecular weight of about 10,000 or less may be referred to as an oligomer.

Exemplary solvents that can be used for the production of the polymer include aprotic solvents, including ureas such as tetramethylurea and N,N-dimethylethylurea, sulfoxides and sulfones such as dimethylsulfoxide, diphenylsulfone and tetramethylsulfone, amides such as N,N-dimethylacetamide (DMAc), N,N-dimethylformamide (DMF), N,N'-diethylacetamide, N-methyl-2-pyrolidone (NMP), γ-butyllactone and hexamethylphosphoric acid triamide, and phosphorylamides; alkyl halides such as chloroform and methylene chloride; aromatic hydrocarbons such as benzene and toluene; phenols such as phenol and cresol; and ethers such as diemethyl ether, diethyl ether and p-cresyl methyl ether. These solvents are typically used alone, but may be used in combination of two or more kinds as necessary. Among these solvents, amides such as DMF, DNAc and NMP are preferably used.

Among the methods as mentioned above, a method of converting the compound to an intermediate that is highly active with resepct to condensation reaction, and then reacting this intermediate with the acetylene compound according to any of aforementioned <1> to <10> is preferred, in view of reactivity and preventing decomposition or reaction of ethyneyl groups of the intended product.

The polymer including an acetylene compound as a structural unit according to the invention may also be suitably produced by referring to a method described in Shin Kobunshi Jikken Gaku. (edited by the Society of Polymer Science, Japan, published by Kyoritu Shuppan Co., Ltd.) or Jikken Kagaku Koza, Vol. 28 (edited by the Chemical Society of Japan, published by Maruzen Co., Ltd.)

A further embodiment of the invention is a method of producing an acetylene compound represented by Formula (1), where -B- is a structure represented by Formula (3), by protecting the amino group of the acetylene compound represented by the following Formula (5), converting the same to a compound represented by Formula (6), and then subjecting this compound and a compound having an acetylene group represented by Formula (7) to condensation reaction.

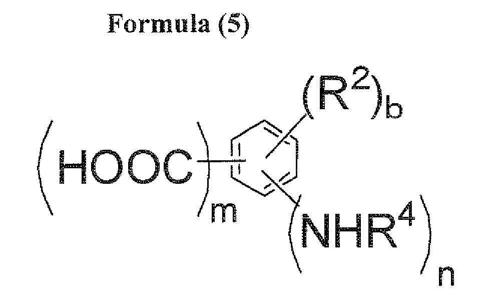

In Formula (5), R², b and m each have the same definitions as R², b and in in Formula (2), where -A- is a structure represetned by Formula (2), and the preferred ranges thereof are also the same.

Examples of the compound represented by Formula (5) include aminobenzoic acids (such as 3-aminobenzoic acid, 4-aminobenzoic acid, 4-aminobenzoic acid hydride, 5-aminobenzoic acid hydride, 2-methyl-3-aminobenzoic acid, 2,6-diemthyi-4-ammobenzoic acid, 2-phenyl-5-ammobenzoic acid, 4-fluoro-5-aminobenzoic acid, 3-aminobenzoic acid hydrochloride, 4-aminobenzoic acid hydrochloride, 2-methyl-5-aminobenzoic acid methanesulfonate, 2,6-dimethyl-4-aminobenzoic acid oxalate, and 2-phenyl-5-aminobenzoic acid sulfate); aminophthalic acids (such as 4-aminophthalic acid, 5-aminoisophthalic acid, 2-aminoterephthalic acid, 2-aminoisophthalic acid, 2-amino-5-methylterephthalie acid, 2-amino-5-methoxyterephthalic acid, 2-amino-5-cyclohexylterephthalic acid, 5-amino-4-methylisophthalic acid, 5.amino-4-methoxyisophthalic acid, 5-amino-4-fluoroisophthalic acid, 4-amino-5-methylphthalic acid, 4-amino-5-inethoxyphthatic acid, and 4-amino-5-ethoxyphthalic acid);
diaminobenzoic acids (such as 3,5-diaminobenzoic acid, 3,4-diaminobenzoic acid, 3,5-bis(N-methylamino)benzoic acid, 3,4-bis(N-methylamino)benzoic acid, 3,5-diaminobenzoic acid dihydride, 3,4-diaminobenzoic acid dihydride, 2-methyl-3,5-diaminobenzoic acid, 2,6-dimethyl-3,5-diaminobenzoic acid, 2,6-dimethyl-3,5-bis(N-methylamino)benzoic acid, 2-phenyl-3,5-diaminobenzoic acid, 4-fluoro-3,5-diaminobenzoic acid, 3,5-diaminobenzoic acid hydrochloride, 3,4-diaminobenzoic acid hydrochloride, 2-methyl-3 ,5-diaminobcnzoic acid methane sulfonate, 2,6-dimethyl-3,5-diaminobenzoic acid oxalate, and 2-phenyl-3,5-diaminobenzoic acid sulfate);
triaminobenzoic acids (such as 2,4,6-triaminobenzoic acid);
tetraaminobenzoic acids (such as 2,3,5,6-tetraaminobenzoic acid); and
diaminophthalic acids (such as 4.5-diaminophthalic acid).
Among these compounds, 3-aminobenzoic acid, 4-aminobenzoic acid, 5-aminobenzoic acid, 4-aminophthalic acid, 5-aminoisophthalic acid, 2-aminoterephthalic acid, 3,5-diaminobenzoic acid and 3,4-diaminobenzoic acid are preferred; and 3-aminobenzoic acid, 4-aminophthalic acid, 5-aminoisophthalic acid, 2-aminoterephthalic acid and 3,5-diaminobenzoic acid are more preferred.

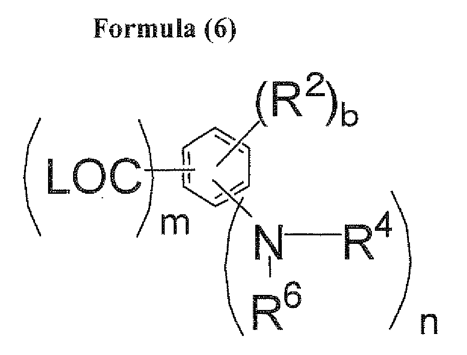

The compound represented by Formula (6) is derived from the compound represented by Formula (5), and R⁶ represents a functional group that can be used as a protective group for an amino group. Specifically, any compounds described as a protective group for an amino group in Non-patent Document 4 (Protective Groups in Organic Synthesis) may be used, and examples thereof include an acetyl group, a benzyloxycarbonyl (BOM) group, a 2-(trimethylsilyl)ethoxycarbonyl (TEOC) group, a t-butoxycarbonyl (Boc) group, an allyloxycarbonyl (AOC) group, a 2,2,2-trichloroethoxycarbonyl (Troc) group, a 9-fluorenylmethoxycarbonyl (FMOC) group, a tosyl (Ts) group, and a mesyl (Ms) group. Among these, a t-butoxycarbonyl group and an acetyl group are preferred.

L represents a monovalent leaving group, and this leaving group is not particularly limited as long as it can be a substituent of the nitrogen atom or an oxygen atom by reaction with an amino group or a hydroxy group. Preferred examples of the monovalent leaving group include a halogen atom (such as a fluorine atom, a chlorine atom, a bromine atom or an iodine atom), a sulfonate group (such as a mesylate group, a tosylate group and a Inflate group), a methanesulfonyl group, an alkoxycarbonyl group, a diazonium group, and a trialkylammonium group (such as a trimethylammonium group). Among these, a halogen atom, a methanesulfonyl group, a sulfonate group and an alkoxycarbonyl group are more preferred, and a halogen atom and a methanesulfonyl group are further preferred. R², b and m have the same definitions as R², b and m in Formula (1) when -A- is a structure represented by Formula (2), and the preferred ranges thereof are also the same.

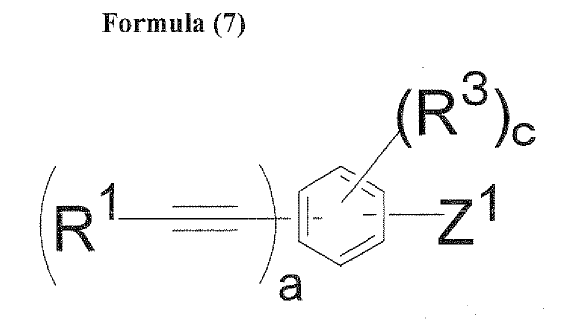

In Formula (7), Z¹ represents -OH or -NHR (R has the same definitions as R in Formula (1)). R¹, R³, a and c have the same definitions as R¹, R³, a and c in Formula (1) when -A- is a structure represented by Formula (2), and the preferred ranges thereof are also the same.

Examples of the compound represented by Formula (7) include anilines (such as m-ethynylaniline, p-ethynylaniline, o-ethynylaniline, 5-ethynyl-2-methylaniline, 3-ethynyl-4-metliyianiline, 5-ethynyl-3-fluoroaniline, 3-ethynyl-4-fluoroaniline, 3-ethynyl-4-methoxyaniline, 3-ethynyl-4-ethoxyaniline, 2,6-dimethyl-4-ethynylaniline, 2,3-diethynylaniline, 3,4-diethynylaniline, 3,5-diethynylaniline, 3,6-diethynylaniline, 2,4,6-triethynylaniline, m-propynytanilrne, m-butynyianiline, m-hexynylaniline, m-dodecyiethynylaniiine, m-t-butylethynylaniline, m-cyclhexylethynylaniline, m-3-pyridylethynylanilne, m-2-pyridylethynylanillne, m-naphthylethynylaniline, m-quinolinylethynylaniline, m-(3-hydroxy-3-methyl-1-buthynyl)aniline, 3-(3-hydroxy-3-methyl-1-butynyl)-5-methylaniline, m-trimethylsilylethynylaniline, m-ethynyltoluidine, p-ethynyltoluidine, o-ethynyl-p-chloroaniline, 2,3-diethynyl-5-methylaniline, 3,4-diethynyltoluidine, 3,5-diethynyltoluidine, 4-chloro-3,6-diethynylaniline, m-propynyltoluidine, m-butynyltoluidine, m-hexynyltoluidine, 3-dodecylethynyl-5-methoxyaniline, 3-t-butylethynyl-5-chloroaniline, 3-cyclohexylethynyl-5-chloroaniline, m-(2-hydroxypropyl-2-ethynyl)toluidine, and m-trimetliylsilylethynyltoluidine);
N-methyl-m-ethynylaniline, N-methyl-p-ethynylaniline, N-methyl-5-ethynyl-2-methylaniline, N-methyl-5-ethynyl-3-fluoroaniline, N-methyl-3-ethynyl-4-methoxyaniline, N-ethyl-3-ethynyl-4-ethoxyaniline, N-methyl-2,6-dimethyl-4-ethynylaniline, N-methyl-3,5-diethynylaniline, N-methyl-2,4,6-triethynylaniline, N-methyl-m-propynylaniline, N-methyl-m-butynylaniline, N-methyl-m-t-butylethynylaniline, N-methyl-m-cyclohexylethynylaniline, N-methyl-m-3-pyridylethynylaniline, in-(3-hydroxy-3-inethyl-1-butynyl)-N-methyl-aniline, N-methyl-m-trimethylsilylethynylaniline, N-methyl-m-ethynyltoluidine, N-methyl-2,3-diethynyl-5-methylaniline, N-methyl-3,5-diethynyltoluidine, N-methyl-m-butynyltoluidine, N-methyl-m-(2-hydroxypropy-1-2-ethynyl)toluidine, and N-methyl-m-trimethylsilylethynyltoluidine);

phenols (such as m-ethynytphenol, p-ethynylphenol, o-ethynylphenol, 5-ethynyl-2-methylphenol, 3-ethynyl-5-fluorophenol, 2,3-diethynylphenol, 3,4-diethynylphenol, 3,5-diethynylphenol, 3,6-diethynylphenol, 2,4,6-triethynylphenol, m-propynylphenol, m-butynylphenol, m-hexynylphenol, m-dodecylethynylphenol, m-t-butylethynylphenol, m-cyclohexylethynylphenol, m-3-pyridylethynylphenol, m-2-pyridylethynylphenol, m-naphthylethynyliphenol, m-quinolinylethynylphenol, m-(2-hydroxypropyl-2-ethynyl)phenol, m-trimethylsilylethynylphenol, m-ethynylcresol, p-ethynylcresol, o-ethynyl-p-chlorophenol, 3-ethynyl-4-methylphenol, 3-ethynyl-4-methoxyphenol, 3-ethynyl-4-ethoxyphenol, 3-ethynyl-4-fluorophenol, 4-ethynyl-2,6-dimethylphenol, 2,3-diethynyl-5-methylphenol, 3,4-diethynylphenol, 3,5-diethynylphenol, 4-chloro-3,6-diethynylphenol, m-propynylcresol, m-butynylcresol, m-hexynylcresol, 3-dodecylethynyl-5-methoxyphenol, 3-t-butylethynyl-5-chlorophenol, 3-cyclohexylethynyl-5-chlorophenol, m-(2-hydroxypropyl-2-ethynyl)ceresol, m-trimethylsilylethynylcresol, and m-(3-hydroxy-3-methyl-1-butynyl)phenol),

Among these compounds, in view of availability of raw materials and reacrivity, m-ethynylaniline, p-ethynylaniline, o-ethynylaniline, 2,3-diethynylaniline, 3,4-diethynylaniline, 3,5-diethynylaniline, 3,6-diethynylaniline, m-propynylaniline, m-hexynylaniline, m-t-butylethynylaniline, m-cyclohexylethynylaniline, m-3-pyridylethynylaniline, m-trimethylsilyiethynylaniline, m-ethynyltoluidine, m-(3-hydroxy-3-methyl-1-butynyl)aniline, m-ethynylphenol, p-ethynylphenol, o-ethynylphenyl, 2,3-diethynylphenol, 3,4-diethynylphenol, 3,5-diethynylphenol, 3,6-diethynylphenol, m-propynylphenol, m-hexynylphenal, m-t-butylethynylphenol, m-cyclohexylethynylphenyol, m-3-pyridylethynylphenol, m-trimethylsilynethynylphenol, m-ethynylcresol, and m-(3-hydroxy-3-methyl-1-butynyl)phenol are preferred; and m-ethynylaniline, p-ethynylaniline, 3,4-diethylaniline, 3,5-diethynylaniline, m-propynylaniline, m-cyclohexylethynylaniline, m-(3-hyd-roxy-3-methyl-1-butynyl)aniline, m-ethynylphenol, p-ethynylphenol, 3,4-diethynylphenol, 3,5-dicthynylphenol, m-propynylphenol, m-cyclohexylethynylphenol, and m-(3-hydroxy-3-methyl-1-butynyl)phenol are particularly preferred.

The following are specific examples of the acetylene compound according to the invention. However, the invention is not limited thereto.

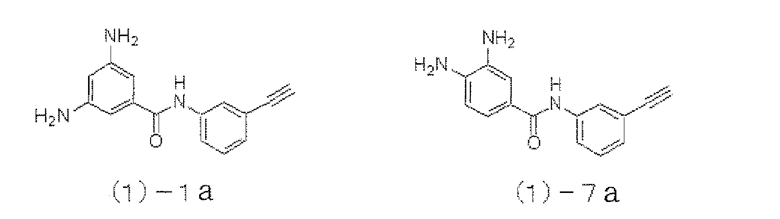

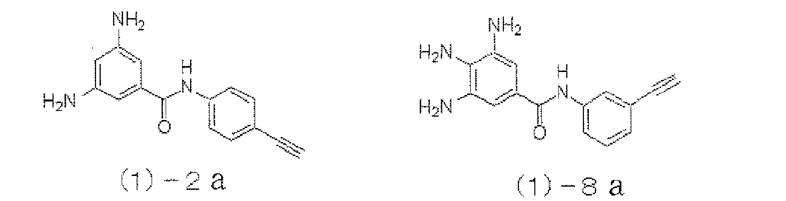

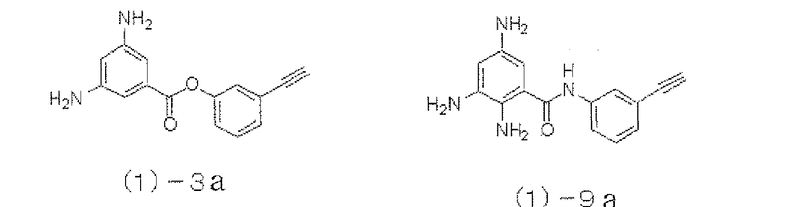

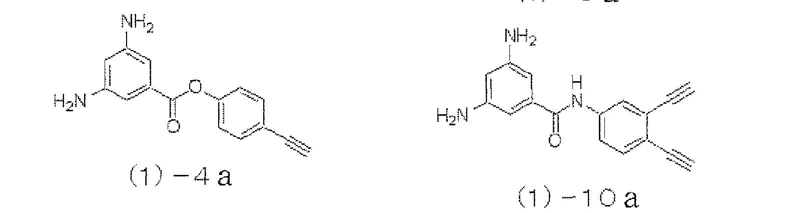

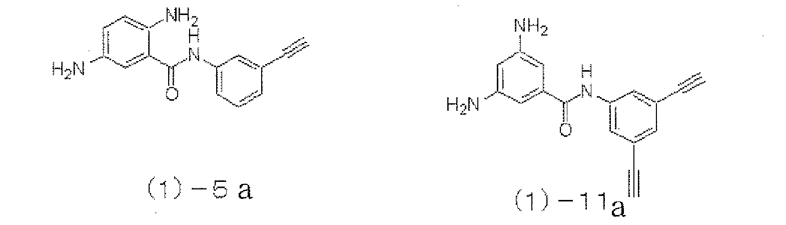

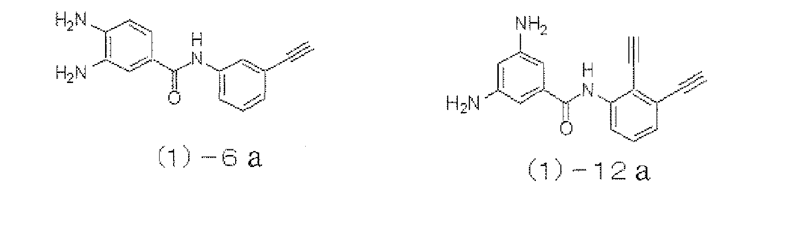

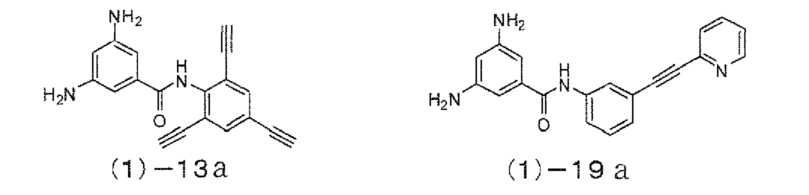

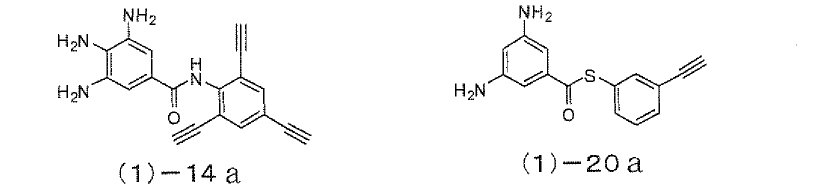

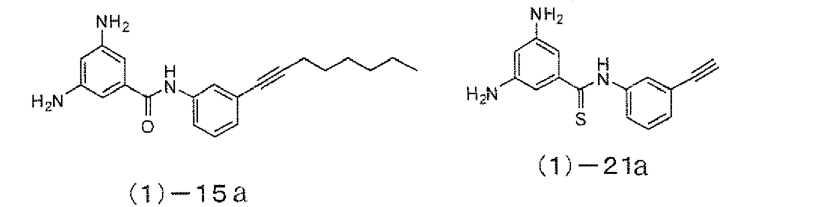

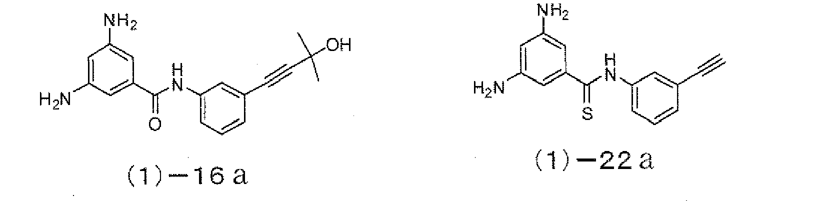

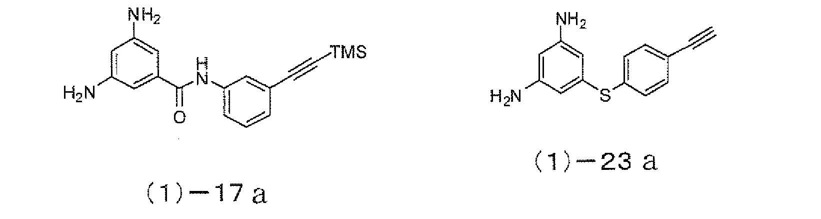

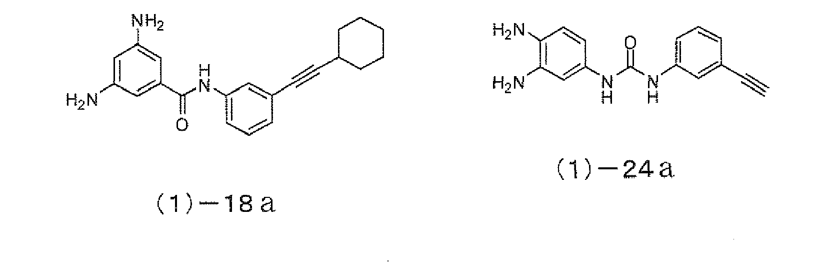

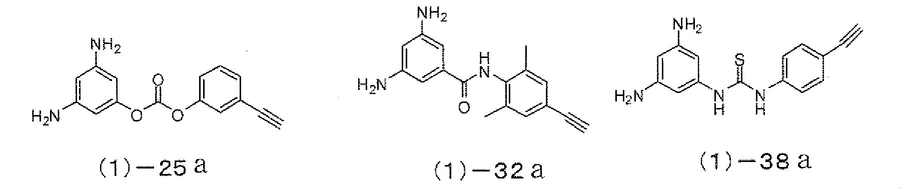

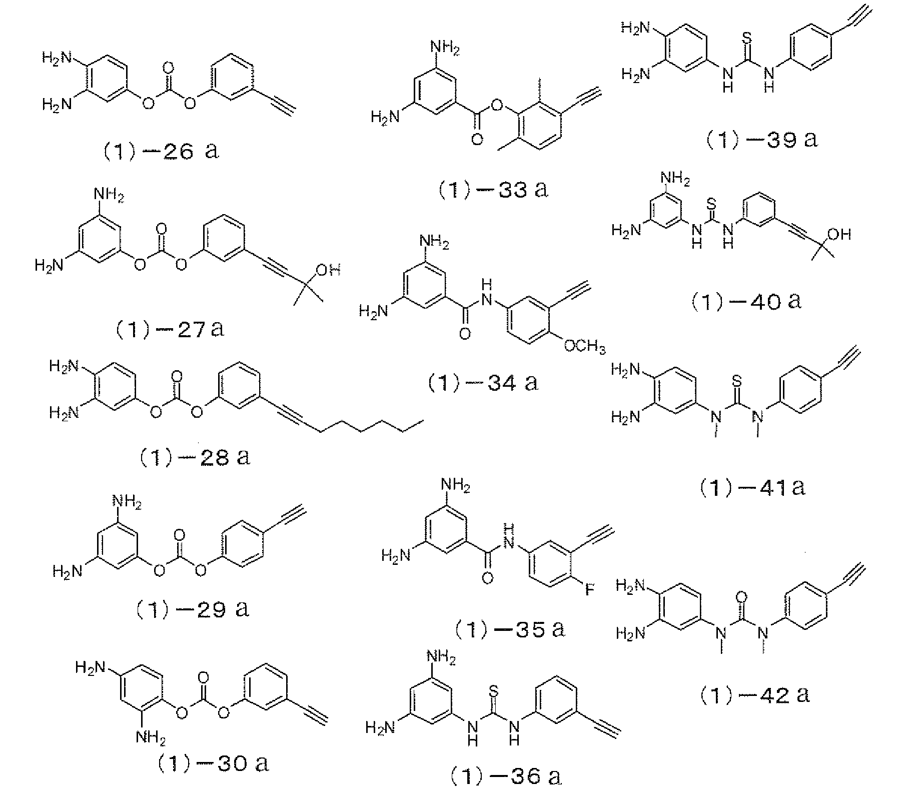

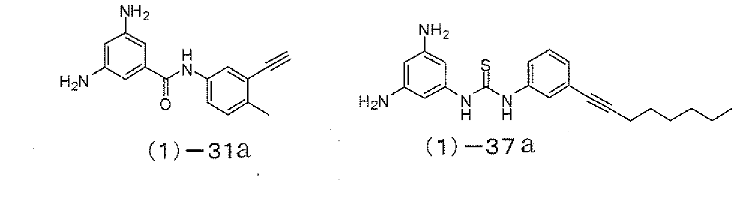

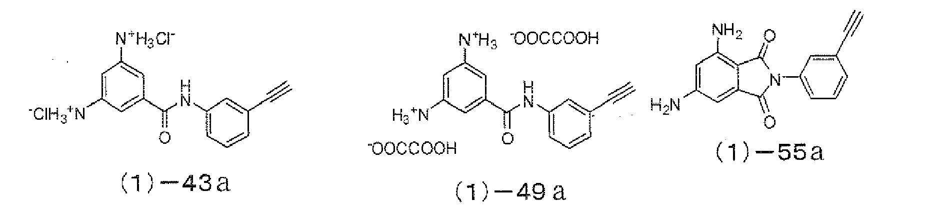

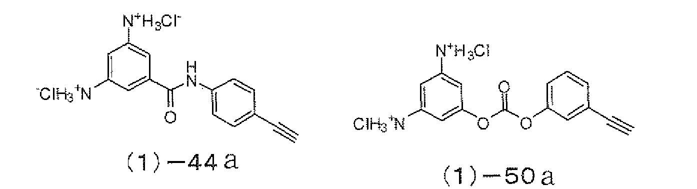

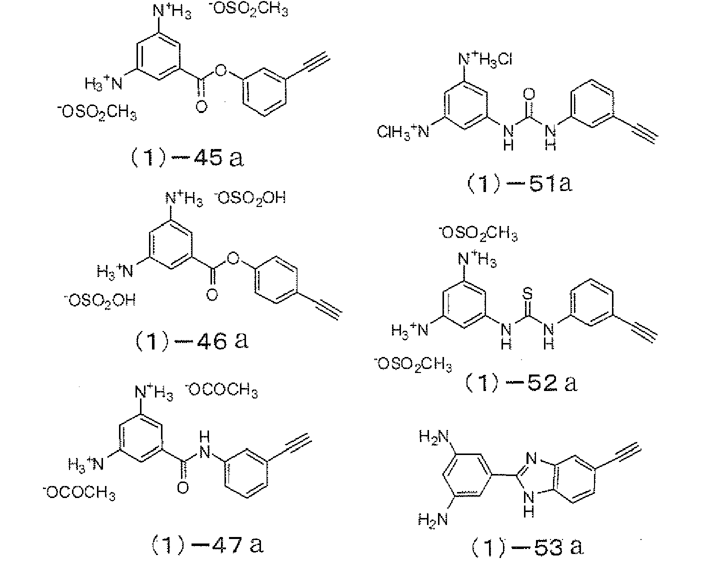

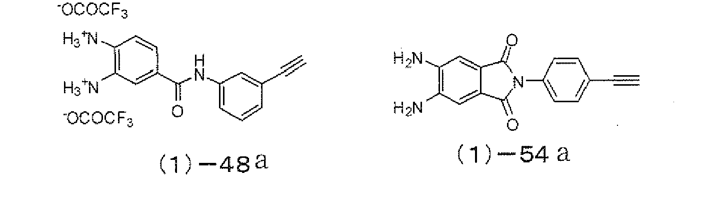

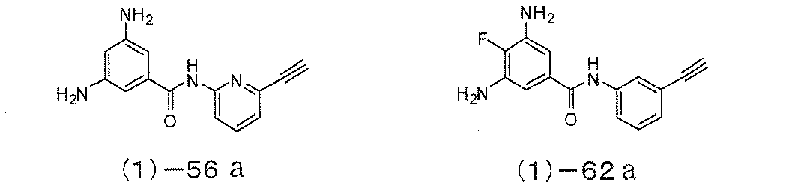

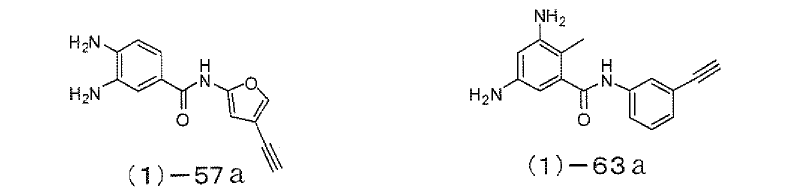

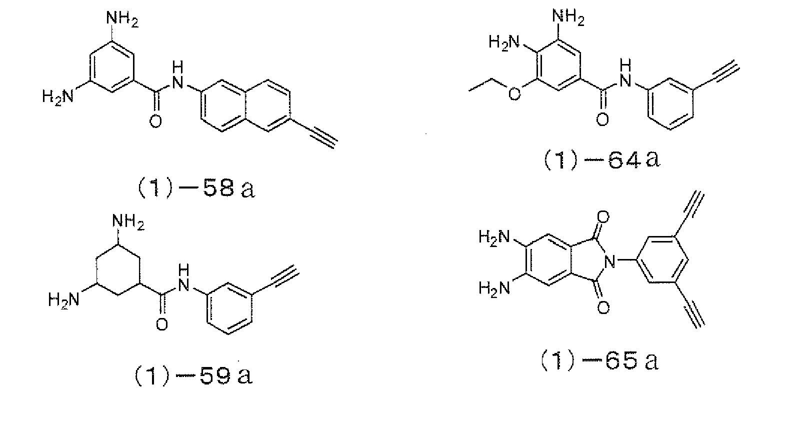

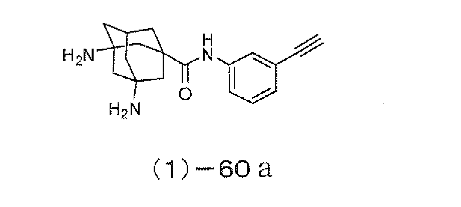

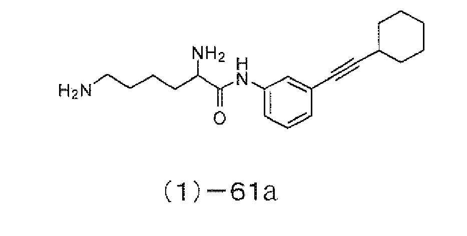

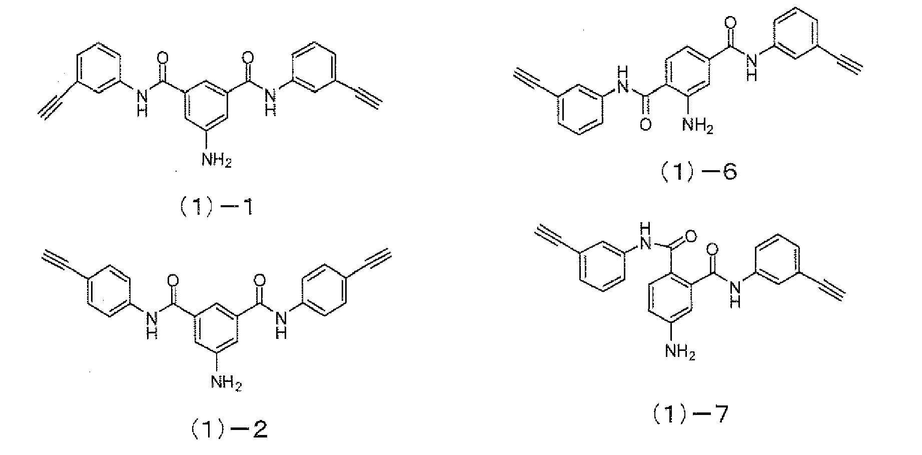

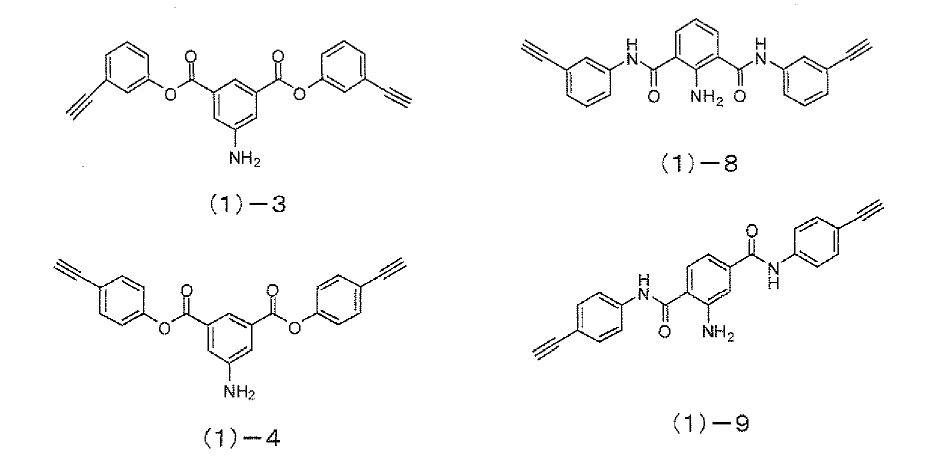

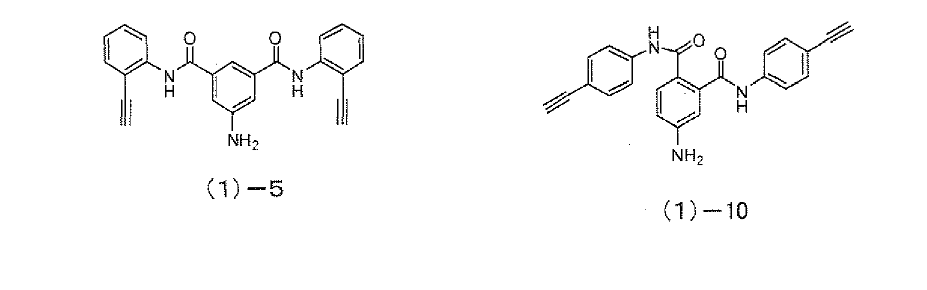

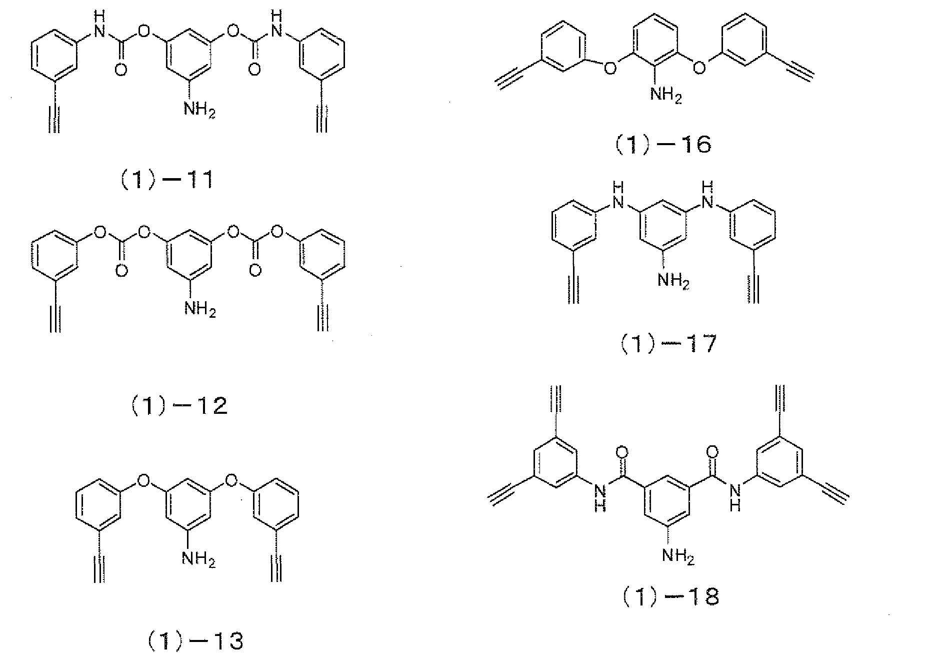

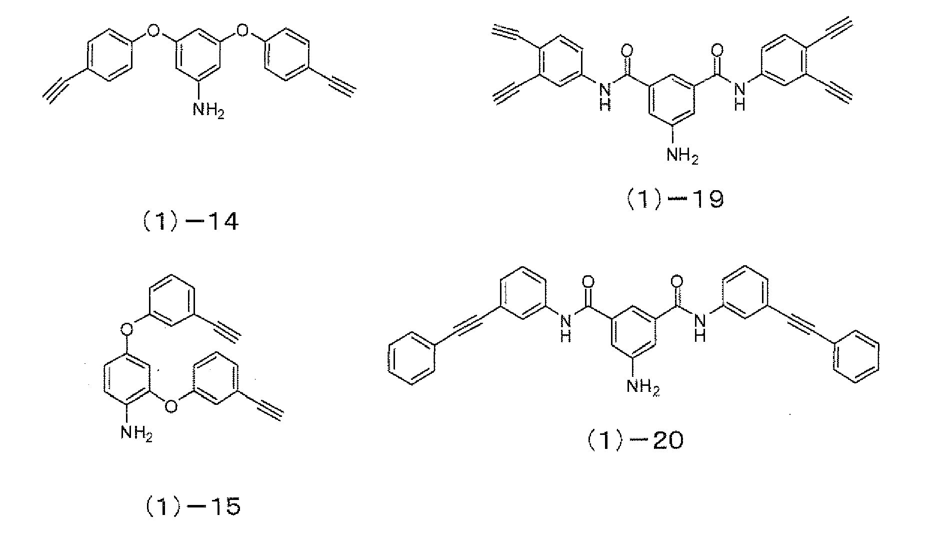

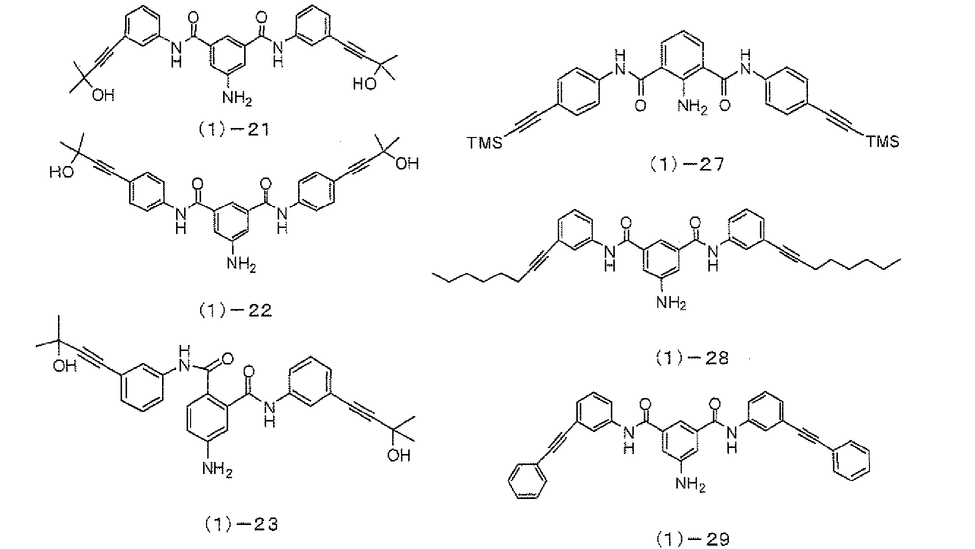

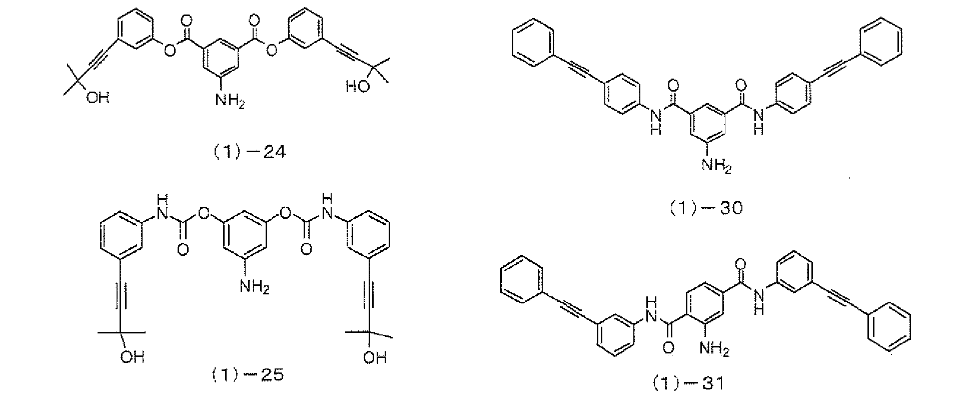

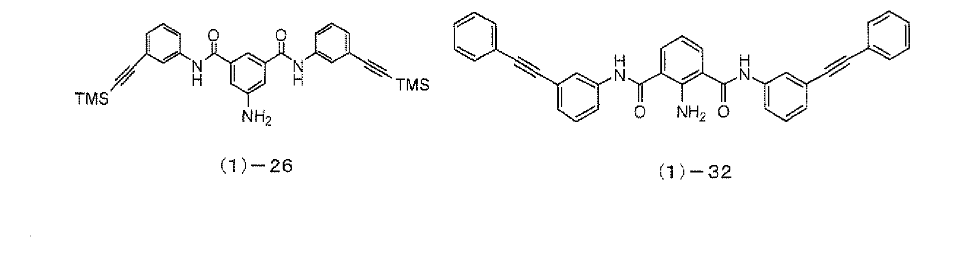

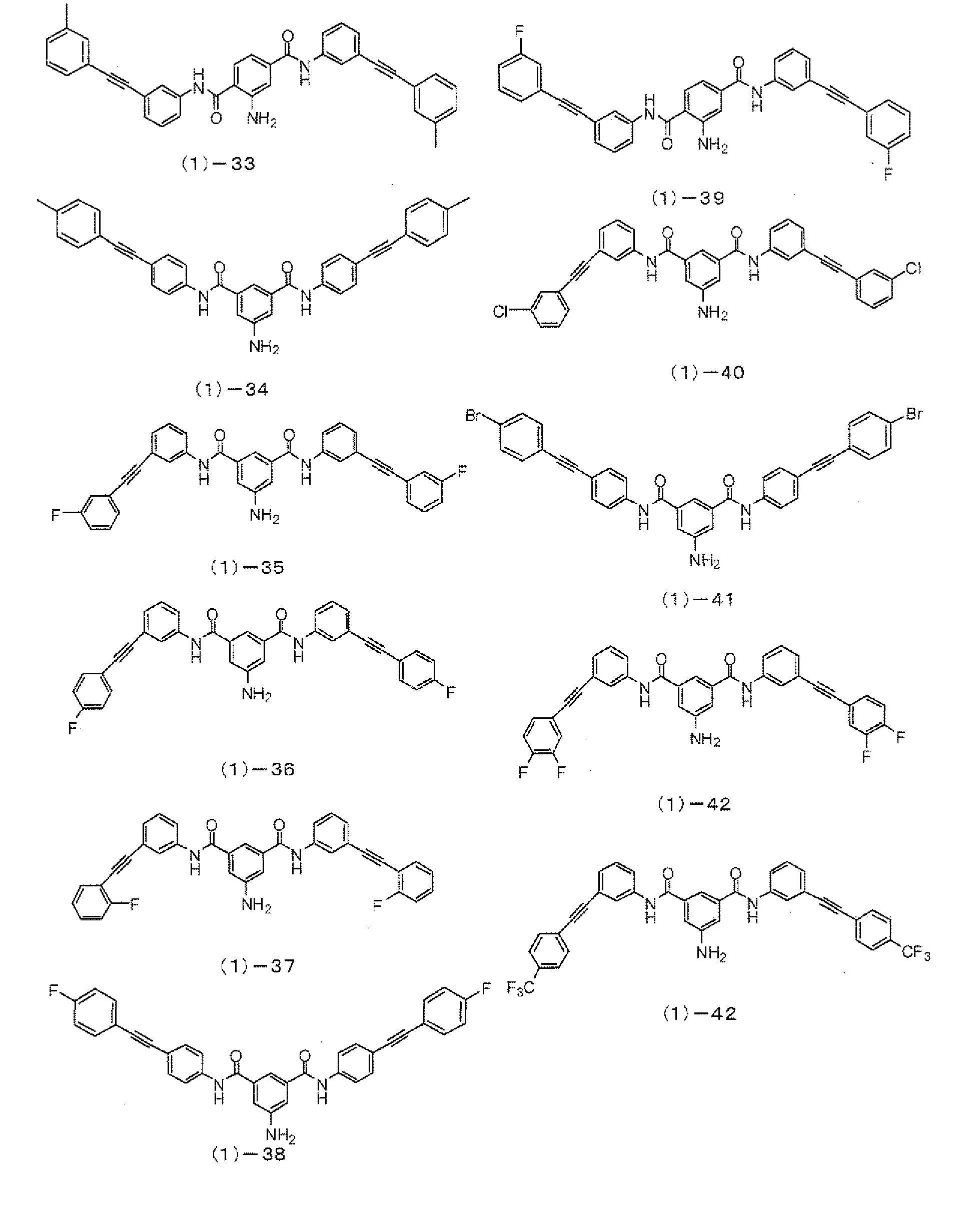

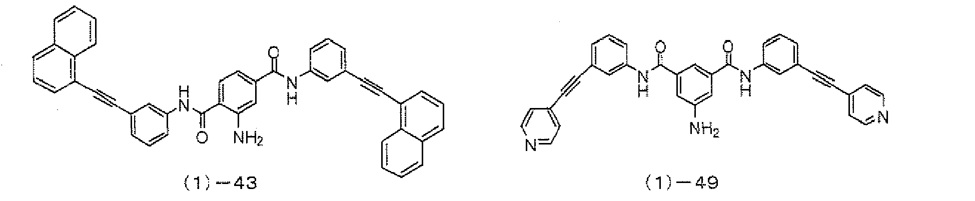

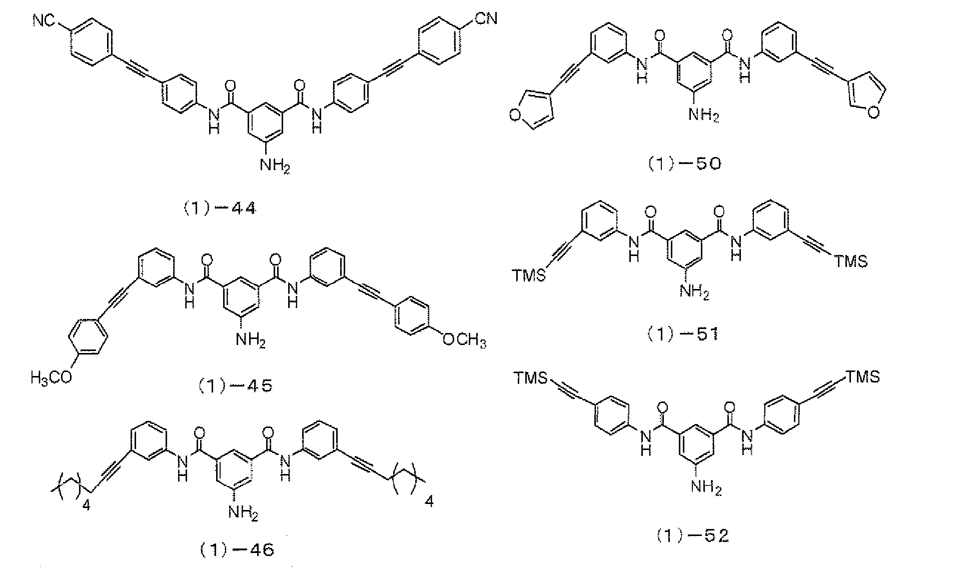

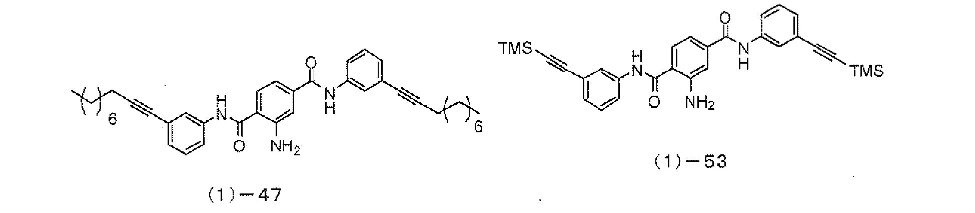

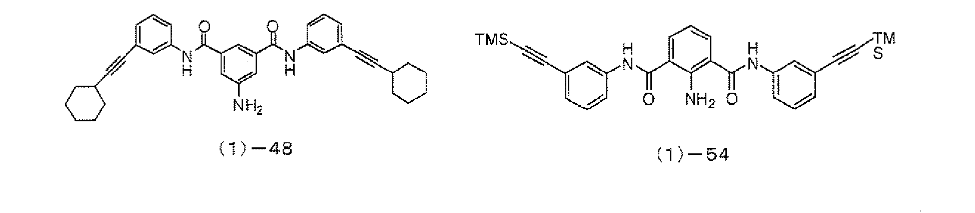

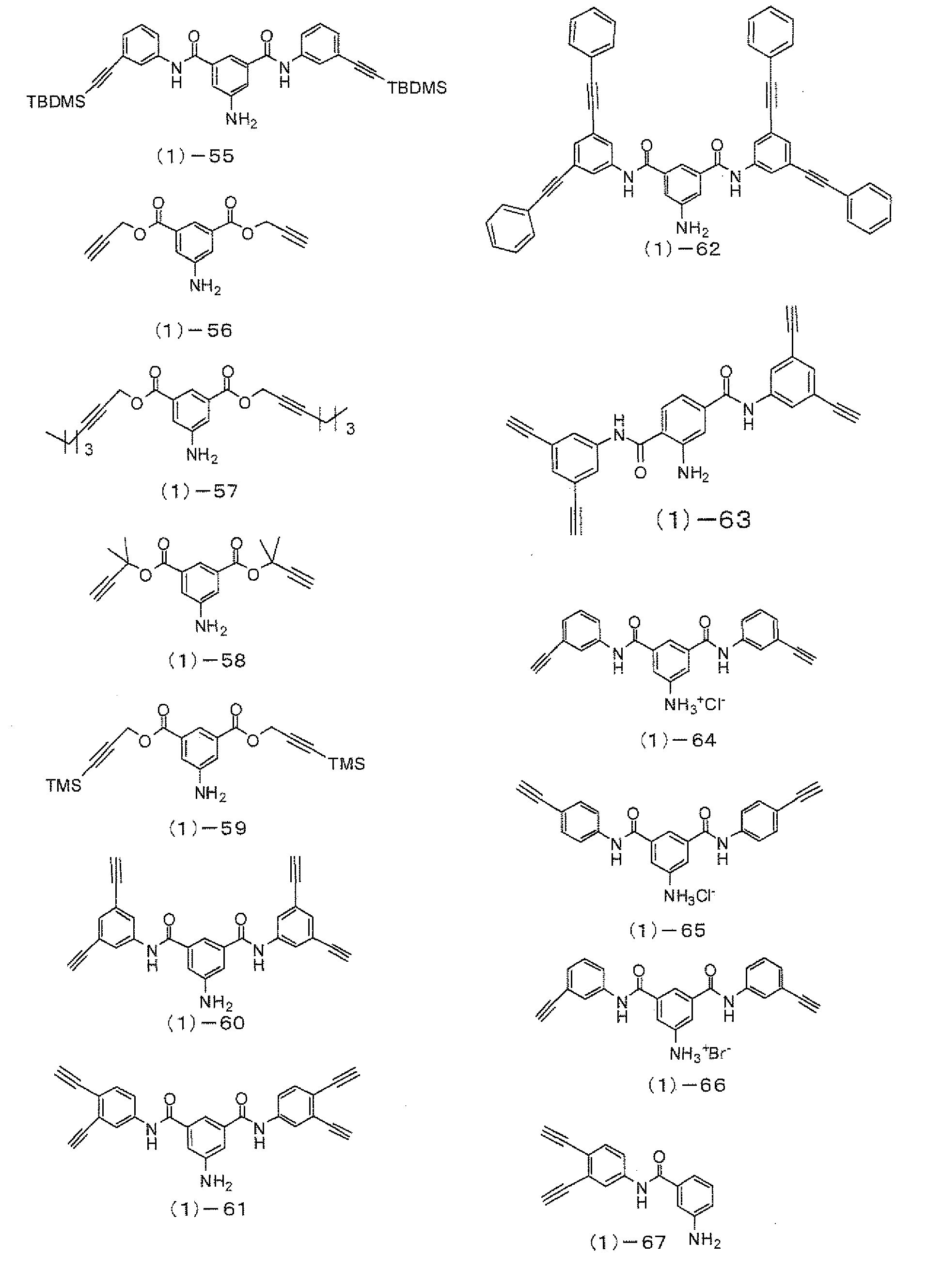

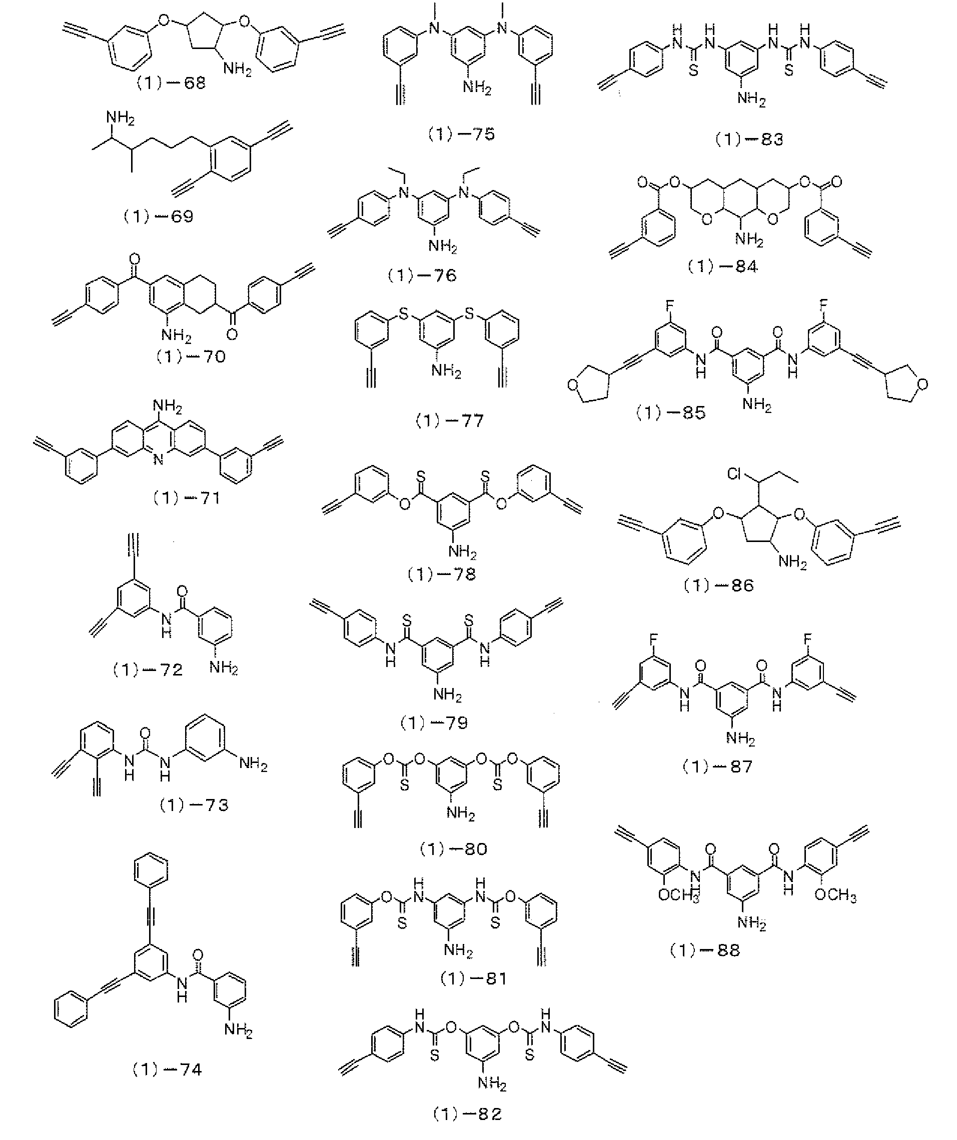

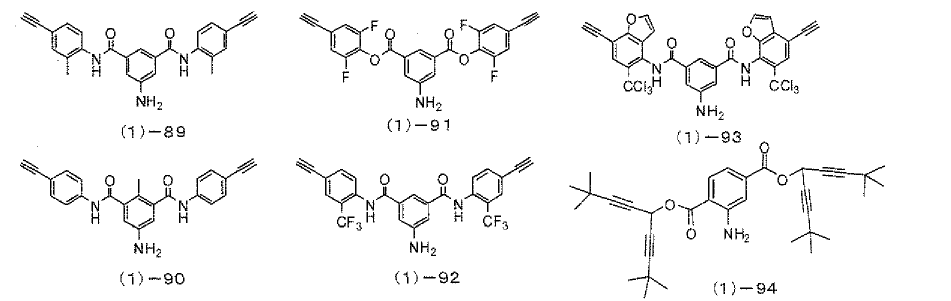

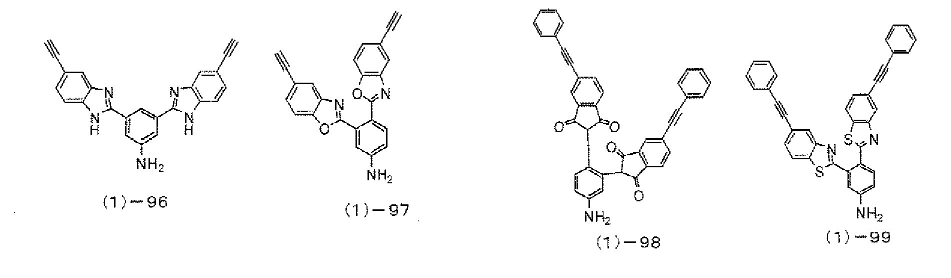

The Following are specific examples of the compound obtained by reacting a compound represented by Formula (1) with a compound having one or more ethynyl groups and any one structure selected from -CHO, -COOH, -CSOH, -COSH, -NCO or -COOH in the molecule or a derivative thereof. However, the invention is not limited thereto. In the follwoing specific examples, n represents an integer of from 0 to 1,000, and these compounds may be used alone or in combination of two or more.

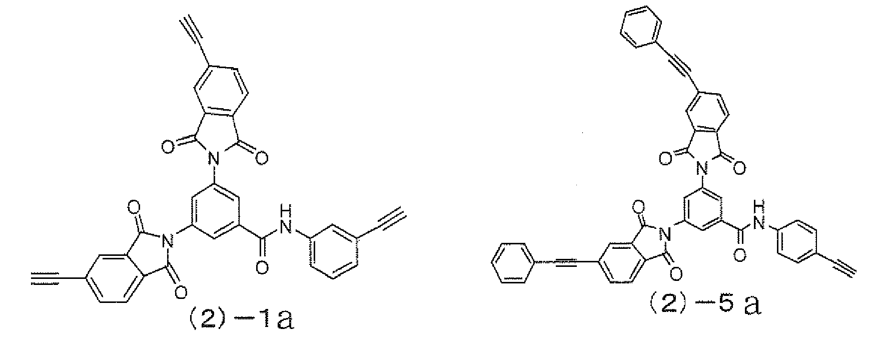

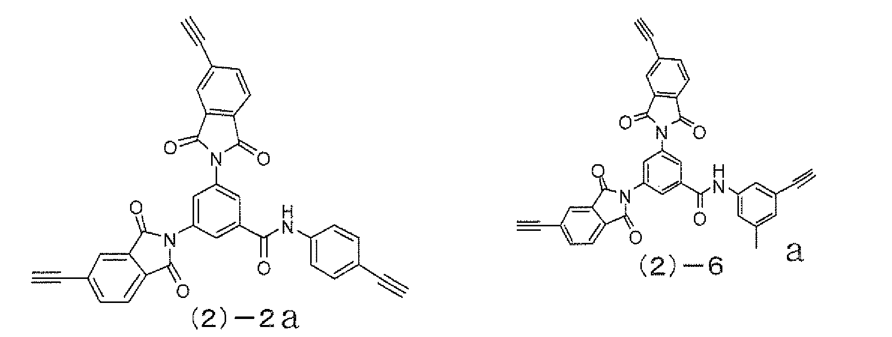

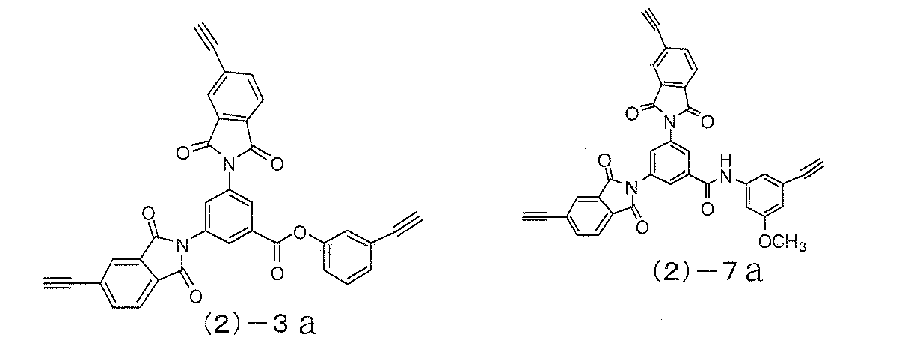

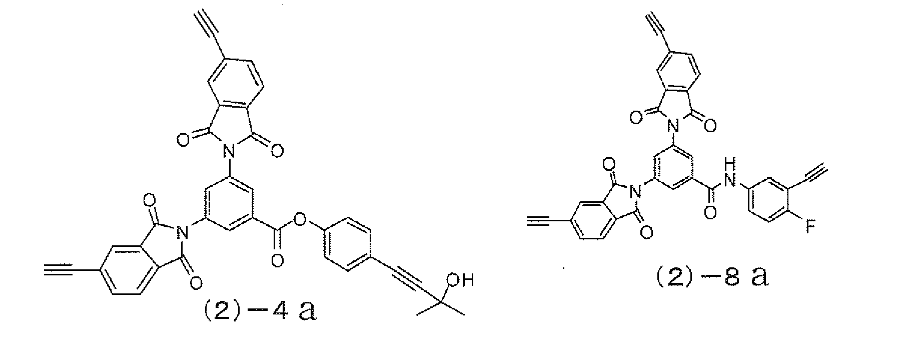

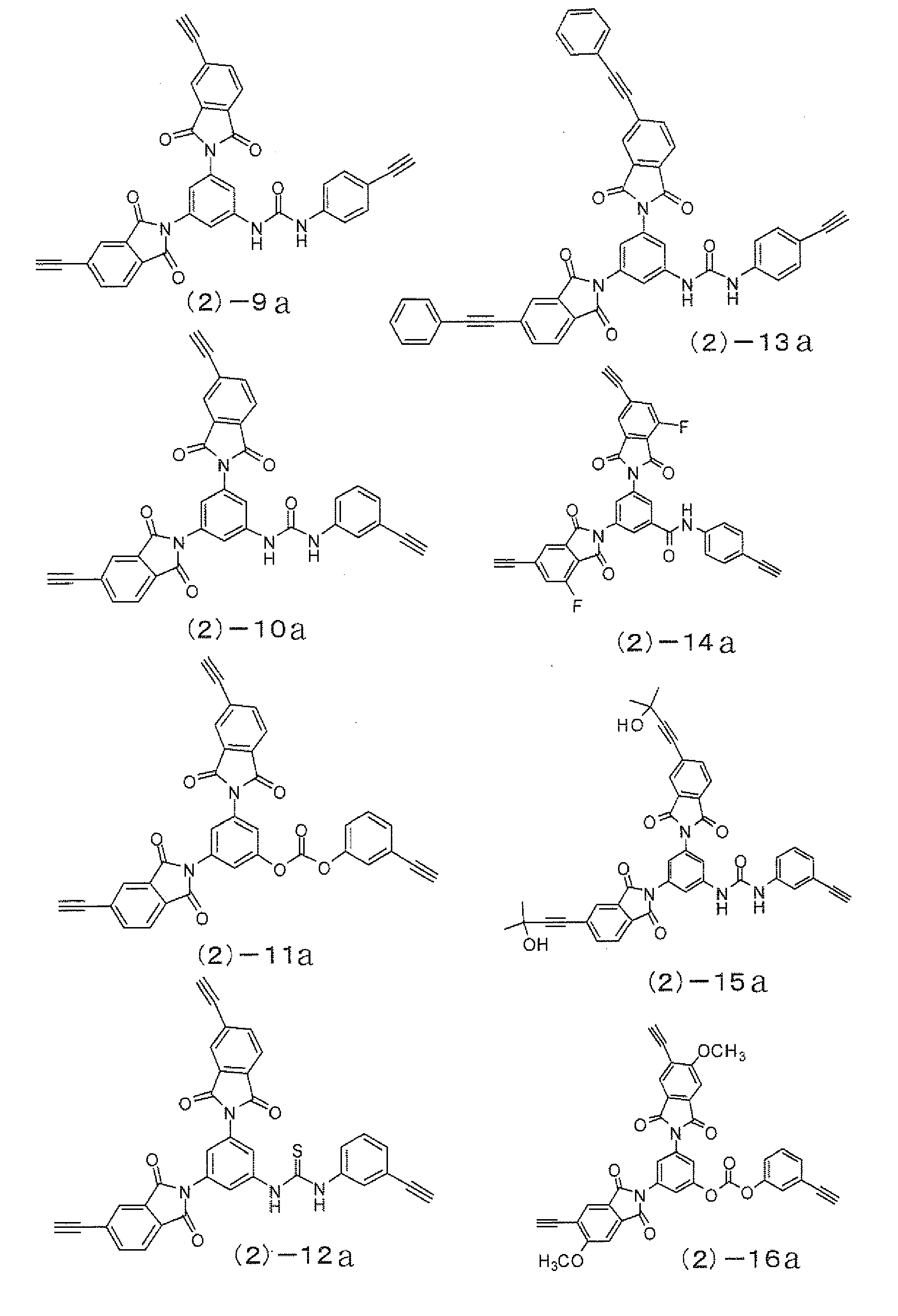

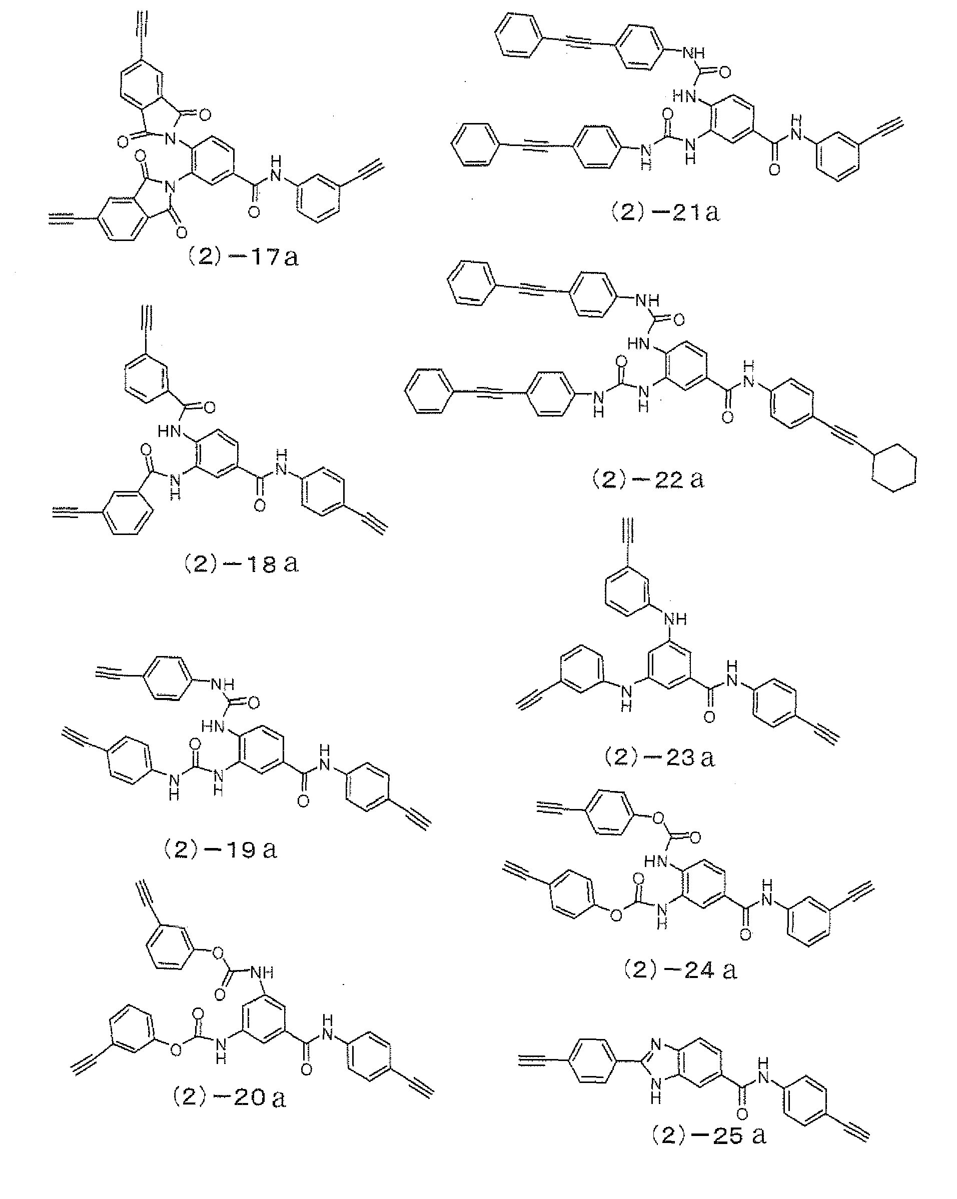

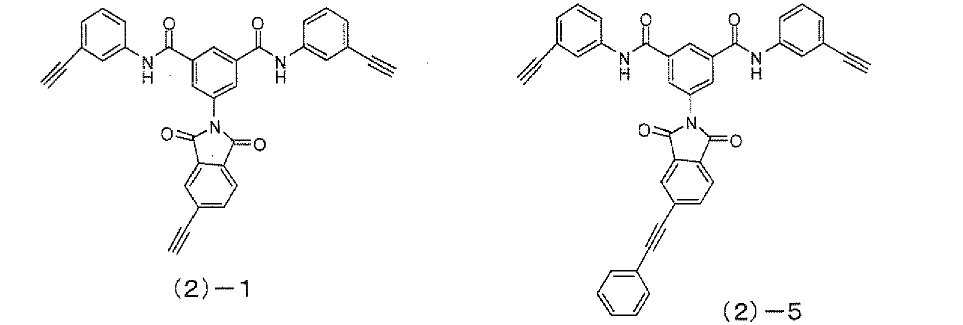

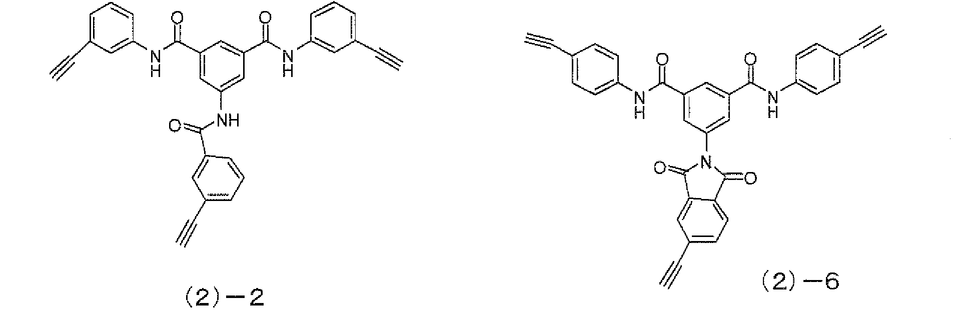

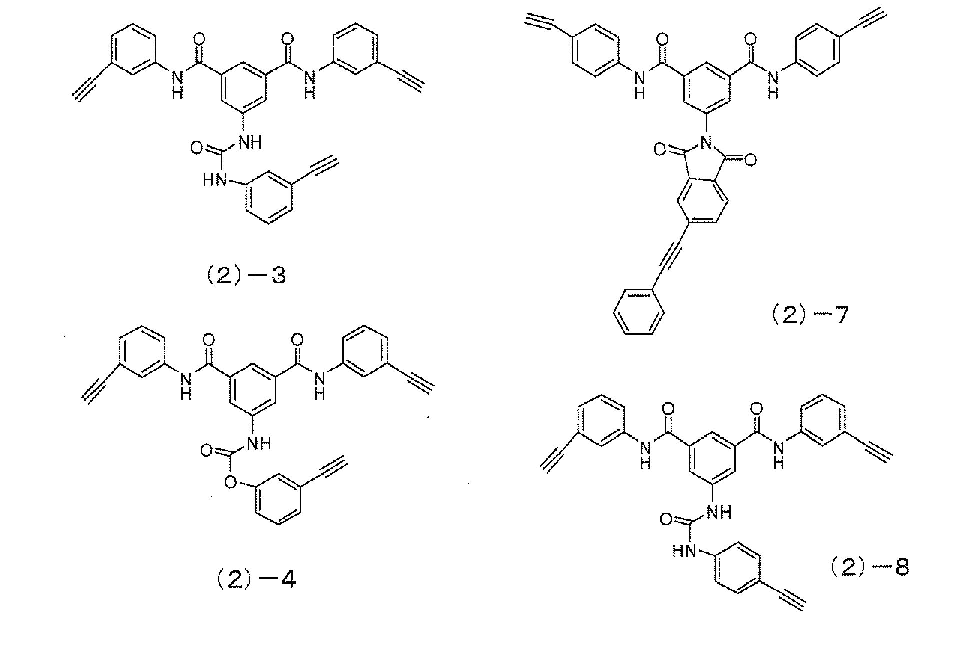

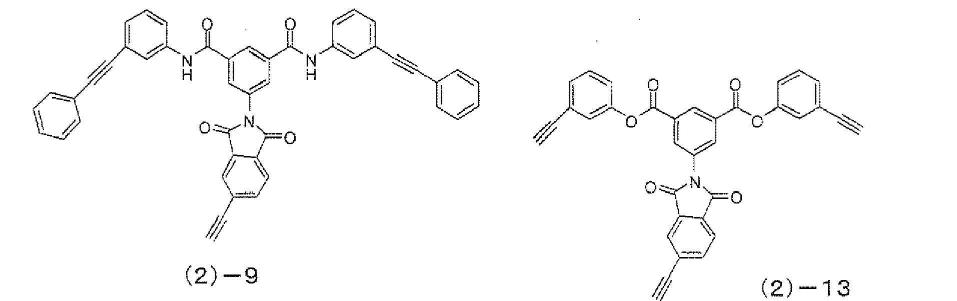

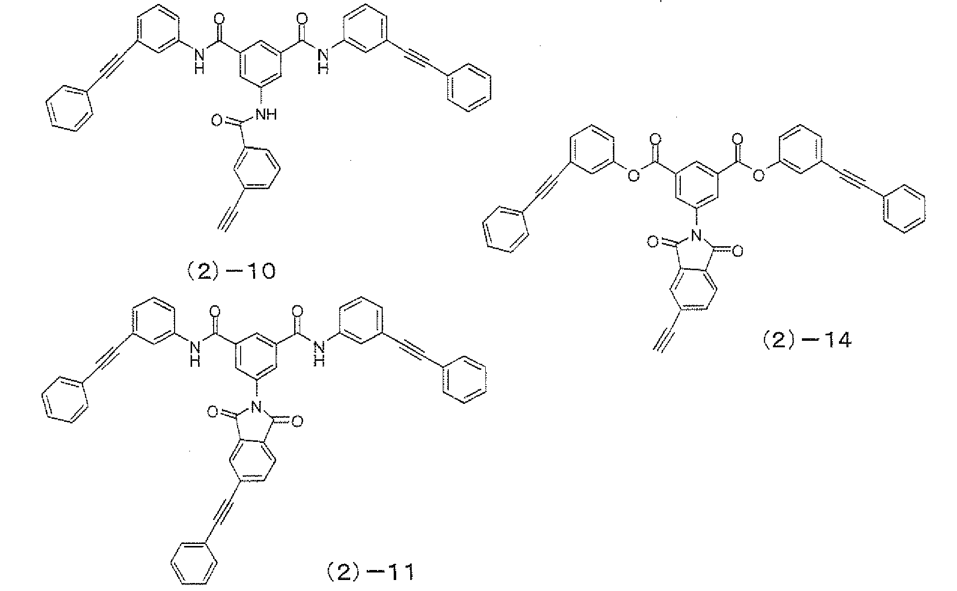

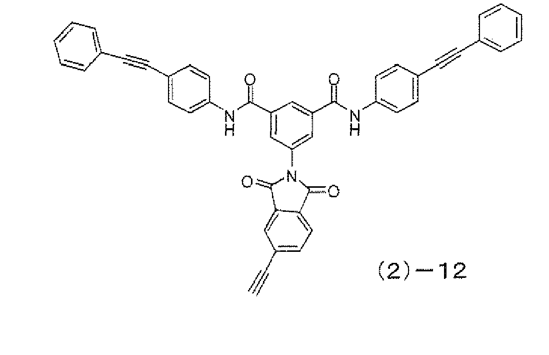

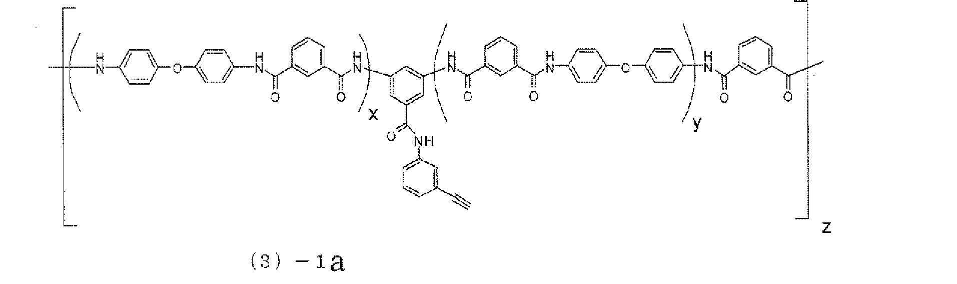

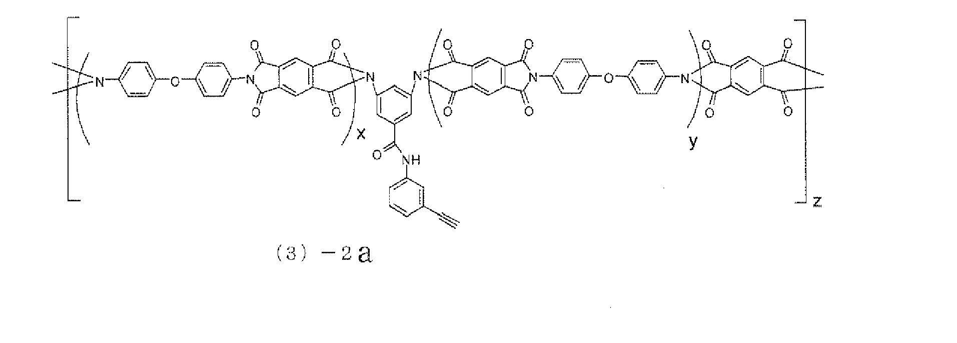

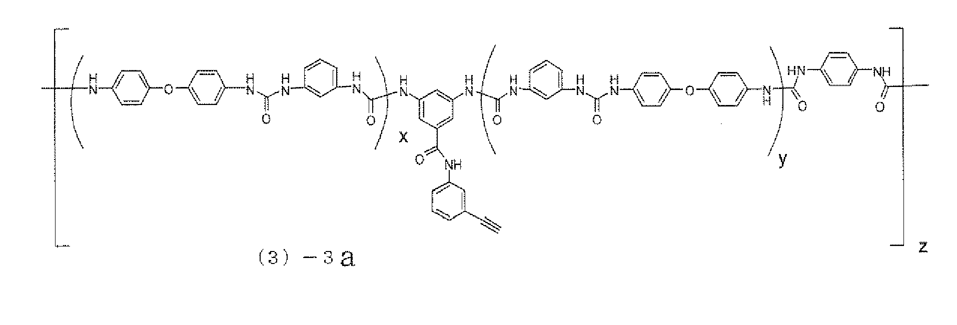

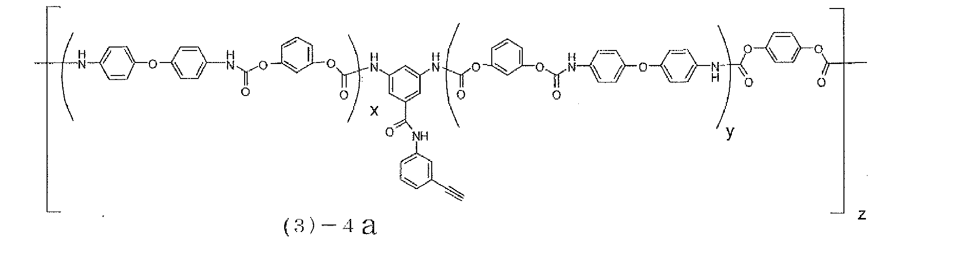

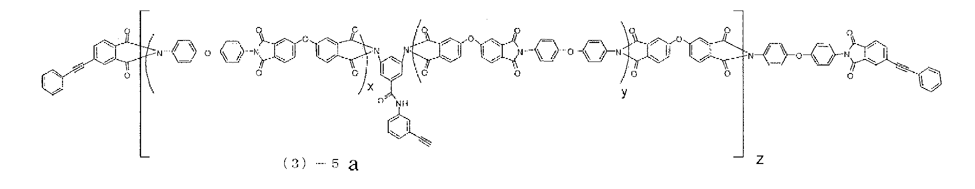

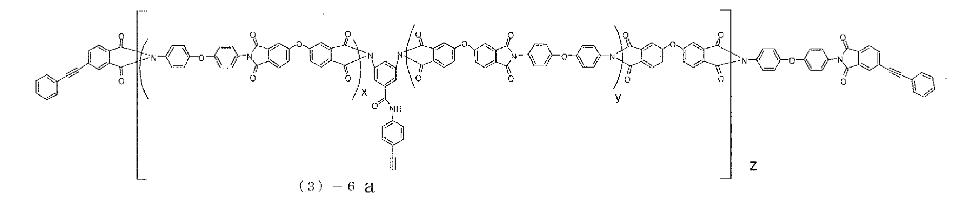

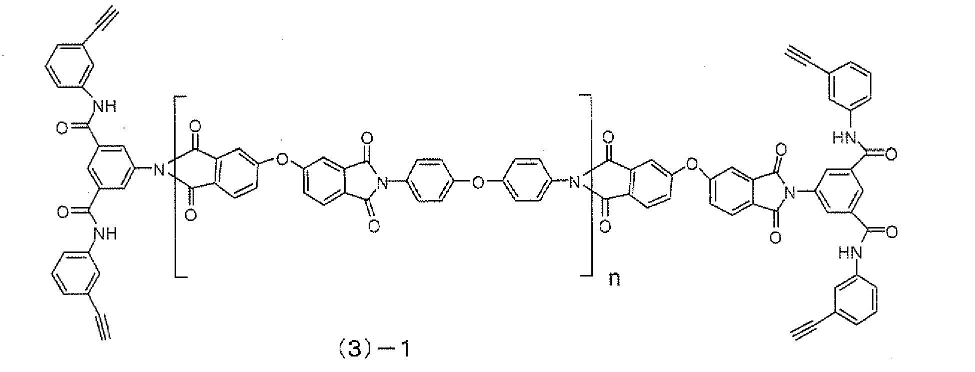

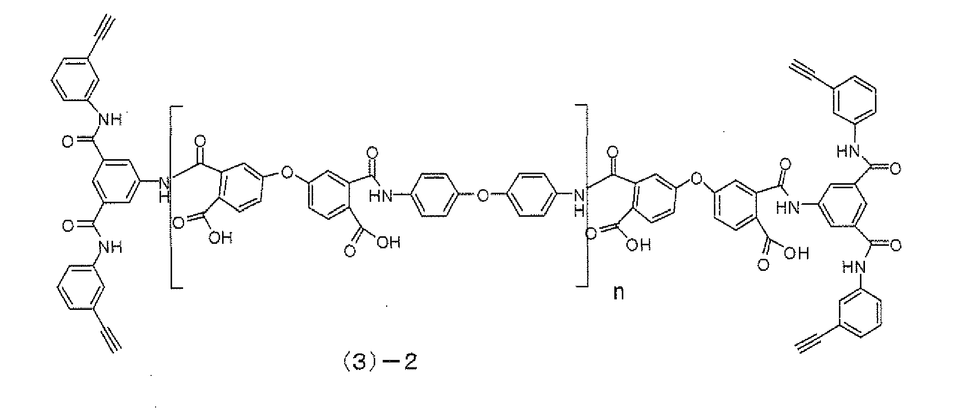

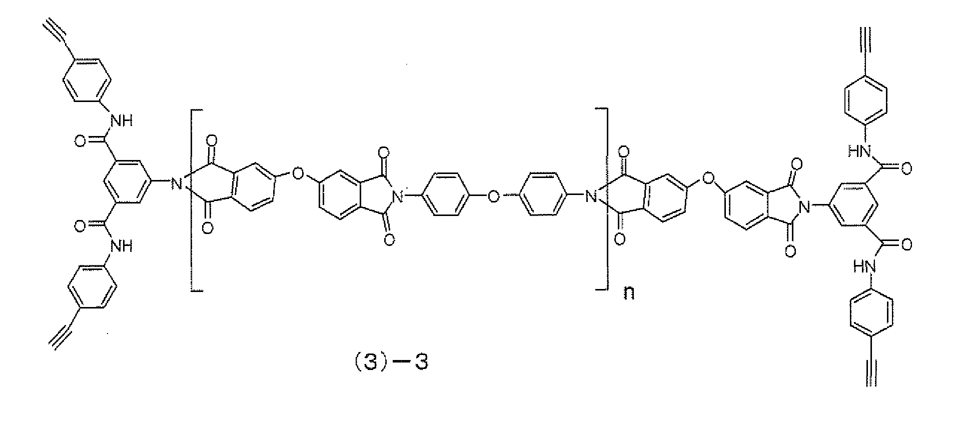

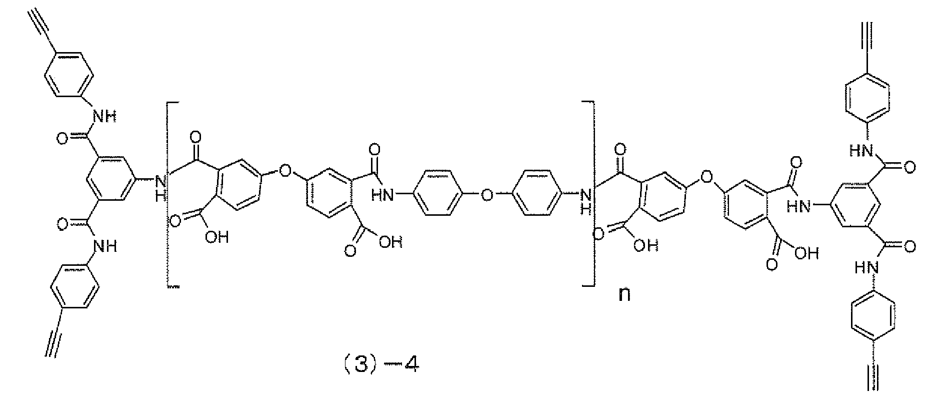

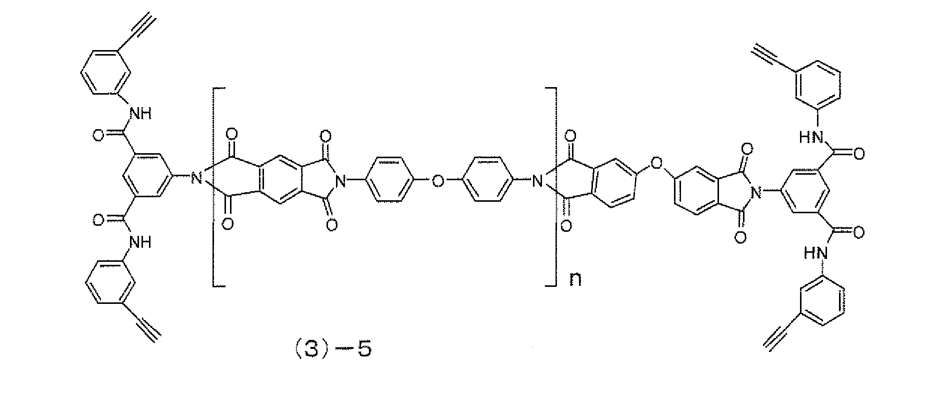

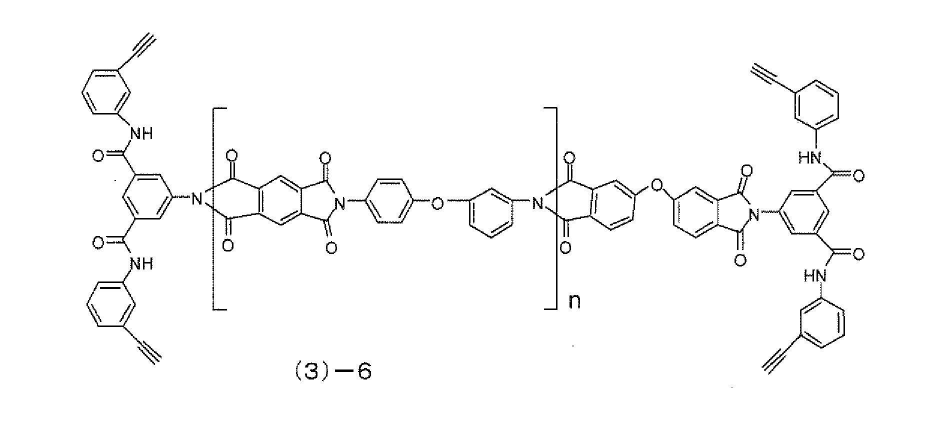

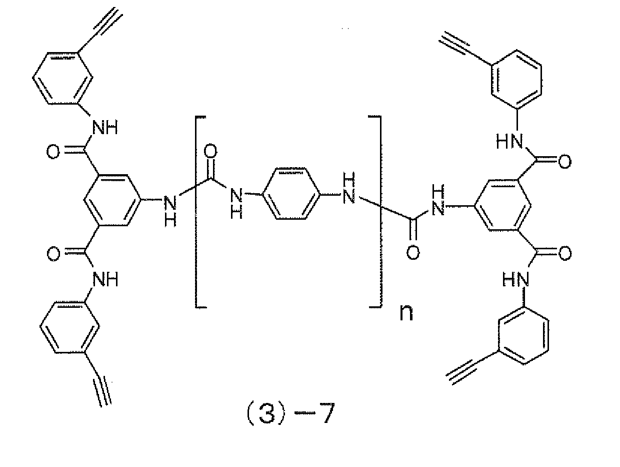

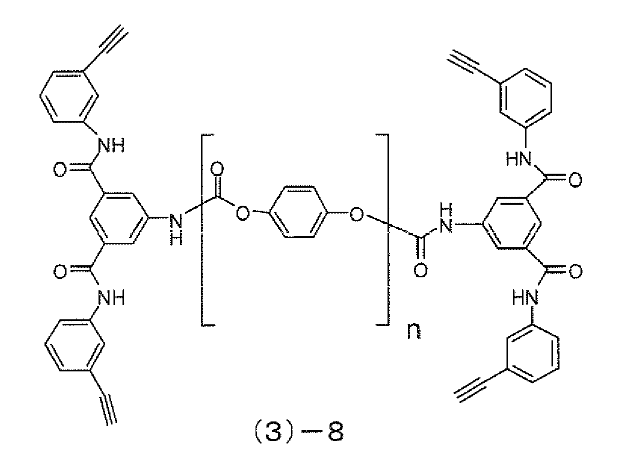

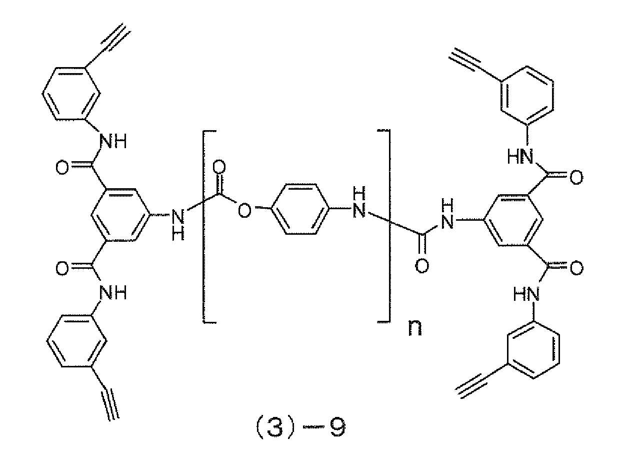

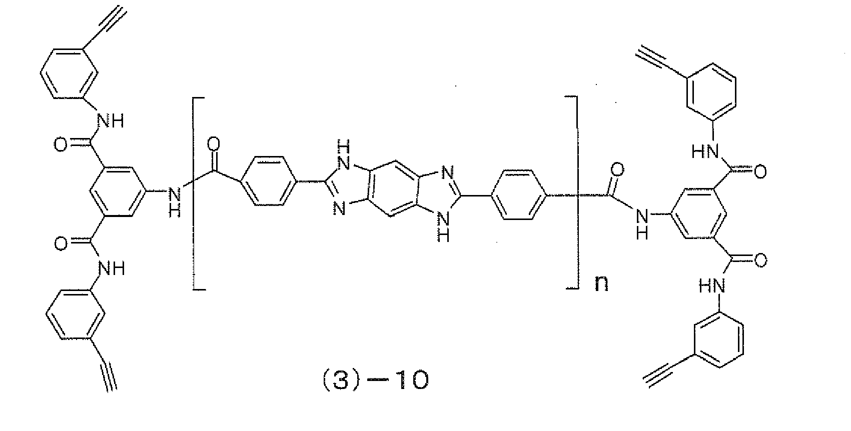

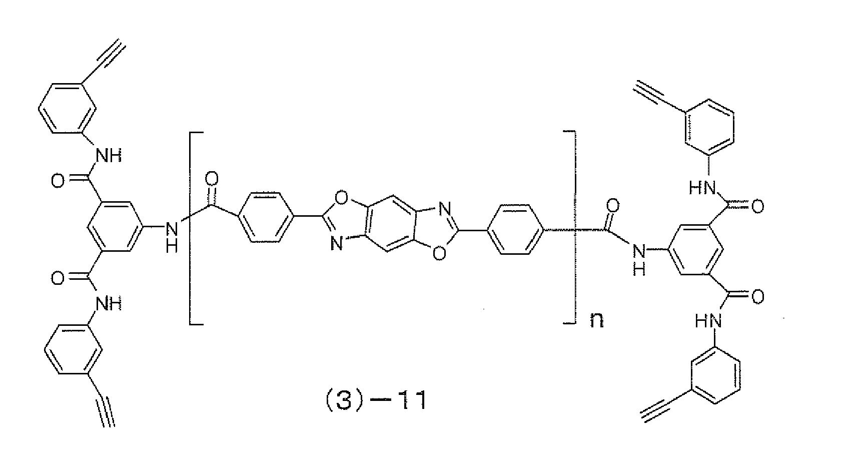

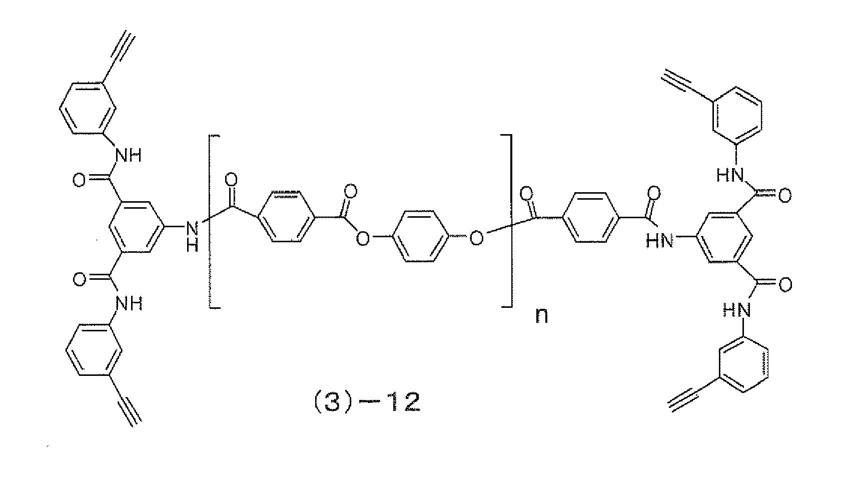

### <Explanation of production method>

In the following, a method of producing the novel acetylene compound according to the invention is explained.
The method of producing the novel acetylene compound in which X represents -OCO-, -NRCO-, -OCS-, -NRCS-, -NRCONR'-, -NRCOO-, -OCONR-, -OCOO-, -NRCSNR'- or -OCSO- is a method of causing condensation reaction of a compound having an amino group and a group having the corresponding structure selected from a carboxylic acid group, a carboxylic ester group, a carbamic ester group, a thiocarboxylic acid group or a thiocarboxylate ester group and a compound having one or more ethynyl group and a group selected from an amino group, a hydroxy group or a thiol group. There are roughly two types of the above methods.
One of them is a method of protecting the amino group of the compound having the amino group, and then directly reacting the same with the compound having one or more ethynyl groups.
Another is a method of protecting the amino group of the compound having the amino group, converting the same to an intermediate in which the reactivity of the carboxylic group to be reacted is increased, and then reacting the same with the compound having one or more ethynyl groups.

The above two methods are explained.
Explanation of a method of of protecting the amino group of the compound having an amino group, and then directly reacting the same with the compound having one or more ethynyl groups
The following is a method of producing an acetylene compound represented by Formula (1) where -B- is a structure represented by Formula (3), by reacting an acetylene compound represented by Formula (7) with a compound represented by Formula (5).
When reacting the compound represented by Formula (5) with the compound represented by Formula (7), although the compound represented by Formula (5) may be directly reacted with the compound represented by Formula (7), it is preferred to protect the amino group of the compound represented by Formula (5), and then react the compound represented by Formula (7) with a compound represented by Formula (6).

As the protective group for the amino group of the compound represented by Formula (5), any protective groups for an amino group described in "Protective Groups in Organic Synthesis" can be used. Specific examples of preferred protective groups include an acetyl group, a benzyloxycarbonyl (BOM) group, a 2-(trimethylsilyl)ethoxycarbonyl (TEOC) group, a t-butoxycarbonyl (Boc) group, an allyloxycarbonyl (AOC) group, a 2,2,2-trichloroethoxycarbonyl (Troc) group, 9-fluorenylmethoxycarbonyl (FMOC) group, a tosyl (Ts) group, and a mesyl (Ms) group. Among these, a t-butoxycarbonyl group and an acetyl group are preferred.

The reaction conditions for protecting the amino group described in the above document can be applied to the invention. The compound with the amino group being protected can be isolated or purified by a process such as reprecipitation or crystallization, but it is also possible to use the compound in the form of a reaction solution in the subsequent reaction.

When reacting the compound represented by Formula (5) with the amino group being protected with the compound represented by Formula (7), a catalyst may be added to the reaction system as necessary, and it is preferable to carry out the reaction under the presence of the catalyst. Specifically, the reaction can be carried out using an acid catalyst, and examples thereof include an inorganic acid such as hydrogen chloride, hydrogen bromide, sulfuric acid or phosphoric acid; an organic acid such as p-tolu.enesulfonic acid or camphor- 10-sulfonic acid; and an acidic ion exchange resin such as AMBERUTE or AMBERLYST; or a condensation agent such as dicyclohexylcarbodiimide or 1-ethyl-3-(3-dimethylaminopyrrolyl)-carbodiimide.

The amount of the acetylene compound represented by Formula (7) with respect to the amount of carboxylic acid or a derivative compound thereof represented by Formula (5) is preferably from 0.5 to 10 times by mole, more preferably from 0.8 to 3.0 times by mole, further preferably from 0.9 to 2.2 times by mole. When the above amount is less than 0.5 times by mole, it is not preferred since the yield after the reaction may decrease. When the above amount is more than 10 times by mole, it is not preferred since the production cost may increase due to the use of surplus raw materials, although it would not become a significant obstacle to the reaction.

The solvent that can be used in the reaction is not particularly limited as long as it does not cause problems during the process operation or hamper the progress of reaction, as well as it does not decompose and adversely affect the reaction during the process of amidization, esterification or thioesterification of the invention. Examples of the solvent include amide-based solvents (such as N,N-dimethylformamide, N,N-dimethylacetamide and 1-methyl-2-pyrrolidone), sulfone-based solvents (such as sulfolane), sulfoxide-based solvents (such as dimethylsulfoxide), ureido-based solvents (such as tetramethylurea), ether-based solvents (dioxane and cyclopenthyl methyl ether), ketone-based solvents (such as acetone and cyclohexanone), hydrocarbon-based solvents (such as toluene, xylene and n-decane), halogen-based solvents (such as tetrachloroethane and chlorobenzene), pyridine-based solvents (such as pyridine, γ-picoline and 2,6-lutidine), ester-based solvents (such as ethyl acetate and butyl acetate) and nitrile-based solvents (such as acetonitrile). These solvents may be used alone or in combination of two or more kinds.

The reaction temperature is preferably within a range of from -30°C to 300°C, more preferably from 0°C to 200°C, further preferably from 20°C to 150°C. The reaction time may differ depending on the amount of raw materials or the reaction temperature, but is preferably within a range of from 0.5 to 12 hours, more preferably from 0.5 to 6 hours. The reaction is preferably carried out in a sufficiently dried inert gas atmosphere. Since the presence of moisture lowers the reaction rate, the amount thereof is preferably as small as possible. Specifically, noble gases such as nitrogen or argon are suitably used as the inert gas.

The acetylene compound according to the invention can be isolated from the reaction mixture by, for example, a separation-purification method that includes extracting the acetylene compound with an organic solvent, and then subjecting the same to chromatography, crystallization or recrystallization. When the acetylene compound precipitates by cooling the solvent that has been extracted with an organic solvent, the acetylene compound can be isolated by an ordinary solid-liquid separation method. Alternatively, the acetylene compound can be isolated by allowing the same to crystallize from a suitable solvent system, and then subjecting the same to solid-liquid separation.

Examples of the organic solvent used for extracting the acetylene compound include ether-based solvents such as diethyl ether, diisopropyl ether, methyl t-butyl ether and methoxybenzene; ester-based solvents such as ethyl acetate and n-butyl acetate; aliphatic hydrocarbon solvents such as hexane, heptane and cyclohexane; aromatic hydrocarbon solvents such as toluene and xylene; and halogen-based solvents such as chlroroform and methylene chloride. Among these, in view of suitability for industrial-scale mass production, safety and availability, ester-based solvents, aliphatic hydrocarbon solvents and aromatic hydrocarbon solvents are preferred. Specific examples of the preferred organic solvent include toluene, xylene (o-xylene, m-xylene or p-xylene or a mixture thereof mixed at an arbitrary ratio), mesitylene, ethylbenzene, isopropylbenzene (cumene), chlorobenzene, hexane, heptane, ethyl acetate, and n-butyl acetate. Among these, toluene, xylene, ethylbenzene, hexane, heptane, ethyl acetate and n-butyl acetate are more preferred, and toluene, hexane, heptane and ethyl acetate are further preferred. The aforementioned solvents may be used alone or in combination of two or more kinds.

One example of the organic solvents used for crystallizing the acetylene compound is a mixed system of the aformentioned organic solvent and other organic solvent. Examples of the other organic solvent to be mixed include ether-based solvents such as diethyl ether, diisopropyl ether, methyl t-butyl ether and methoxybenzene; nitrile-based solvents such as acetonitrile; aliphatic hydrocarbon solvents such as hexane, heptane and cyclohexane; aromatic hydrocarbon solvents such as toluene and xylene; and alcohol-based solvents such as 2-propanol and t-butanol. Among these, in view of suitability for industrial-scale mass production, safety and availability, ester-based solvents, aliphatic hydrocarbon solvents, aromatic hydrocarbon solvents and water are preferred. Specific examples of the preferred organic solvent include toluene, xylene (o-xylene, m-xylene or p-xylene or a mixture thereof mixed at an arbitrary ratio), 2-propanol, t-butanol, mesitylene, ethylbenzene, isopropylbenzene (cumene), hexane, heptane, acetonitrile, propionitrile, diisopropyl ether, methyl t-butyl ether and methoxybenzene. Among these, toluene, acetonitrile, diisopropyl ether, methyl t-butyl ether and water are more preferred. The aforementioned solvents may be used alone or in combination of two or more kinds.

The conditions for deprotecting the protective group for an amino group described in "Protective Groups in Organic Synthesis" can be applied to the invention.

The acetylene compound according to the invention can be isolated from the reaction mixture by, for example, a separation-purification method including extracting the acetylene compound with an organic solvent, and then subjecting the same to chromatography, crystallization or recrystallization. When the acetylene compound precipitates by cooling the solvent that has been extracted with an organic solvent, the acetylene compound can be isolated by an ordinary solid-liquid separation method. Alternatively, the acetylene compound can be isolated by crystallizing the same from a suitable solvent system, and then subjecting the same to solid-liquid separation.

Examples of the organic solvent used for extracting the acetylene compound include ether-based solvents such as diethyl ether, diisopropyl ether, methyl t-butyl ether and methoxybenzene; ester-based solvents such as ethyl acetate and n-butyl acetate; aliphatic hydrocarbon solvents such as hexane, heptane and cyclohexane; aromatic hydrocarbon solvents such as toluene and xylene; and halogen-based solvents such as chloroform and methylene chloride. Among these, in view of suitability for industrial-scale mass production, safety and availability, ester-based solvents, aliphatic hydrocarbon solvents and aromatic hydrocarbon solvents are preferred. Specific examples of the preferred organic solvent include toluene, xylene (o-xylene, m-xylene or p-xylene or a mixture thereof mixed at an arbitrary ratio), mesitylene, ethylbenzene, isopropylbenzene (cumene), chlorobenzene, hexane, heptane, ethyl acetate, n-butyl acetate, diethyl ether, diisopropyl ether and methyl t-butyl ether. Among these, toluene, xylene, ethylbenzene, hexane, heptane, ethyl acetate, n-butyl acetate and diethyl ether are more preferred, and toluene and ethyl acetate are further preferred. The aforementioned solvents may be used alone or in combination of two or more kinds.

One example of the organic solvents used for crystallizing the acetylene compound is a mixed system of the aformentioned organic solvent and other organic solvent. Examples of the other organic solvent to be mixed include ether-based solvents such as diethyl ether, diisopropyl ether, methyl t-butyl ether and methoxybenzene; nitrile-based solvents such as acetonitrile; aliphatic hydrocarbon solvents such as hexane, heptane and cyclohexane; aromatic hydrocarbon solvents such as toluene and xylene; and alcohol-based solvents such as 2-propanol and t-butanol. Among these, in view of suitability for industrial-scale mass production, safety and availability, ester-based solvents, aliphatic hydrocarbon solvents, aromatic hydrocarbon solvents and water are preferred. Specific examples of the preferred organic solvent include toluene, xylene (o-xylene, m-xylene or p-xylene or a mixture thereof mixed at an arbitrary ratio), 2-propanol, t-butanol, mesitylene, ethylbenzene, isopropylbenzene (cumene), hexane, heptane, acetonitrile, propionitrile, diisopropyl ether, methyl t-butyl ether and methoxybenzene. Among these, toluene, acetonitrile, diisopropyl ether, methyl t-butyl ether and water are more preferred. The aforementioned solvents may be used alone or in combination of two or more kinds.

The acetylene compound can be isolated also in the form of a salt by adding a suitable acid to the reaction solution, after the completion of reaction or after the extraction. Exemplary acids include inorganic acids such as hydrochloric acid, sulfuric acid and phosphoric acid; and organic acids such as oxalic acid, methane sulfonic acid and acetic acid.
The compound isolated in the form of a salt can be collected as an amino group by adding an inorganic base or an organic base thereto for neutralization, and then crystallizing the same with a poor solvent.

2) Explanation of a method of protecting the amino group of the compound having the amino group, converting the same to an intermediate in which the reactivity of the carboxylic group to be reacted is increased, and then reacting the same with the compound having one or more ethynyl groups.

In this method, when protecting the compound represented by Formula (5) and reacting the same with the compound represented by Formula (7), the compound represented by Formula (5) is converted to an active intermediatae represented by Formula (6) and then allowed to react.

The compound represented by Formula (6) is preferably synthesized by reacting the compound represented by Formula (5) with an activator after protecting the compound represented by Formula (5).
When L in the compound represented by Formula (6) is a chlorine atom, examples of the activator include chlorine, thionyl chloride, oxalyl chloride, phosphorous pentachloride, N-chlorosuccinimide and carbon tetrachloride; when L is a bromine atom, examples of the activator include bromine, N-bromosuccinimide and carbon tetrabromide; when L is a sulfonyl derivative, examples of the activator include methanesulfonyl chloride and p-toluenesulfonyl chloride; and when L is an acid anhydride, examples of the activator include alkyl chlorocarbonates such as methyl chlorocarbonate, ethyl chlorocarbonate and isopropyl chlorocarbonate.
Among these, a method of using thionyl chloride, oxalyl chloride or methanesulfonyl chloride is preferred. Although the activator may be added to the reaction system from the beginning of the reaction, it is preferred to drop the activator in the reaction system during the reaction.

When synthesizing the compound represented by Formula (6) from the compound represented by Formula (5) having the amino group being protected, a base may be added to the reaction system as necessary. The base that can be used is not particularly limited, and both organic base and an inorganic base can be used.
The amount of the activator used in the synthesis of the compound represented by Formula (6) is preferably from 1.0 to 20 times by mole, more preferably from 1.0 to 3.0 times by mole, further preferably from 1.0 to 2.2 times by mole, in view of the fact that the acetylene compound represented by Formula (1) where -B- is a structure represented by Formula (3) can be obtained at high yield, and that the amount of unreacted activator is small. When the above amount is less than 1.0 times by mole, it is not preferred since the yield may decrease because of the invebitable generation of the unreacted compound represented by Formula (5). When the above amount is more than 20 times by mole, it is not preferred since the production cost may increase due to the use of surplus raw materials, although it would not become a significant obstacle to the reaction.

The solvent used for the reaction is not particularly limited as long as it does not cause problems in the process operation or hamper the progress of reaction, as well as it does not adversely affect the reaction during the process of halogenation, acid-anhydrization or sulfonyl-derivatization of the invention. Examples of the solvent include amide-based solvents (such as N.N-dimethylformamide, N,N-dimethylacetamide and 1-methyl-2-pyrrolidone), sulfone-based solvents (such as sulfolane), sulfoxide-based solvents (such as dimethylsulfoxide), ureido-based solvents (such as tetramethylurea), ether-based solvents (dioxane and cyclopenthyl methyl ether), ketone-based solvents (such as acetone and cyclohexanone), hydrocarbon-based solvents (such as toluene, xylene and n-decane), halogen-based solvents (such as tetrachloroethane and chlorobenzene), pyridine-based solvents (such as pyridine, γ-picoline and 2,6-lutidine), ester-based solvents (such as ethyl acetate and butyl acetate) and nitrile-based solvents (such as acetonitrile), and these solvents may be used alone or in combination of two or more kinds. Among these, amide-based solvents, sulfone-based solvents, sulfoxide-based solvents, ureido-based solvents, ether-based solvents, halogen-based solvents, pyridine-based solvents and nitrile-based solvents are preferred; amide-based solvents, ether-based solvents, halogen-based solvents and nitrile-based solvents are more preferred; and amide-based solvents and nitrile-based solvents are further preferred. These solvents may be used alone or in combination of two or more kinds.

The reaction temperature is preferably within a range of from -30°C; to 50°C, more preferably from -20°C to 30°C, further preferably from -10°C to 20°C. The reaction time may differ depending on the amount of raw materials or the reaction temperature, but is preferably within a range of from 0.5 to 12 hours, more preferably from 0.5 to 6 hours. The reaction is preferably carried out in a sufficiently dried inert gas atmosphere. Since the presence of moisture lowers the reaction rate, the amount thereof is preferably as small as possilbe. Specifically, noble gases such as nitrogen or argon are suitably used as the inert gas.

Next, the reaction of the compound represented by Formula (6) and the compound represented by Formula (7) is explained.
The amount of the acetylene compound represented by Formula (7) with respect to the compound represented by Formula (5) is preferably in a range of from 1.5 to 10 times by mole, more preferably from 1.8 to 3.0 times by mole, further preferably from 1.9 to 2.2 times by mole, in view of the fact that the intended compound can be obtained at high yield, and that the amount of an unreacted product of the compound represented by Formula (5), which is used as the raw material, is small.

The method of adding the compound represented by Formula (7) is not particularly limited, but the compound is preferably added dropwise since heat may be produced by the addition thereof.
When the compound represented by Formula (6) is used without being isolated, it is possible to use the same solvent that was used in the synthesis of the compound represented by Formula (6). When the compound represented by Formula (6) is isolated and newly prepared, the solvent is not particularly limited as long as it does not cause problems in the process operation or hamper the progress of reaction, as well as it does not decompose to adversely affect the reaction in the process of amidization, esterification or thioesterification of the invention. Examples of the solvent include amide-based solvents (such as N,N-dimethylformamide, N,N-dimethylacetamide and 1-methyl-2-pyrrolidone), sulfone-based solvents (such as sulfolane), sulfoxide-based solvents (such as dimethylsulfoxide), ureido-based solvents (such as tetramethylurea), ether-based solvents (dioxane and cyclopenthyl methyl ether), ketone-based solvents (such as acetone and cyclohexanone), hydrocarbon-based solvents (such as toluene, xylene and n-decane), halogen-based solvents (such as tetrachloroethane and chlorobenzene), pyridine-based solvents (such as pyridine, γ-picoline and 2,6-lutidine), ester-based solvents (such as ethyl acetate and butyl acetate) and nitrile-based solvents (such as acetonitrile), and these solvents may be used alont or in combination. Among these, amide-based solvents, sulfone-based solvents, sulfoxide-based solvents, ureido-based solvents, ether-based solvents, halogen-based solvents, pyridine-based solvents and nitrile-based solvents are preferred; amide-based solvents, ether-based solvents, halogen-based solvents and nitrile-based solvents are more preferred; and amide-based solvents and nitrile-based solvents are further preferred. These solvents may be used alone or in combination of two or more kinds.

The reaction temperature is preferably within a range of from -30°C to 200°C, more preferably from -10°C to 10°C, further preferably from 0°C to 50°C. The reaction time may differ depending on the amount of raw materials or the reaction temperature, but is preferably within a range of from 0.1 to 12 hours, more preferably from 0.5 to 6 hours. The reaction is preferably carried out in a sufficiently dried inert gas atmosphere. Since the presence of moisture may cause decomposition of the compound represented by Formula (6), the amount thereof is preferably as small as possilbe. Specifically, noble gases such as nitrogen or argon are suitably used as the inert gas.
After the reaction, the acetylene compound is collected under the same conditions for collection as those described in the above process 1).
The conditions for deprotecting the protective group for the amino group are the same as the reaction conditions as described in 1) or the like.

When X in the novel acetylene compound according to the invention represents -O-, -NR-, -S-, -SO-, -SO₂-, -CO- or -CS-, the method of producing the same is not particularly limited as long as it is an ordinary method of synthesizing an ether bond, a thioether bond or a substituted amine. For example, the acetylene compound according to the invention can be suitably produced by a method described on pages 273 to 412 of Chemical Reviews (1951), Vol. 49; pages 1699 to 1875 of Shin Jikken Kagaku Koza 14, Synthesis and Reaction of Orgainc Compounds (III), published by Maruzen Co., Ltd.; or pages 149 to 353 of Jikken Kagaku Koza, 4th Edition, Vol. 21, Organic Synthesis II, published by Maruzen Co., Ltd.

A further embodiment of the invention is a composition including an acetylene compound according to at least one of the aforementioned <1> to <14> and/or a polymer according to at least one of the aforementioned <15> to <19>. This composition preferably includes a polymer according to at least one of the aforemenetioned <15> to <19>, and may further include other additives of various kinds at an arbitrary amount, according to applications or purposes in each industrial field to which the composition is used as a final product or an intermediary product, and exemplary applications include functional materials such as liquid cyrstal materials, non-linear optical materials, electronic materials (such as semiconductor protection films and substrates for a flexible print curcuit board), materials for adhesive, materials for lubricant, additives for photography, and raw materiasl for medical and agricultural intermediates.

### <Other additives>

Examples of the other additives include polymerizable compounds, resins, crosslinkable resins, solvents, polymerization initiators, colorants, polymerization inhibitors, fillers, silane coupling agents and release agents.

Exemplary polymerizable compounds include an addition-polymerizable compound having at least one ethylenically unsaturated double bond, which can be selected from compounds having at least one ethylenically unsaturated bond, preferably two or more. These compounds are widely known in this industrial field, and can be used in the invention without any particular limitation. These compounds have a chemical structure of a monomer, a prepolymer (a dimer, a trimer or an oligomer), a mixture thereof, a copolymer thereof, or the like. Examples of the monomer or the copolymer thereof include unsaturated carboxylic acids (such as acrylic acid, methacrylic acid, itaconic acid, crotonic acid, isocrotonic acid and maleic acid), esters thereof, and amides thereof. Esters of an unsaturated carboxylic acid and an aliphatic polyhydric alcohol compound, and amides of an unsaturated carboxylic acid and an aliphatic polyhydric alcohol compound are preferably used. Further, an addition-reaction product obtained from an unsaturated carboxylic acid ester or an amide having a nucleophilic substituent such as a hydroxy group, an amino group or a mercapto group and a monofunctional or polyfunctional isocyanate or epoxy compound; and a dehydration-condensation-reaction product obtained from an unsaturated carboxylic acid ester or an amide and a monofunctional or polyfunctional carboxylic acid, are also suitably used. Moreover, an addition-reaction product obtained from an unsaturated carboxylic acid ester or an amide having an electrophilic substituent such as an isocyanate group or an epoxy group and a monofunctional or polyfunctional alcohol, amine or thiol; and a substitution-reaction product obtained from an unsaturated carboxylic acid ester or an amide having a leaving substituent such as a halogen group or a tosyloxy group and a monofunctional or polyfunctional alcohol, amine or thiol are also suitable. Further examples include compounds in which the aforementioned unsaturated carboxylic acid is substituted with an unsaturated phosphonic acid, styrene, vinyl ether or the like.

Further, for example, the aliphatic alcohol-based esters described in Japanese Patent No. 46-27926, Japanese Patent No. 51-47334 and JP-A No. 57-196231; the compounds having an aromatic skeleton described in JP-A Nos. 59-5240, 59-5241 and 2-226149; the compounds having an amino group described in JP-A No. 1-165613 are also preferably used. Moreover, the aforementioned ester monomers may be used as a mixture.

Specific examples of the monomer of an amide formed from an aliphatic polyvalent amine compound and an unsaturated carboxylic acid include methylenebis-acrylamide, methylenebis-methacrylamide, 1,6-hexamethylenebis-acrylamide, 1,6-hexamethylenebis-methacrylamide, diethylenetriamine trisacrylamide, xylylene bisacrylamide, and xylylene bismethacryalmide. Other examples of the preferred amide-based monomers include those having a cyclohexylene structure as described in Japanese Patent No. 54-21726.

Urethane-based addition-polymerizable compounds, which are produced via addition reaction of an isocyanate and a hydroxy group, are also suitable. Specific examples thereof include vinylurethane compounds having two or more polymerizable vinyl groups in one molecule as described in JP-A No. 2004-252201, which are obtained by adding a vinyl monomer having a hydroxy group to a polyisocyanate compound having two or more isocyanato groups in one molecule as described in Japanese Patent No. 48-41708.

Further, urethane acrylates as described in JP-ANo. 51-37193, Japanese Patent No. 2-32293 and Japanese Patent No. 2-16765; urethane compounds having an ethyleneoxide-based skeleton as described in Japanese Patent No. 58-49860, Japanese Patent No. 56-17654, Japanese Patent No. 62-39417 and Japanese Patent No. 62-39418; and addidtion-polymerizable compounds having an amino structure or a sulfide structure in its molecule as described in JP-ANo. 63-277653, JP-A No. 63-260909 and JP-A No. 1-105238 can also be mentioned.

Further examples include polyfunctional acrylates or methacrylates such as polyester acrylates and epoxy acrylates obtained by reacting an epoxy resin with (meth)acrylic acid, as described in JP-A No. 48-64183, Japanese Patent No. 49-43191 and Japanese Patent No. 52-30490. Further, specific unsaturated compounds as described in Japanese Patent No. 46-43946, Japanese Patent No. 1-40337 and Japanese Patent No. 1-40336, and vinylphosphonic acid-based compounds as described in JP-A No. 2-25493 can also be mentioned. In certain cases, a structure having a perfluoroalkyl group as described in JP-A No. 61-22048 is suitably used. Further, photo-curable monomers or oligomers as described on pages 300 to 308 of Jounal of the Adhesion Society of Japan, Vol. 20, No. 7 (1984) are also applicable. In addition, polymerizable compounds as described in JP-A No. 2004-252201, 3P-A No. 2007-138105 and JP-A No. 2007-177177 are also applicable.

Radically polymerizable or crosslinkable monomers, oligomers and polymers which are available as commercial products or known in the industry that can be used in the invention include those described in "Crosslinking Agent Handbook" (1981), edited by Shinzo Yamashita, published by Taiseisha Ltd.; "UV-EB Curing Handbook -Raw Materials-" (1985) edited by Seishi Kato, published by KobunshiKankokai; "Applications and Markets for UV-EB curing techniques" (1989), edited by RadTech Japan, published by CMC Publishing Co., LTd., p. 79; and "Polyester Resin Handbook" (1988), authored by Eiichiro Takiyama, published by the Nikkan Kogyo Shinbun, Ltd.

As the polymerization compound, photo-curable polymerizable compound materials used for a photopolymerizable composition described in the following documents are also suitably used in the invention: JP-A No, 7-159983, JP-A No. 7-31399, JP-A No. 8-224982, JP-A No. 10-863 and JP-A No. 9-134011.

Further examples include aromatic vinyl compounds such as styrene, vinyl toluene and α-methylstyrene; vinylesters such as vinyl acetate, vinyl propionate and vinyl versatate; allylesters such as allyl acetate; halogen-containing monomers such as vinylidene chloride and vinyl chloride; vinyl cyanides such as (meth)acrylonitrile; and high-boiling olefins.

Exemplary resins that may be optionally added include alkyd-based resin, polyester-based resin, polyether-based resin, polyurethane-based resin, vinyl-based resin, acrylic-based resin, rubber-based resin, polyolefin-based resin, polyurea-based resin, melamine resin, epoxy resin, nylon resin, polyimide resin, polystyrene-based resin, polyacetal resin, polybutyral resin, polyketal resin, novolac resin, resol resin, silicone resin, cellulose-modified resin, and waxes.

A crosslinking agent may be added to the composition according to the invention in order to adjust the curability or the curing rate thereof. The crosslinking agent is not particularly limited as long as it can cure a film by crosslinking reaction, and those that form a crosslinked structure by heat, light, UV rays, electron beams or the like are applicable. Exemplary crosslinking agents include polyisocyanate, polyimide derivatives, epoxy resin, melamine compounds or guanamine compounds that are substituted by a methylol group and at least one selected from an alkoxymethyl group or an acyloxymethyl group, glycoluril compounds or urea compounds, phenol compounds that are substituted by a methylol group and at least one selected from an alkoxymethyl group or an acyloxymethyl group, naphthol compounds, and hydroxyanthracene compounds. Among these, a polyfunctional epoxy resin is particularly preferred.

The epoxy resin is not particularly limited as long as it has an epoxy group and an ability of crosslinking, and examples thereof include divalent glycidyl group-containing low-molecular compounds such as bisphenol-A-diglycidyl ether, ethylene glycol diglycidyl ether, butanediol diglycidyl ether, hexanediol diglycidyl ether, dihydroxybiphenyl diglycidyl ether, phthalic diglycidyl ether, and N,N-diglycidyl aniline; trivalent glycidyl group-containing low-molecular compounds such as trimethylolpropane triglycidyl ether, trimethylolphenol triglycidyl ether and TrisP-PA triglycidyl ether; tetravalent glydidyl group-containing low-molecular compounds such as pentaerythritol tetraglycidyl ether and tetramethylol bisphenol-A-tetraglycidyl ether; and glycidyl group-containing high-molecular compounds such as polyglycidyl (meth)acrylate and a 1,2-epoxy-4-(2-oxyranyl)cyclohexane-adduct of 2,2-bis(hydroxymethyl)-1-butanol

Commercially available products include bisphenol A-type epoxy resins such as EPICOAT 828 EL and EPICOAT 1004 (manufactured by Japan Epoxy Resins Co., Ltd.) and EPICOAT 806 and EPICOAT 4004 (manufactured by Japan Epoxy Resins Co., Ltd.); bisphenol F-type epoxy resins such as EPICLON 830 CRP (manufactured by DIC Corporation); bisphenol S-type epoxy resins such as EPICLON EXA 1514 (manufactured by DIC Corporation); 2,2'-diallyl bisphenol A-type epoxy resins such as RE-810 NM (manufactured by Nippon Kayaku Co., Ltd.); hydrogenated bisphenol-type epoxy resins such as EPICLON EXA 7015 (manufactured by DIC Corporation); propylene oxide-added bisphenol A-type epoxy resins such as EP-40005 (manufactured by ADEKA Corporation); resolcinol-type epoxy resins such as EX-201 (manufactured by Nagase ChemTeX Corporation); biphenyl-type epoxy resins such as EPICOAT YX-4000H (manufactured by Japan Epoxy Resins Co., Ltd.); sulfide-type epoxy resins such as YSLV-50TE (manufactured by Tohto Kasei Co., Ltd.); ether-type epoxy resins such as YSLV-80DE (manufactured by Tohto Kasei Co., Ltd.); dicyclopentadiene-type epoxy resins such as EP-4088S (manufactured by ADEKA Corporation); naphthalene-type epoxy resins such as EPICLON HP 4032 and EPICLON EXA-4700 (manufactured by DIC Corporation); phenol novolac-type epoxy resins such as EPICLON N-770 (manufactured by DIC Corporation); orthocresol novolac-type epoxy resins such as EPICLON N-670-EXP-S (manufactured by DIC Corporation); dicyclopentadiene novolac-type epoxy resins such as EPICLON HP 7200 (manufactured by DIC Corporation); biphenyl novolac-type epoxy resins such as NC-3000P (manufactured by Nippon Kayaku Co., Ltd.); naphthalene phenol novolac-type epoxy resins such as ESN-165S (manufactured by Tohto Kasei Co., Ltd.); glycidylamine-type epoxy resins such as EPICOAT 630 (manufactured by Japan Epoxy Resins Co., Ltd.), EPICLON 430 (manufactured by DIC Corporation) and TETRAD-X (manufactured by Mitsubishi Gas Chemical Company, Inc.); alkylpolyol-type epoxy resins such as ZX-1542 (manufactured by Tohto Kasei Co., Ltd.). EPICLON 726 (manufacrued by DIC Corporation), EPOLITE 80 MFA (manufactured by Kyoeisha Chemical Co., Ltd.) and DENACOL EX-611 (manufactured by Nagase ChemTeX Corporation); rubber modified-type epoxy resins such as YR-450 and YR-207 (manufactured by Tohto Kasei Co., Ltd.) and EPOLEAD PB (manufactured by Daicel Chemical Industries, Ltd.); glycidylester compounds such as DENACOL EX-147 (manufactured by Nagase ChemTeX Corporation); bisphenol A-type episulfide resins such as EPICOAT YL-7000 (manufactured by Japan Epoxy Resins Co., Ltd.); and other products such as YDC-1312, YSLV-80XY and YSLV-90CR (manufactured by Tohto Kasei Co., Ltd.), XAC 4151 (manufactured by Asahi Kasei Corporation), EPICOAT 1031 and EPICOAT 1032 (manufactured by Japan Epoxy Resins Co., Ltd.), EXA-7120 (manufacrued by DIC Corporation), and TEPIC (manufactured by Nissan Chemical Industries, Ltd.)

The amount of the epoxy resin to be formulated is not particularly limited, and may be adjusted as appropriate according to the type or the amount of the aforementioned (meth)acrylate, such as an epoxy (meth)acrylate.

### <Thermal-curing agent>

A thermal-curing agent may be included in the composition according to the invention in order to further promote the thermal curing of the epoxy resin or the like. The thermal-curing agent is used to cause reaction of unsaturated bonds, epoxy groups or the like in the curable resin by heating, thereby allowing the same to crosslink. Therefore, the thermal-curing agent plays a role of improving adhesion or moisture resistance of the cured product after being cured. The thermal-curing agent is not particularly limited, but when the composition according to the invention is cured at a relatively low temperature of, for example, from 100 to 150°C, the thermal-curing agent preferably includes an amine group and/or a thiol group that has superior low-temperature reactivity.

Examples of the thermal-curing agent having an amine group and/or a thiol group include organic acid dihydrazide compounds such as 1,3-bis[hydrazinocarbonoethyl-5-isopropylhydantoin] and adipic acid dihydrazide; dicyanamide, guanidine derivatives, 1-cyanoethyl-2-phenylimidazole, N-[2-(2-methyl-1-imidazolyl)ethyl)urea, 2.4-diamino-6-[2'-methylimidazolyl-(1')]-ethyl-s-triazine, N,N'-bis(2-methyl-I-imidazolylethyl)urea, N,N'-(2-methyl-1-imidazolylethyl)-adipamide, 2-phenyl-4-methyl-5-hydroxymethylimidazole, 2-imidazoline-2-thiol, 2-2'-thiodiethanethiol, and addition products of various amines and an epoxy resin. These thermo-curing agents may be used alone or in combination of two or more kinds.

A solvent may be added to the composition according to the invention. The solvent is not particularly limited as long as it does not hamper the progress of reaction during curing the composition or the like, or does not adversely affect the preservation storability of the composition. Exemplary solvents include amide-based solvents (such as N,N-dimethylformamide, N,N-dimethylacetamide and 1-methyl-2-pyrrolidone), sulfone-based solvents (such as sulfolane), sulfoxide-based solvents (such as dimethylsulfoxide), ureido-based solvents (such as tetramethylurea), ether-based solvents (dioxane and cyclopenthyl methyl ether), ketone-based solvents (such as acetone and cyclohexanone), hydrocarbon-based solvents (such as toluene, xylene and n-decane), halogen-based solvents (such as tetrachloroethane and chlorobenzene), pyridine-based solvents (such as pyridine, γ-picoline and 2,6-lutidine), ester-based solvents (such as ethyl acetate and butyl acetate) and nitrile-based solvents (such as acetonitrile), and these solvents may be used alone or in combination. Among these, amide-based solvents, sulfone-based solvent, sulfoxide-based solvents, ureido-based solvents, ether-based solvents, halogen-based solvents, pyridine-based solvents and nitrile-based solvents are preferred; amide-based solvents, ether-based solvents, halogen-based solvents and nitrile-based solvents are more preferred; and amide-based solvents and nitrile-based solvents are further preferred. These solvents may be used alone or in combination of two or more kinds. The amount of the solvent to be added to the composition according to the invention may be selected according to applications or necessary performances in the applied area, but is preferably from 0 to 90% by mass, more preferably from 0 to 80% by mass, further preferably from 0 to 70% by mass, with respect to the total amount of the composition. Further, use of a solvent may not be preferred in some cases.

The composition according to the invention may include a polymerization initiator such as a photo-polymerization initiator or a thermal-polymeirzation initiator, in order to promote polymerization of a polymerizable compound or reaction of a crosslinking agent.
Exemplary photo-polymerization initiators include photo-initiators described in JP-A No. 2004-252201; peroxides described in the U.S. Patent No. 4,950,581; aromatic sulfoniums, phosphoniums or iodiniums described in the U.S. Patent No. 4,950,581; cyclopentadienyl-arene-metal complex salts, oxime sulfonic acid esters described in European Patent No. 780,729; and pyridinium and (iso)quinolinium salts described in European Patent Nos. 497,531 and 441,232. Further examples include halomethyltriazines described in G. Buhr, R. Dammel and C. Lindley, Polym. Mater. Sci. Eng. 61, 269 (1989) and European Patent No. 022788; halomethyloxazole photoinitiators described in the U.S. Patent Nos. 4,371,606 and 4,371,607; 1,2-disulfones described in E.A.Bartmann, Synthesis 5,490 (1993); hexaarylbisimidazole and a hexaarylbisimidazole/co-initiator system (such as 2-mercaptobenzthiazole and ferrocenium compounds), or titanocenes (such as a mixture of o-chlorohexaphenyl-bisimidazole and bis(cyclopentadienyl)-bis(2,6-difluoro-3-pyrylphenyl)titanium). A photosensitizer may be used in combination, and examples thereof include amines such as triethanolamine and 4-dimethylaminobenzoic acid ethyl ester, benzophenone and derivatives thereof, thioxanthone and derivatives thereof, anthraquinone and derivatives thereof, and coumarin derivatives.

Exemplary thermo-polymerization initiators include azo compounds such as 2,2'-azobis(4-methoxy-2,4-dimethylvaleronitrile), triazene, diazosulfide and pentaazadiene; and organic peroxides such as hydroperoxide, peroxycarbonate and tert-butylhydroperoxide. Organic peroxides that do not produce air bubbles are preferably used as the thermo-polymerization initiator. Widely used organic peroxides are applicable, and examples thereof inlcude peroxydicarbonate, peroxyester, peroxyketal, ketoneperoxide and hydroperoxide. These organic peroxides may be used alone or in combination of two or more kinds, and may be used by diluting with a solvent or adsorbing to a powder material. The amount of the polymerization initiator is preferably from 0.01 to 10% by mass with respect to the total amount of the composition. When the above proportion is less than 0.01 % by mass, the degree of curing during heating may not be sufficient; and when exceeds 10% by mass, the curing reaction may be adversely affected.

Further, for the purpose of suppressing undesired reaction during storage, known additives such as polymerization inhibitors, chain transfer agents, UV absorbers or stabilizers may be added to the composition according to the invention. Exemplary polymerization inhibitors include hydroquinone, hydroquinone derivatives, p-methoxyphenol, and sterically-hindered phenols such as 2,6-di-tert-butyl-p-cresol. In order to improve the stability during dark storage, copper compounds (such as copper naphthenate, copper stearate and copper octanoate), phosphorous compounds (such as triphenyl phosphine, tributyl phosphine, triethyl phosphite, triphenyl phosphite and tribenzyl phosphite), quartenary ammoniuom compounds (such as tetramethylammonium chloride and trimethylbenzyl ammonium chloride) or hydroxyamine derivatives (such as N-diethymydroxyamine) may be added. Exemplary chain transfer agents include mercaptan, amine and benzothiazole.

Further, a small amount of light stabilizer may be added, and examples thereof include UV absorbers (such as hydroxyphenylbenzotriazole, hydroxyphenylbenzophenone, oxalamide or hydroxyphenyl-s-triazine-type). These compounds may be used alone or in combination of two or more kinds, under the presence or absence of a sterically-hindered amine. Exemplary UV absorbers or light stabilizers include 2-(2'-hydroxyphenyl)benzotriazole, 2-hydroxybenzophenone, substituted or unsubstituted benzoates, acrylates, sterically-hindered amines, oxalamides, 2-(2-hydroxyphenyl)-1,3,5-triazine, phosphite esters, and phosphonate esters.

The composition according to the invention may include other components such as a known silane coupling agent, a fluidity modifier or an adhesion promotor, in order to improve the adhesion of the composition. Exemplary silane coupling agents include vinyltrimethoxysilane, vinyltriethoxysilane, vinyltris(2-methoxyethoxy)silane, vinyltrichlorosilane (KA-1003, manufactured by Shin-Etsu Chemical Co., Ltd.), 2-(3,4epoxycyclohexyl)ethyltrimethoxysilane (KBM-303, manufactured by Shin-Etsu Chemical Co., Ltd.), p-styryltrimethoxysilane (KBM-1403, manufactured by Shin-Etsu Chemical Co., Ltd.), 3-methacryloxypropylmethyldimethoxysilane (KBM-502, manufactured by Shin-Etsu Chemical Co., Ltd.), 3-acryloxypropyltrimethoxysilane (KBM-5103, manufactured by Shin-Etsu Chemical Co., Ltd.), 3-aminopropyltriethoxysilane, 3-aminopropyltrimethoxysilane (KBM-903, manufactured by Shin-Etsu Chemical Co., Ltd.), N-(2-aminoethyl)-3-aminopropylmethyldimethoxysilane, N-(2-aminoethyl)-3-aminopropyltrimethoxysilane, 3-glycidoxypropyltrimethoxysilane, 3-glycidoxypropylmethyldimethoxysilane, 3-chloropropylmethyldimethoxysilane, 3-chloropropyltrimethoxysilane, and 3-mercaptopropyltrimethoxysilane.

### <Filler>

The composition according to the invention may include a filler in order to adjust the viscosity or preservation stability thereof, or the rigidity, viscoelasticity, bulk density or expansion coefficient of the cured product. The filler is not particularly limited, and examples thereof include inorganic fillers such as silica, diatom earth, alumina, zinc oxide, iron oxide, magnesium oxide, tin oxide, titanium oxide, alumina, magnesium hydroxide, aluminum hydroxide, carcium carbonate, magnesium carbonate, barium sulfate, magnesium sulfate, plaster, calcium silicate, aluminum silicate, zirconium silicate, potassium titanate, kaolin, talc, glass beads, sericite, activated clay, bentonite, aluminum nitride, silicon nitride, glass microparticles described in the U.S. Patent No. 5,013,768, or micronized glass fibers; and known organic fillers such as polymethyl(meth)acrylate, polystyrene, copolymers of these polymers and a monomer than can be copolymerized with these polymers, polyester microparticles, polyurethane microparticles, and rubber microparticles.

### <Colorant>

The composition according to the invention may include a colorant such as a dye or a pigment in view of texture, appearance or design. Known dyes including those commercially obtainable or those described in "Dye Handbook", edited by the Society of Synthetic Organic Chemistry, Japan, (1970), or the like, can be used in the invention. Specific examples of the dye include triarylmethane-based dyes, carbonium-based dyes, anthraquinone-based dyes, naphthoquinone-based dyes, quinone-imine-based dyes, azomethine-based dyes, azo-based dyes, metal complex salt azo-based dyes, benzilidene-based dyes, oxonol-based dyes, cyanine-based dyes, phenothiazine-based dyes, xanthene-based dyes, phthalocyanine-based dyes, benzopyrane-based dyes, indigo-based dyes, methine-based dyes, azine-based dyes, oxazine-based dyes, thiazine-based dyes, anthrapyridone-based dyes, squarylium-based dyes, pyrylium salt-based dyes, and metal thiolate-based complexes.
Preferred dyes include the cyanine dyes described in JP-A No. 58-125246, JP-A No. 59-84356, JP-A No. 59-202829 and JP-A No. 60-78787; the methine dyes described in JP-A No. 58-173696, JP-A No. 58-181690 and JP-A No. 58-194595; the naphthoquinone dyes described in JP-A No. 58-112793, JP-A No. 58-224793, JP-A No. 59-48187, JP-A No. 59-73996, JP-A No. 60-52940 and JP-A No. 60-63744; the squarylium dyes described in JP-A No. 58-112792; and the cyanine dyes described in British Patent No. 434,875.

Further examples of the dye include the near-infrared-absorbing sensitizers described in the U.S. Patent No. 5,156,938; the substitued arylbenzo(thio)pyrylium salts described in the U.S. Patent No. 3,881,924; the trimethinethiapyrylium salts described in JP-A No. 57-142645 (the U.S. Patent No. 4,327,169); the pyrylium compounds described in JP-A Nos. 58-181051, 58-220143, 59-41363, 59-84248, 59-84249, 59-146063 and 59-146061; the cyanine dyes described in JP-A No. 59-216146; the pentamethinethiopyrylium salts described in the U.S. Patent No. 4,283,475; and the pyrylium compounds described in Japanese Patent Nos. 5-13514 and 5-19702.
Futher preferred examples of dye include the near-infrared-absorbing dyes described in the U.S. Patent No. 4,756,993.
It is also possile to use the dyes disclosed in JP-A No. 64-90403, JP-A No. 64-91102, JP-A No. 1-94301, JP-A No. 6-11614, Japanese Patent No. 2592207, the U.S. Patent No. 4,808,501, the U.S. Patent No. 5,667,920, the U.S. Patent No. 5,059,500, JP-A No. 5-333207, JP-A No. 6-35183, JP-A No. 6-51115, JP-A No. 6-194828 and JP-A No. 2004-252201.
Since many dyes may cause decrease in sensitivity of the polymerization system, a pigment is particularly preferably used as the colorant.

Examples of the pigment that may be optionally added in the invention include commercially available pigments and pigments described in the Color Index (C.I.) Handbook; "Saishin Ganryou Binran (Newest Pigment Handbook)", published by the Japan Society of Color Material, 1977; "Saishin Ganryou Ouyou Gjutsu (Newest Pigment Application Technique)", published by CMC Publishing Co., Ltd., 1986; "Insatsu Ink Gijutsu (Printing Ink Technique)", published by CMC Publishing Co., Ltd., 1984; JP-A No. 2004-252201; JP-A No. 2007-138105; and JP-A No. 2007-177177.

Exemplary pigments that can be used in the invention include black pigments, yellow pigments, orange pigments, brown pigments, red pigments, purple pigments, blue pigments, green pigments, fluorescent pigments, metal powder pigments, and polymer-bonded colorants. Specific examples thereof include insoluble azo pigments, azo lake pigments, condensed azo pigments, chelate azo pigments, phthalocyanine pigments, anthraquinone pigments, perylene and perinone-based pigments, thioindigo-based pigments, quinacridone-based pigments, dioxazine-based pigments, isomdolinone-based pigments, quinophthalone-based pigments, colored lake pigments, azine pigments, nitroso pigments, nitro pigments, natural pigments, phosphorescent pigments, inorganic pigments, and carbon black. Among these pigments, carbon black is preferred.

Surface treatment of the pigment may be conducted or may not. Methods of the surface treatment include a method of coating the surface of the pigment with resin or wax, a method of attaching a surfactant to the surface of the pigment, and a method of binding a reactive substance (such as a silane coupling agent, an epoxy compound or a polyisocyanate) to the surface of the pigment. The above surface treatment methods are described in "Kinzoku Sekken no Seishitu to Ouyou (Properties and Applications of Metal Soap)", publizhed by Saiwai Shobo; "Insatsu Ink Gijutsu (Printing Ink Technique)", published by CMC Publishing Co., Ltd., 1984; and "Saishin Ganryou Ouyou Gjutsu (Newest Pigment Application Technique)", published by CMC Publishing Co., Ltd., 1986.

The particle diameter of the pigment is preferably in a range of from 0.01 µm to 10 µm, more preferably from 0.05 µm to 1 µm, particularly preferably from 0.1 µm to 1 µm. When the particle diameter of the pigment is 0.01 µm or greater, stability of materials dispersed in the coating liquid for forming an image recording layer can be improved; and when the particle diameter of the pigment is 10 µm or less, uniformity of the image recording layer can be improved.

Dispersing of the pigment may be carried out by known dispersing techniques used for the production of inks and toners. Exemples of the dispersing machine include a sand mill, an attritor, a pearl mill, a super mill, a ball mill, an impller, a disperser, a KD mill, a colloid mill, a dynatron, a three-roll mill, and a pressure kneader. Details of these machines are described in "Saishin Ganryou Ouyou Gjutsu (Newest Pigment Application Technique)", published by CMC Publishing Co., Ltd., 1986.

In addition, known additives may be added to the composition according to the invention, as necessary. Examples of the additives that can be used include surfactants, matting agents, promotors and co-initiaors such as thiol, thioether, disulfide, phosphonium salts, phosphine oxide and phosphine described in European Patent No. 438,123, British Patent No. 2,180,358 and JP-A No. 6-68309, autoxidizers, optical brightners, moisturerizers, smoothing aids, dispersants, coagulation inhibitors, defoaming agents, leveling agents, ion-trap agents, ion-exchange agents, plasticizers such as dioctylphthaltate, didodecylphthaltate, triethylene glycol dicaprylate, dimethyl glycol phthalate, tricresyl phosphate, dioctyl adipate, dibutyl sebacate and triacetylglycerin, and other additives.

The additives as mentioned above are preferably selected in accordance with the intended purpose of the composition or the characteristics required in the intended porpose. The additives as mentioned above are commonly used in the present technique, and therefore these additives are preferably added in an ordinary amount with respect to each intended purpose.

A further embodiment of the invention is a cured product produced by curing a composition including the acetylene compound according to at least one of <1> to <14> and/or the polymer according to at least one of <15> to <19>; or a cured product produced by curing the polymer according to <15> to <19>. Methods of obtaining the cured product include a method of heating and drying the composition of the invention as mentioned above and/or at least one of the polymer according to <15> to <19> or a solution thereof; and a method of melting and solidifying a powder of the composition according to the invention. However, the invention is not limited thereto.
The obtained polymer according to the invention has an acetylene group as a structural component, and by allowing this acetylene group to further polymerize, a cured product having even more improved mechanical properties and heat resistance can be obtained (here, a product produced by the reaction of acetylene in the polymer is referred to as the "cured product"). The method of reaction of the acetylene group is not particularly limited, but it is possible to progress a reaction among acetylene groups (polymerization reaction) by applying heat, light or radiation. By causing the polymerization reaction of acetylene groups, the obtained product (cured product) has a branched or three-dimensional structure, thereby making it possible to obtain a shaped product having excellent tensile modulus of elasticity or heat resistance (glass transition temperature).

The composition according to the invention has superior preservation stability without occurrence of polymerization during preservation. When energy such as heat, light, UV rays or electron beams is imparted, polymerization of a polymerizable compound in the composition is efficiently initiated in a short time, the effectiveness of which depending on the nature of the crosslinking group or polymerizable group, or a crosslinking group incorporated in a side chain, main chain or terminal of the polymer chain or the compound according to the invention crosslinks and forms a cured resin product. As a result, the thus obtained cured product is insoluble with respect to an organic solvent and exhibits improved solvent resistance, chemical resistance, heat resistance, mechanical strength or the like. Therefore, the composition according to the invention can be formed into various kinds of shaped products through various kinds of shaping methods, as a matrix resin of various kinds being soluble in an organic solvent. Moreover, the compositon is highly versatile and by curing the same by crosslinking after the shaping, the resulting product exhibits excellent solvent resistance, chemical resistance or mechanical strength. Therefore, the cured product can be used as an excellent resin material, and is suitably used for a mechanical member or an electrical-resistance body. Accordingly, the composition according to the invention is suitably used also for printing inks (such as inks for screen printing, off-set printing and flexographic printing); clear finishing agents (such as white or color finishing agents applied to wood or metal); powder coatings (in particular, coating materials applied to paper, wood, metal or plastics); marking materials for architecture, objects or roads; materials for photoduplication, holographic recording, image recording or production of print precursors; sun light-curable coating materials for producing masks used in screen printing; filler compositions for dental use; adhesives; pressure-sensitive ahesives; resins for forming a laminate; etching resists in the form of a liquid or a film; soldering resists; electroplating resists; permanent resists; dielectrics used for print curcuit boards or electic circuits, which are constructable by a photolithograpy; materials for various kinds of displays; materials for producing color filters (such as the color filters described in the U.S. Patent No. 5,853,446, European Patent No. 863,534, JP-A Nos. 9-244230, 10-62980 and 8-171863, the U.S. Patent No. 5,840,465, European Patent No. 855,731, JP-A No. 5-571576 and JP-A No. 5-67405); materials for producing optical switches, optical grids (interference grids) and optical circuits; materials for producing three-dimentional objects by mass-curing (UV-curing using a clear molding) or stereolithography (such as those described in the U.S. Patent No. 4,575,330); composite materials (such as styrene-based polyester at least including glass fiber and/or other fiber in combination with other aiding agents); materials for producing other compositions that forms a thick layer; resists used for coating or sealing electronic members or intergrated curcuits; and optical lens (such as coating agents for contact lens and Fresnel lens). Further, the composition according to the invention is suitably used for producing medical instruments, aiding devices and dental implants. Moreover, the composition according to the invention is suitably used for producing a gel having thermotropic properties, such as those described in German Patent No. 19,700,064 and European Patent No. 678,534.

By curing the composition by crosslinking the same after shaping, the composition can exhibit extremely high solvent resistance, chemical resistance and mechanical strength. Threfore, the compositon can be used as excellent resin materials. In particular, the composition is particularly suitable for electrical resistance materials or moisture-proof coating materials, such as lubricants described in JP-A No. 2006-225481, JP-A No. 2006-176548, JP-A No. 2006-169398, JP-A No. 2005-194370 and JP-A No. 2005-036158. For example, the composition can be used also as a binder resin for carbon resistance materials, or a moisture-proof coating material for semiconductor materials. The composition can be used as a resistance material for a variable resistor by forming a resistance paste by mixing with carbon, and then sintering the same.

In the invention, the composition is preferably heated to form a crosslinked structure. When energy is applied by heating, the heating can be carried out with an oven or a hot plate having a heater, or by means of photothermal conversion using infrared rays or visible light. The curing is preferably carried out by heating the polymer according to the invention, and the heating temperature is preferably from 50 to 500°C, more preferably from 150 to 450°C, further preferably from 200 to 400°C. The time for curing varies according to the temperature, but is typically from 0.1 seconds to 24 hours, preferably from 10 minutes to 10 hours, more preferably from 30 minutes to 5 hours. When the curing is carried out under the conditions within the above ranges, a cured product having excellent mechanical properties and heat resistance can be obtained.

When energy is applied by exposing the composition to light, the means for light irradiation can be selected from light sources that emit light of from ultraviolet rays to visible rays, such as mercury lamps of from low pressure to super-high pressure, metal halide lamps, and Xe lamps.
The cured product obtained from the above methods may be in the form of a film, pellets, fiber or ohter shaped objects, but is not particularly limited thereto. This application claims priority from Japanese Patent Application No. 2007-242585 filed September 19, 2007, Japanese Patent Application No. 2007-256373 filed September 28, 2007, Japanese Patent Application No. 2008-094262 filed March 31, 2008 and Japanese Patent Application No. 2008-094269 filed March 31, 2008, the disclosures of which is incorporated by reference herein.

### [Examples]

In the following, the invention is explained in further details with reference to the examples. However, the invention is not limited thereto. The H-NMR and MS spectra of the obtained compounds were measured in order to evaluate the properties of the compounds. The measurement condisions of the properties were as follows.

### <Test method>

(1) Nuclear magnetic resonance spectrum analysis (¹H-NMR) was conducted using a measurement device (AV400M, manufactured by Bruker Japan Co., Ltd.) at a resonance frequency of 400 MHz. Deuterated dimethylsulfoxide (DMSO-d₆), which is a deutrated solvent, was used as the solvent for measurement.
(2) Mass spectrometry measurement (MS) was conducted by an ESI method using a measurement device (API QSTAR PULSAR I, manufactured by Biosystems).

### (Example 1a)

Intermediate compound 1a was synthesized in accordance with the following reaction scheme.

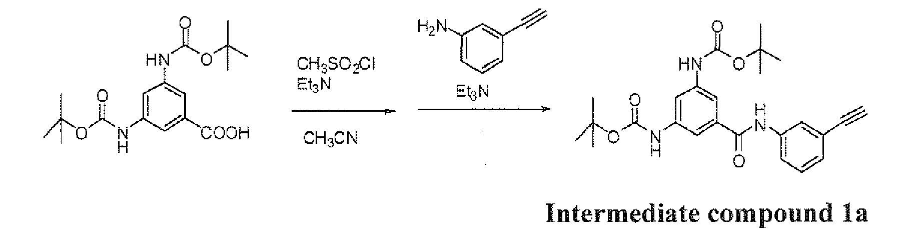

Under a nigrogen flow, in a 300-ml three-neck flask in this order, 10.0 g (28.4 mmol) of 3,5-(d-t-butylcarboxy)diaimino benzoic acid, 100 ml of acetonitrile and 2.87 g (28.4 mmol) of triethylamine were placed and stirred. 3.25 g (28.4 mmol) of methanesulfonyl chloride was dropped therein while cooling with ice, and this was stirred for 30 minutes while cooling with ice. To this reaction solution, a solution prepared by mixing 3.32 g (28.4 mmol) of 3-ethynylaniline and 2.87 g (28.4 mmol) of triethylamine in 5 ml of acetonitrile was dropped. After confirming the dissapearance of the raw materials by HPLC, 250 ml of water and 250 ml of ethyl acetate were added thereto to separate the solution. A 1N hydrochloric acid aqueous solution was added to the obtained ethyl acetate layer to separate the solution, and the resultant was neutralized with a sodium bicarbonate aqueous solution and washed with distilled water. The obtained ethyl acetate layer was dried with anhydrous magnesium sulfate, and was condensed using a rotary evaporator. 9.6 g of a brown solid was obtained.

To this 9.6 g of the brown solid, 80 ml of methanol was added and heated to reflux while stirring. After confirming that the brown solid was completely dissolved, the solution was cooled with ice for 2 hours. 5.3 g (11.7 mmol) of intermediate compound 1a were obtained by collecting a crystal formed in the solution by filtering (yield: 41%). The properties of the obtained compound were as follows.

¹H-NMR (400 MHz, DMSO-d₆): δ10.40 (s, 1H), 9.86 (s, 2H), 8.27 (s, 1H), 7.87 (s, 2H), 7.81 (s, 1H), 7.62 (d, 1H), 7.29 (s, 1H), 7.14 (d, 1H), 4.10 (s, I H), 1.49 (s, 18H)
MS: M⁺ = 451.21

### (Example 2a)

Exemplary compound (1)-43a was synthesized in accordance with the following reaction scheme.

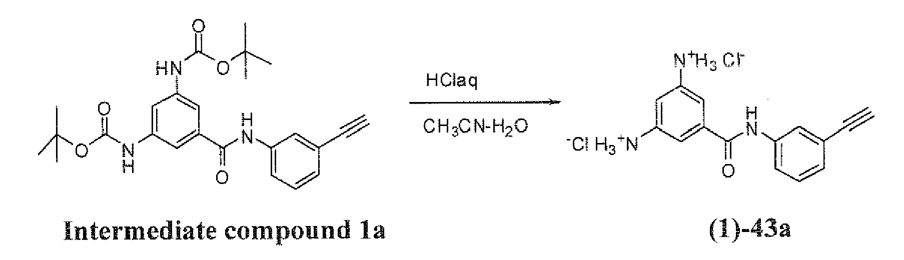

To a 200-ml three-neck flask, 3.0 g (6.64 mmol) of intermediate compound 1a, 60 ml of acetonitrile and 10 ml of distilled water were added and the mixture was stirred at room temperature. To this solution, 17 ml of concentrated hydrochloric acid were added and stirred for 4 hours. After adding 60 ml of acetonitrile thereto, 1.29 g of the intended compound (1)-43a were obtained by collecing a solid precipitated in the solution by filtering (yield: 61%). The properties of the obtained compound were as follows.

¹H-NMR (400 MHz, DMSO-d₆): δ10.04 (s, 1H), 7.92 (s, 1H), 7.76 (d, 1H), 7.36 (t, 1H), 7.22 (d, 1H), 6.96 (s, 1H), 6.68 (s, 1H), 4.19 (s, 1H)
MS: M⁺ = 323.06

### (Example 3a)

Exemplary compound (1)-1a was synthesized in accordance with the following reaction scheme.

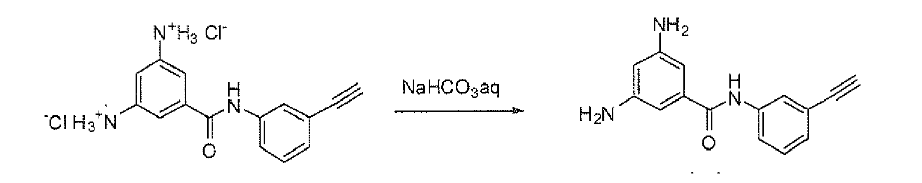

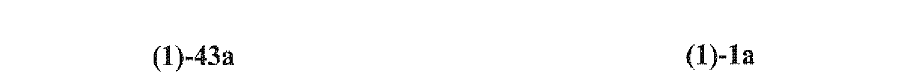

1.0 g (3.08 mmol) of compound (1)-43a were added to 45 ml of distilled water and stirred. To this solution, sodium hydrogen carbonate was added until the pH was 7. 0.77 g of the intended compound (1)-1a were obtained by collecting a solid precipitated in the solution by filtering (yield: 99.4%). The properties of the obtained compound were as follows.

¹H-NMR (400 MHz, DMSO-d₆): δ10.03 (s, 1H), 7.92 (s, 1H), 7.77 (d, 1H), 7.32 (t, 1H), 7.14 (d, 1H), 6.28 (s, 1H), 6.00 (s, 1H), 4.95 (s, 1H), 4.16 (s, 1H)
MS: M⁺ = 251.11

### (Example 4a)

Exemplary intermediate compound 2a was synthesized in accordance with the following reaction scheme.

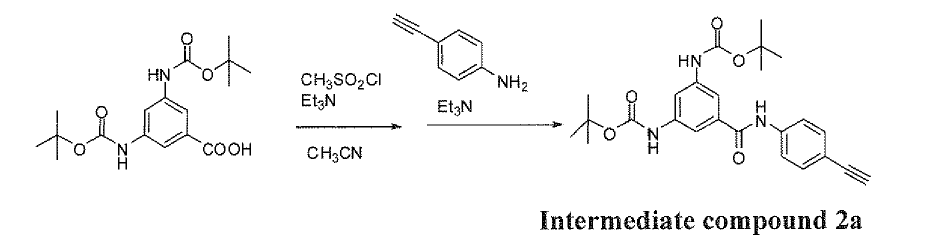

8.3 g of intermediate compound 2a shown by the following formula were obtained in a similar manner to the synthesis of intermediate compound 1a (yield: 65%), except that 4-ethynylaniline (28.4 mmol) was used instead of 3-ethynylaniline (28.4 mmol). The properties of the obtained compound were as follows.

¹H-NMR (400 MHz, DMSO-d₆): δ10.40 (s, 1H), 9.51 (s, 2H), 7.81 (s, 1H), 7.75 (d, 2H), 7.52 (s, 2H), 7.44 (d, 2H), 4.10 (s, 1H), 1.48 (s, 18H)
MS: M⁺ = 451.21

### (Example 5a)

Exemplary compound (1)-2a was synthesized in accordance with the following reaction scheme.

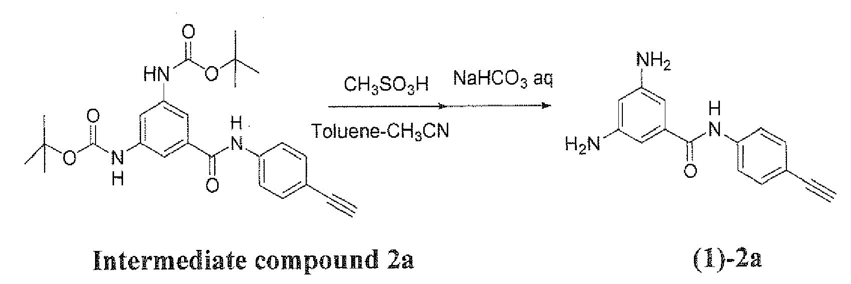

To a 500-ml three-neck flash, 3.0 g (6.64 mmol) of intermediate compound 2a, 100 ml of toluene and 150 ml of acetonitrile were added and stirred at room temperature. To this solution, 1.2 g of methanesulfonic acid were added and stirred for 4 hours. This reaction solution was added to a sodium bicarbonate aqueous solution and ethyl acetate was added thereto for separation, and this was washed with distilled water. The obtained ethyl acetate layer was dried with anhydrous magnesium sulfate and condensed using a rotary evaporator. 0.74 g of the intended compound (1)-2a were obtained by collecting a solid precipitated in the ethyl acetate layer (yield: 44%). The properties of the obtained compound were as follows.

¹H-NMR (400 MHz, DMSO-d₆): δ10.12 (s, 1H), 7.77 (d, 2H), 7.42 (d, 2H), 6.27 (s, 1H), 5.99 (s, 1H), 4.96 (s, 4H), 4.08 (s, 1H)
MS: M⁺ = 251.11

### (Example 6a)

Exemplary intermediate compound 3a was synthesized in accordance with the following reaction scheme.

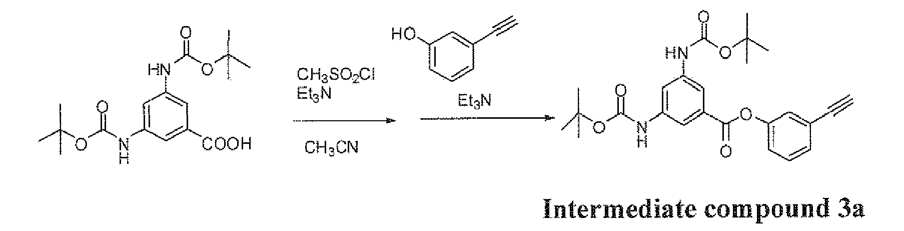

8.35 g of intermediate compound 3a was obtained in a similar manner to the synthesis of intermedaite compound 1 a (yield: 65%), eccept that 3-ethynylphenol (28.4 mmol) was used instead of 3-ethynylaniline (28.4 mmol). The properties of the obtained compound were as follows.

¹H-NMR (400 MHz, DMSO-d₆): δ9.86 (s, 2H), 8.27 (s, 1H), 8.12 (s, 2H), 7.32 (d, 1H), 7.3 (t, 1H), 7.29 (s, 1H), 7.13(d, 1H), 3.06 (s, 1H), 1.49 (s, 18H)
MS: M⁺ = 452.19

### (Example 7a)

Exemplary compound (1)-3a was synthesized in accordance with the following reaction scheme.

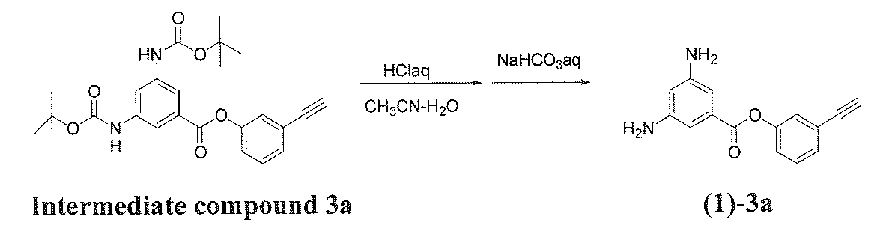

To a 500-mol three-neck flask, 2.5 g (5.52 mmol) of intermediate compound 3a, 50 ml of acetonitrile and 10 ml of distilled water were added and stirred. To this solution, 14 ml of condensed hydrochloric acid were added and stirred for 4 hours. A sodium bicarbonate aqueous solution was added to the reaction solution, and ethyl acetate was added thereto for separation and washed with distilled water. The obtained ethyl acetate layer was dried with anhydrous magnesium sulfate and condensed using a rotary evaporator. 1.15 g of the intended compound (1)-3a were obtained by collecing a solid precipitated in the ethyl acetate layer by filtering (yield: 82%). The properties of the compound were as follows.

¹H-NMR (400 MHz, DMSO-d₆): δ7.32 (d, 1H), 7.31 (s, 1H), 7.30 (t, 1H), 7.13 (d, 1H), 6.70 (s, 2H), 5.91 (s, 1H), 5.85 (s, 4H), 3.06 (s, 1H)
MS:M⁺ = 252.09

### (Example 8a)

Exemplary intermediate compound 4a was synthesized in accordance with the following reaction scheme.

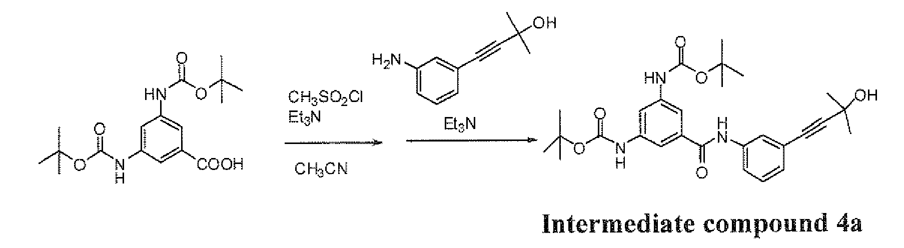

6.51 g of intermediate compound 4a were obtained in a similar manner to the synthesis of intermediate compound 1a (yield: 45%), except that 4-(3-aminophenyl)-2-methyl-3-butyn-2-ol (28.4 mmol) was used instead of 3-ethynylaniline (28.4 mmol). The properties of the obtained compound were as follows.

¹H-NMR (400 MHz, DMSO-d₆): δ10.40 (s, 1H), 9.86 (s, 2H), 8.27 (s, 1H), 7.87 (s, 2H), 7.81 (s, 1H), 7.62 (d, 1H), 7.29 (t, 1H), 7.16 (d, 1H), 5.45 (s, 1H), 1.96 (s, 18H), 1.47 (s, 6H)
MS: M⁺ = 509.59

### (Example 9a)

Exemplary compound (1)-16a was synthesized in accordance with the following reaction scheme.

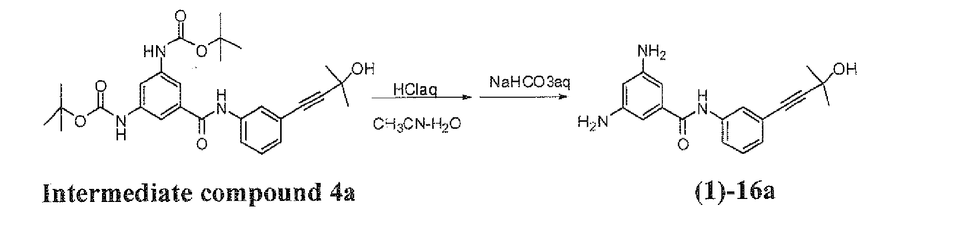

1.22 g of compound (1)-16a were obtained in a similar manner to the synthesis of compound (1)-3a (yield: 71%), except that intermediate compound 4a (5.52 mmol) was used instead of intermediate compound 3a (5.52 mmol). The properties of the obtained compound were as follows.

¹H-NMR (400 MHz, DMSO-d₆): δ10.40 (s, 1H), 7.81 (s, 1H), 7.62 (d, 1H), 7.29 (t, 1H), 7.16 (d, 1H), 6.51 (s, 2H), 5.91 (s, 1H). 5.85 (s, 4H), 5.45 (s, 1H), 1.47 (s, 6H)
MS: M⁺ = 309.15

### (Example 10a)

### Synthesis of compound (1)-1a by a consecutive method

In a 1000-ml three-neck flask, 10 g (65.7 mmol) of 3,5-diaminobenzoic acid, 150 ml of t-butanol and 150 ml of a 1N NaOH aqueous solution were added and stirred, ant it was confirmed that the compound was dissolved. 29.4 g (134.7 mmol) of di-t-butyl dicarbonate were dropped thereto and stirred while cooling with ice. After confirming the disappearance of the raw materials by HPLC, 300 ml of toluene were added therto. After neutralizing this solution by adding hydrochloride acid thereto, the solution was separated. After adding 200 ml of water and separating the solution, 6.7 g (65.7 ml) of triethylamine were added to the oil layer and stirred under a nitrogen atmosphere. To this reaction solution, 7.5 g (65.7 mmol) of methanesulfonyl chloride were added and stirred for 30 minutes while cooling with ice. To this reaction solution, a solution prepared by mixing 7.7 g (65.7 mmol) of 3-ethynylaniline and 6.7 g (65.7 mmol) of triethylamine in 10 ml of toluene was dropped. After confirming the disappearance of the raw materials by HPLC, 200 ml of water was added and the solution was separated. To the obtained toluene layer, 22.1. g (230 mmol) of methanesulfonic acid were added and stirred at room temperature. After confirming the disappearance of the raw materials, the solution was neutralized with a sodium bicarbonate aqueous solution and washed with distilled water. The obtained organic layer was condensed using an evaporator, thereby obtaining 7.74 g of compound (1)-1a (yield: 47%). The properties of the obtained compound were the same as those of the compound obtained in Example 3a.

### (Example 11a)

Compound (1)-11a was obtained in a similar manner to the synthesis in Example 10a, except that 3,5-diethynylaniline was used instead of 3-ethynylaniline.

### (Example 12a)

Compound (1)-11a was obtained in a similar manner to the synthesis in Example 10a, except that 3,5-diethynylaniline was used instead of 3-ethynylaniline.

### Synthesis of compound (1)-30a

To a 500-ml three-neck flask, 10 g (0.050 mol) of 2.4-diaminophenol dihydrochloride, 200 ml of water and 21.0 g (0.15 mol) of pottasium carbonate were added in this order, and this mixture was stirred to dissolve the components. While cooling with ice, 22.3 g (0.10 mol) of di-t-butyl bicarbonate were dropped to the solution over 1.5 hours. After the completion of dropping, the temperature was raised to room temperature and the solution was allowed to react. After confirming the disappearance of the raw materials by HPLC, HCl was added to neutralize the solution, and intermediate compound 1 a was allowed to precipitate with an acid and collected by filtering. The obtained intermediate compound 1a was dissolved in NMP and 19.8 g (0.15 mol) of pyridine were added thereto. After dropping phenyl chlorocarbonate to the solution while cooling with ice, the temperature was raised to room temperatuer and the solution was allowed to react. After cofirming the dissapearance of the raw materials by HPLC, 11.8 g (0.10 mol) of 3-ethynylphenol was added thereto and heated to 50°C. After confirming the disappearance of 3-ethynylphenol by HPLC, 96.1 g (1.0 mol) of methansulfonic acid were added while cooling with ice, and the solution was allowed to react. The obtained reaction solution was neutralized with sodium bicarbonate water, and 60 g of compound (1)-30a were allowed to crystallize with toluene (yield: 45%). The properties of the obtained compound were as follows.

¹H-NMR (400 MHz, DMSO-d₆): δ7.24 (s, 1H), 7.23 (d, 2H), 7.20 (t, 1H), 7.05 (d, 1H), 6.57 (d, 2H), 5.79 (d, 1H), 5.63 (s, 1H), 5.85 (s, 4H), 3.06 (s, 1H)
MS: M⁺ = 268.08

### (Example 14a)

Compound (1)-35a was obtained in a similar manner to the synthesis in Example 10a, except that 3-ethynyl-4-fluoroamline was used instead of 3-ethynylaniline.

### (Example 15a)

Compound (1)-59a was obtained in a similar manner to the synthesis in Example 10a, except that 3,5-diaminocyclohexanecarboxylic acid was used instead of 3,5-diaminobenzoic acid.

### (Example 16a)

Compound (2)-1a was synthesized in accordance with the following method.

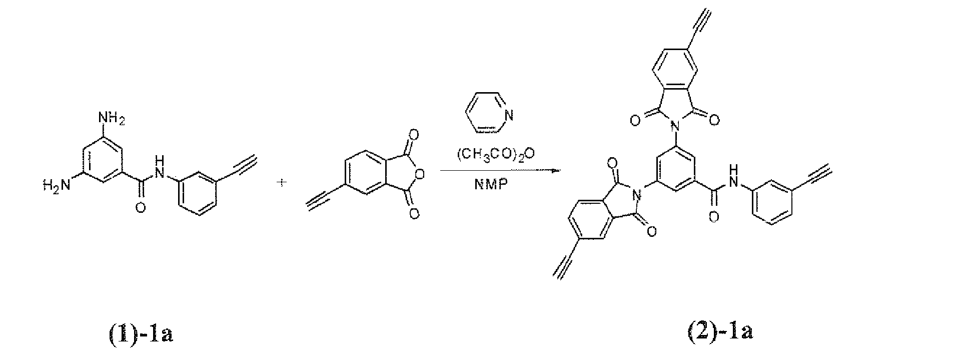

To a 100-ml four-neck flask, 3.0 g (11.9 mmol) of compound (1)-1a and 20 g of NMP were placed, and the compound was allowed to dissolve under a nitrogen flow. 4.11 g (23.9 mmol) of 4-ethynylphthalic anhydride were added to this solution in multiple steps, and stirred at room temperature for 4 hours, thereby synthesizing an amic acid solution. Subsequently, 0.19 g (2.39 mmol) of pyridine and 7.32 g (71.6 mmol) of acetic anhydride were added to the solution. After stirring at room temperature for several hours, a crystal precipiated in the solution was collected by filtering, and dried. 5.01 g of compound (1)-30a were thus obtained (yield: 75%). The properties of the obtained compound were as follows.

¹H-NMR (400 MHz, DMSO-d₆): δ10.40 (s, 1H), 8.29 (s, 2H), 8.27 (s, 1H), 8.10 (d, 2H), 8.07 (s, 2H), 7.85 (d, 2H), 7.81 (s, 1H), 7.62 (d, 1H), 7.29 (t, 1H), 7.1.4 (d, 1H), 4.08 (s, 1H), 3.06 (s, 2H)
MS: M⁺ = 559.12

### (Example 17a)

### Synthesis of compound (3)-5a

A film of a crosslinked product of imide oligomer was obtained by a method described in the Non-patent document 2 ("Polymer" (1994), Vol. 35. pp. 4857-4864). Specifically, a solution of an amic acid oligomer having an average molecular weight of about 9,000 was prepared from a solution of N-methylpyrolidone (NMP) containing compound (1)-1a, 4-phenylethynylphthalic anhydride, 4,4'-oxydianiline and 4,4'-oxydiphthalic anhydride. Then, the obtained amic acid oligomer was subjcted to centrifugal separation, coating, drying and heat treatments for 1 hour at 100°C, 1 hour at 250°C and 1 hour at 350°C, respectively, thereby forming the film of a crosslinked product of imide oligomer. In a separate process, toluene was added to the NMP solution of amic acid oligomer, and the imide oligomer was isolated through azeotropic dehydration, cooling, filtering, washing with water and methanol in this order, and drying.

The mechanical properites at 23°C of the film as prepared by the above method were measured in accordance with a method of ASTM D882. The results are shown in Table 1.

### (Example 18a)

An imide oligomer was obtained in a similar manner to the synthesis in Example 17a, except that compound (1)-35a was used instead of compound (1)-1a. The measurement results of the physical properties of the obtained polymer film are shown in Table 1.

### (Example 19a)

An imide oligomer was obtained in a similar manner to the synthesis in Example 17a, except that compound (1)-59a was used instead of compound (1)-1a. The measurement results of the physical properties of the obtained polymer film are shown in Table 1.

### (Example 20a)

To a 200-ml three-neck flask purged with an inert gas, 0.018 mol of bis(4-aminophenyl) ether, 0.005 mol of compound (1)-1a and 0.004 mol of aniline were placed, and 110 ml of NMP was further added to dissolve the compounds. While stirring this reaction solution at room temperature, 0.025 mol of 4,4'-(2,2-hexafluoroisopropylidene)diphthalic dianhydride in the form of a solid were added thereto, and stirred at room temperatuer for 2 hours. Subsequently, 0.05 mol of acetic anhydride and 0.005 mol of pyridine were added thereto and stirred at room temperature for 1 hour, and this was heated 60°C and stirred for three hours. A polyimide solution was thus obtained.

The obtained solution was dropped in 300 ml of acetonirtile, and a deposition formed therein was colleted by filtering and dried. A powder of a polyimide having (1)-1a at both terminals thereof was thus obtained. 10 g of this powder was dissolved in 50 ml of N-methyl-2-pyrrolidone to obtain a solution of a polyimide having (1)-1a at both terminals thereof. This solution was applied on a silica glass plate using a blade and dried, and was then heated to cure at 250°C. Thereafter, the mechanical properites at 23°C of this polyamide film forked on the silica glass plate were measured in accordance with a method of ASTM D882. The results are shown in Table 1.

### (Example 21 a)

An imide oligomer was obtained in a similar manner to the experiment conducted in Example 20a, except that (1)-35a was used instead of (1)-1a.

The mechanical properites at 23°C of the film as prepared by the above method were measured in accordance with a method of ASTM D882. The results are shown in Table 1.

### (Example 22a)

To a 200-ml three-neck flask purged with an inert gas, compound (1)-1a (0.018 mol) and triaethylamine (0.090 mmol) were placed and 110 ml of NMP were further added to dissolve the compounds. This reaction solution was cooled with ice and 4-nitrophenol (0.020 mol) were dropped therein, and the solution was stirred at the same temperature for 30 minutes. To this solution, 3,4'-diaminodiphenyl ether (0.018 mol) dissolved in 20 ml of NMP was gradually added. After the completion of dropping, the temperature was raised to room temperature and the solution was stirred for 2 hours. This solution was applied on a silica glass plate with a blade and dried, and was then heated to cure at 250°C. Thereafter, the mechanical properites at 23°C of this polyimide film formed on the silica glass plate were measured in accordance with a method of ASTM D882. The results are shown in Table 1.

### (Example 23a)

To a 200-ml three-neck flask purged with an inert gas, compound (1)-1a (0.018 mol) and triaethylamine (0.090 mol) were placed and 110 ml of NMP were further added to dissolve the compounds. This reaction solution was cooled with ice and 4-nitrophenol (0.020 mol) were dropped therein, and the solution was stirred at the same temperature for 30 minutes. To this solution, 4,4'-dihydroxydiphenylmethane (0.018 mol) was gradually added. After the completion of dropping, the temperature was raised to room temperature and the solution was stirred for 2 hours. This solution was applied on a silica glass plate with a blade and dried, and was then heated to cure at 250°C. Thereafter, the mechanical properites at 23°C of this polyimer film formed on the silica glass plate were measured in accordance with a method of ASTM D882. The results are shown in Table 1.

### (Example 24a)

To a 200-ml three-neck flask purged with an inert gas, compound (1)-1a (0.018 mol), aniline (0.004 mol) and triaethylamine (0.090 mol) were placed and 110 ml of NMP were further added to dissolve the compounds. While stirring this reaction solution at room temperature, 0.018 mol of 3,3'-naphthalenedicarboxylic dichloride in the form of a solid were gradually added and the mixture was stirred for 2 hours at room temperature. This solution was applied on a silica glass plate with a blade and dried, and was then heated to cure at 250°C. Thereafter, the mechanical properites at 23°C of this polyamide film formed on the silica glass plate were measured in accordance with a method of ASTM D882. The results are shown in Table 1.

### (Comparative Example 1a)

A film of a crosslinked product of imide oligomer was obtained in accordance with the method described on pages 4857 to 4864 of Polymer, 1994, Vol. 35. Speficically, a solution of an amic acid oligomer having an average molecular weight of about 9,000 was prepared from a N-methylpyrrolidone solution containing 4-phenylethynylphthalic anhydride, 3,4-'-oxydianiline and 4,4'-oxydiphthalic anhdride. The obtained amic acid oligomer was subjected to centrifugal separation, coating, drying and heat treatments for 1 hour at 100°C, 1 hour at 250°C and 1 hour at 350°C. respectively, thereby forming the film of a crosslinked product of imide oligomer. In a separate process, toluene was added to the NMP solution of amic acid oligomer, and an imide oligomer was isolated through azeotropic dehydration, cooling, filtering, washing with water and methanol in this order, and drying. The obtained powder was dissolved in NMP to form a solution, and this solution was applied on a silica glass plate with a blade, dried, and heated to cure at 250°C, in a similar manner to Example 17a. Thereafter, the mechanical properites at 23°C of this polyimide film formed on the silica glass plate were measured in accordance with a method of ASTM D882. The results are shown in Table 1.

### (Comparative Example 2a)

An experiment was performed in a similar manner to that in Example 22a, except that 3,5-dimethylaniline was used instead of (1)-1a in Example 22a. The measurement results of the physical properties of the obtained polymer film are shown in Table 1.

### (Comparative Example 3a)

An experiment was performed in a similar manner to that in Example 23a, except that 3,5-dimethylaniline was used instead of (1)-2a in Example 23a. The measurement results of the physical properties of the obtained polymer film are shown in Table 1.

### (Comparative Example 4a)

An experiment was performed in a similar manner to that in Example 24a, except that 3,4'-diaminodiphenyl ether was used instead of (1)-1a in Example 24a, and 4-phenylethynylphthalic anhydride (0.004 mol) was added instead of aniline in Example 24a. The measurement results of the physical properties of the obtained polymer film are shown in Table 1.

### (Comparative Example 5a)

An experiment was performed in a similar manner to that in Example 16a, except that aniline was used instead of (1)-1a in Example 16a. The measurement results of the physical properties of the obtained polymer film are shown in Table 1.

The mechanical properites at 23°C of the polymer film prepared by the method described above were measured in accordance with a method of ASTM D882. The results are shown in Table 1.

**Table 1**

| | | |
|---|---|---|
| Polymer | Tensile Strength | Modulus of Elasticity |
| Example 17a | 1.39.1MPa | 3.8 GPa |
| Example 18a | 150.3MPa | 4.1 GPa |
| Example 19a | 137.5MPa | 3.6 GPa |
| Example 20a | 156.4MPa | 3.3 GPa |
| Example 21a | 157.8MPa | 3.4 GPa |
| Example 22a | 83.2MPa | 1.9 GPa |
| Example 23a | 1447.2MPa | 1.4 GPa |
| Example 24a | 105.1MPa | 2.7 GPa |
| Comparative Example 1a | 122.2MPa | 3.0 GPa |
| Comparative Example 2a | 75.9MPa | 1.3 GPa |

### (Example 1)

Intermedaite compound 1 was synthesized in acordance with the following reaction scheme.

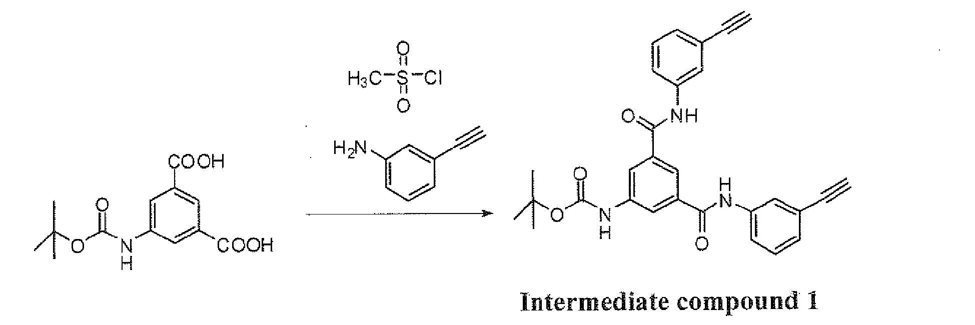

Under a nitrogen flow, in a 300-ml three-neck flask, 8.0 g (28.4 moml) of 5-(t-butylcarboxy)aminoisophthalic acid, 100 ml of acetonirile and 5.74 g (56.8 mmol) of triethylamine were added in this order, and the mixture was stirred. While cooling with ice, 6.5 g (56.8 mmol) of methanesulfonyl chloride were added to the mixture and stirred for 30 minutes while cooling with ice. To this reaction solution, a solution prepared by mixing 6.64 g (56.8 mmol) of 3-ethynylaniline and 5.74 g (56.8 mmol) of triethylamine in 5 ml of acetonitrile was dropped. After confirming the disppearance of the raw materials by HPLC, 250 ml of water and 250 ml of ethyl acetate were added thereto and the solution was separated. After adding a 1N hydrochloride aqueous solution to the obtained ethyl acetate layer to separate the solution, this was neutralized with a sodiuom bicarbonate aqueous solution and washed with distilled water. The obtained ethyl acetate layer was dried with anhydrous magnesium sulfate and condnesed using a rotary evaporator. 9.7 g of a brown solid were obtained.
To the 9.7 g of the brown solid, 80 ml of methanol were added and heated to flux while stirring. After confirming that the solid was completely dissolved, the solution was cooled with ice for 2 hours. Thereafter, 15.6 g (11.7 mmol) of the intended intermediate compound was obtained by colltecting a crystal formed in the solution by filtering (yield: 41%). The properties of the obtained compound were as follows.

¹H-NMR (400 MHz, DMSO-d₆): δ10.34 (s, 2H), 9.80 (s, 1H), 7.96 (t, 2H), 7.79 (d, 2H), 7.61 (s, 1H), 7.37 (t, 2H), 7.26 (d, 2H), 7.20 (d, 2H), 4.19 (s, 2H), 1.50 (s, 9H)
MS: M⁺ = 479.53

### (Example 2)

Exemplary compound (1)-64 was synthesized in accordance with the following reaction scheme.

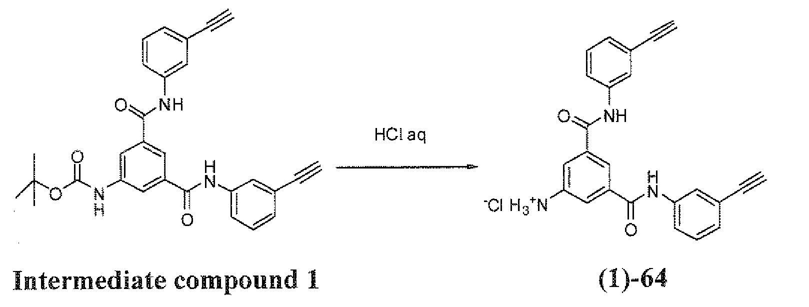

To a 200-ml three-neck flask, 3.18 g (6.64 mmol) of intermediate compound 1, 60 ml of acetonitrile and 10 ml of distilled water were added and the solution was stirred at room temperature. To this solution, 17 ml of condensed hydrochloric acid were added and stirred for 4 hours. 60 ml of acetonitrile were added thereto and 1.68 g of the intended compound (1)-64 were obtained by collecteing a solid precipitated in the solution by filtering (yield: 61%). The properties of the obtained compound were as follows.
MS:M+ = 415.88

Exemplary compound (1)-1 was synthesized in accordance with the following reaction scheme.

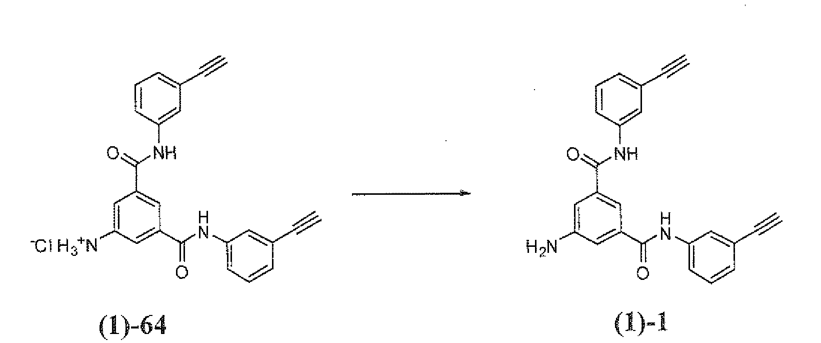

1.28 g (3.08 mmol) of compound (1)-64 and 45 ml of distilled water were added and stirred. To this solution, sodium hydrogen carbonate was added until the pH was 7. 1.16 g of the intended compound (1)-1 were obtained by separating a solid precipitated in the solution by filtering. The properties of the obtained compound were as follows.

¹H-NMR (400 MHz, DMSO-d₆): δ10.34 (s, 2H), 7.96 (t, 2H), 7.79 (d, 2H), 7.61 (s, 1H), 7.37 (t, 2H), 7.26 (d, 2H), 7.20 (d, 2H), 5.65 (s, 2H), 4.19 (s, 2H)
MS: M⁺ = 379.41

### (Example 4)

Exemplary intermediate compound 2 was synthesized in accordance with the following reaction scheme.

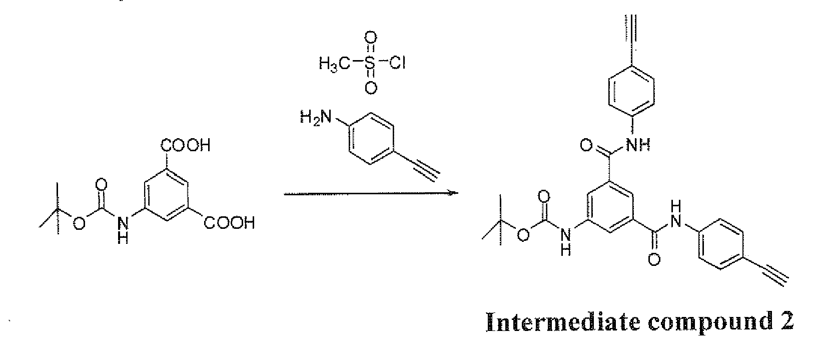

8.87 g of intermediate compound 2 was obtained in a similar manner to the synthesis of intermediate compound 1, except that 4-ethynylaniline (56.8 mmol) was used instead of 3-ethynylaniline (56.8 mmol). The properties of the obtained compound were as follows.

¹H-NMR (400 MHz, DMSO-d₆): δ10.34 (s, 2H), 9.80 (s, 1H), 8.16 (s, 2H), 8.09 (s, 1H), 7.81 (d, 4H), 7.48 (d, 4H), 4.12 (s, 2H), 1.50 (s, 9H)
MS: M⁺ = 479.53

### (Example 5)

Exemplary compound (1)-2 was synthesized in accordance with the following reaction scheme.

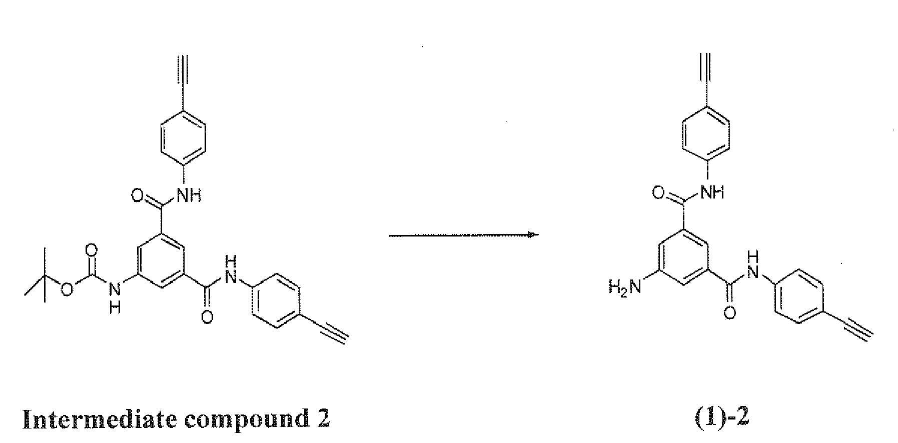

To a 500-ml, three-neck flask, 3.18 g (6.64 mmol) of intermediate compound 2, 100 ml of toluene and 150 ml of acetonitrile were added and stirred at room temperature. To this solution, 1.2 g of methanesulfonic acid were added and stirred for 4 hours. This reaction solution was added to a sodium bicarbonate aqueous solution, and ethyl acetate was added to separate the solution and washed with distilled water. The obtained ethylacetate layer was dried with anhydrous magnesium sulfate and condensed using a rotary evaporator. 1.11 g of the intended compound (1)-2 were obtained by collecting a solid precipitated in the ethyl acetate layer by filtering (yield: 44%). The properties of the obtained compound were as follows.

¹H-NMR (400 MHz, DMSO-d₆): δ10.34 (s, 2H), 7.81 (d, 4H), 7.60 (s, 1H), 7.46 (d, 4H), 7.26 (s, 2H), 5.65 (s, 2H), 4.12 (s, 2H)
MS: M⁺ = 379.41

### (Example 6)

Exemplary intermediate compound 3 was synthesized in accordance with the following reaction scheme.

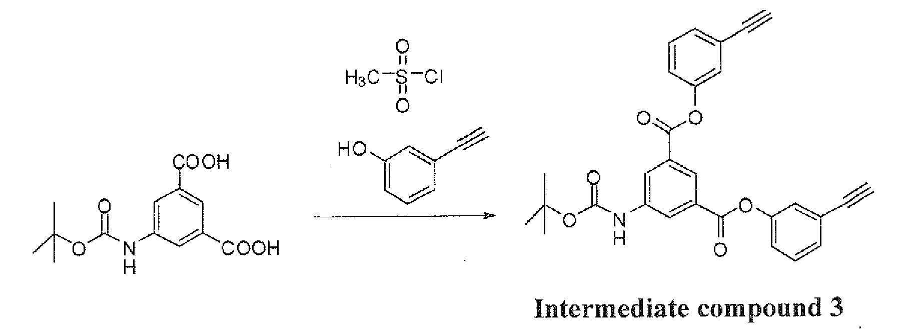

8.89 g of intermedaite compound 3 were obtained in a similar manner to the synthesis of intermediate compound 1 (yield: 65%), except that 3-ethynylphenol (56.8 mmol) was used instead of 3-ethynylaniline (56.8 mmol). The properties of the obtained compound were as follows.

¹H-NMR (400 MHz, DMSO-d₆): δ9.77 (s, 1H), 7.98 (s, 1H), 7.59 (s, 2H), 7.32 (d, 2H), 7.31 (t, 2H), 7.30 (t, 2H), 7.13 (d, 2H),3.08 (s, 2H), 1.50 (s, 9H)
MS: M⁺ = 481.50

### (Example 7)

Exemplary compound (1)-3 was synthesized in accordance with the following reaction scheme.

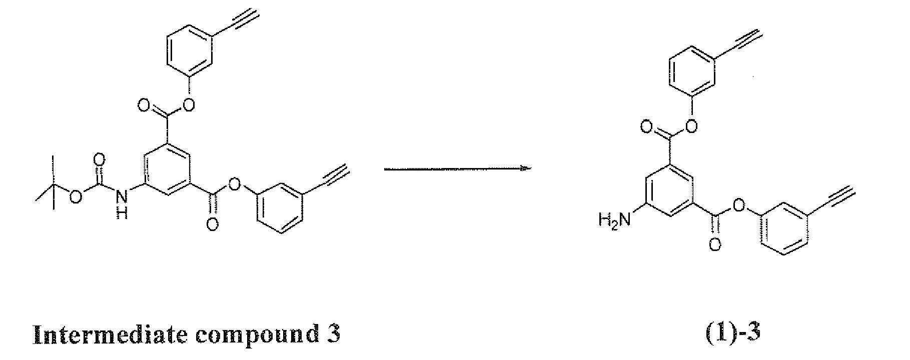

To a 200-mol three-neck flask, 2.65 g (5.52 mmol) of intermediate compound 3, 50 ml of acetonitrile and 10 ml of distilled water were added and stirred. To this solution, 14 ml of concentrated hydrochloric acid were added and stirred for 4 hours. To this reaction solution, a sodium bicarbonate aqueous solution was added, and ethyl acetate was added to separate the solution and washed with distilled water. The obtained ethyl acetate layer was dried with anhydrous magnesium sulfate and condensed using a rotary evaporator. 0.84 g of the intended compound (1)-3 were obtained by collecting a solid precipitaed in the ethyl acetate layer by filtering (yield: 40%). The propertis of the obtained compound were as follows.

¹H-NMR (400 MHz, DMSO-d₆): δ7.98 (s, 1H), 7.59 (s, 2H), 7.32 (d, 2H), 7.31 (t, 2H), 7.30 (t, 2H), 7.13 (d, 2H), 5.67 (s, 2H), 3.08 (s, 2H)
MS:M⁺ = 381.38

### (Example 8)

Intermediate compound 4 was synthesized in accordance with the following reaction scheme.

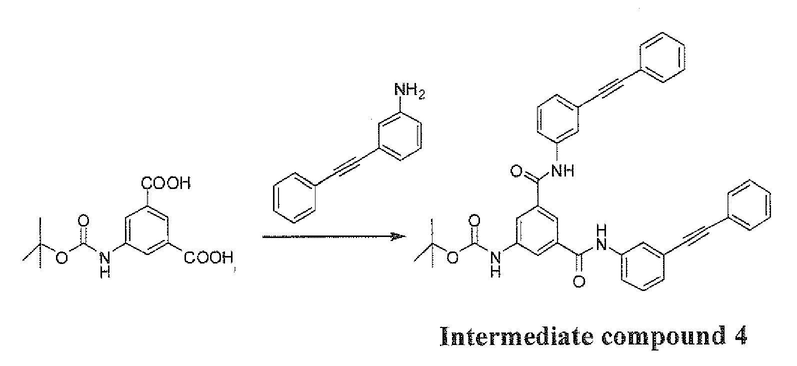

9.50 g of intermedaite compound 4 were obtained in a similar manner to the synthesis of intermediate compound 1 (yield: 53%), except that 3-phenylethynyl aniline (56.8 mmol) was used instead of 3-ethynylaniline (56.58 mmol). The properties of the obtained compound were as follows.

¹H-NMR (400 MHz, DMSO-d₆): δ10.40 (s, 2H), 9.86 (s, 1H), 8.38 (s, 2H), 8.24 (s, 1H), 7.85 (s, 2H), 7.59 (d, 4H), 7.57 (d, 2H), 7.41 (t, 4H), 7.39 (t, 2H), 7.28 (t, 2H), 7.20 (d, 2H), 1.49 (s, 9H)
MS: M⁺ = 631.25

### (Eample 9)

Exemplary compound (1)-20 was synthesized in accordance with the following reaction scheme.

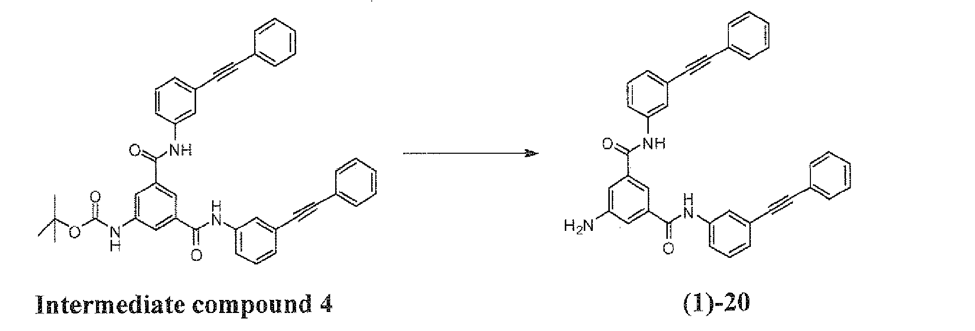

2.49 g of compound (1)-20 were obtained in a similar manner to the synthesis of compound (1)-3 (yield: 85%), exept that intermediate compound 4 (5.52 mmol) was used instead of intermediate compound 3 (5.52 mmol). The properties of the obtained compound were as follows.

¹H-NMR (400 MHz, DMSO-d₆): δ10.40 (s, 2H), 7.86 (s, 1H), 7.85 (s, 2H), 7.59 (d, 4H), 7.57 (d, 2H), 7.41 (t, 4H), 7.40 (s, 2H), 7.39 (t, 2H), 7.28 (t, 2H), 7.20 (d, 2H), 5.85 (s, 2H)
MS: M⁺ = 531.19

### (Example 10)

Intermediate compound 5 was synthesized in accordance with the following reaction scheme.

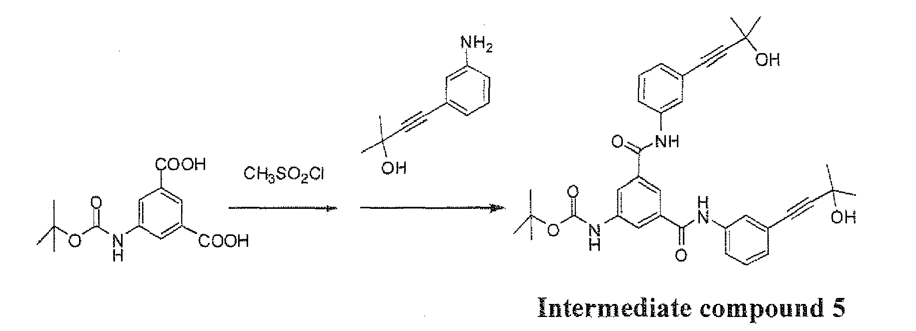

5.92 g of intermediate compound 5 were synthesized in a similar manner to the synthesis of intermediate compound 1 (yield: 35%), except that 4-(3-aminophenyl)-2-methyl-3-butyn-2-ol (56.8 mmol) was used instead of 3-ethynylamiline (56.8 mmol). The properties of the intermediate compound were as follows.

¹H-NMR (400 MHz, DMSO-d₆): δ10.40 (s, 2H), 9.86 (s, 1H), 8.38 (s, 2H), 8.24 (s, 1H), 7.81 (s, 2H), 7.62 (d, 2H), 7.29 (t, 2H), 7.16 (d, 2H), 5.45 (s, 2H), 1.49 (s, 9H), 1.47 (s, 12H)
MS: M⁺ = 595.27

### (Example 11)

Compound (1)-21 was synthesized in accordance with the following reaction scheme.

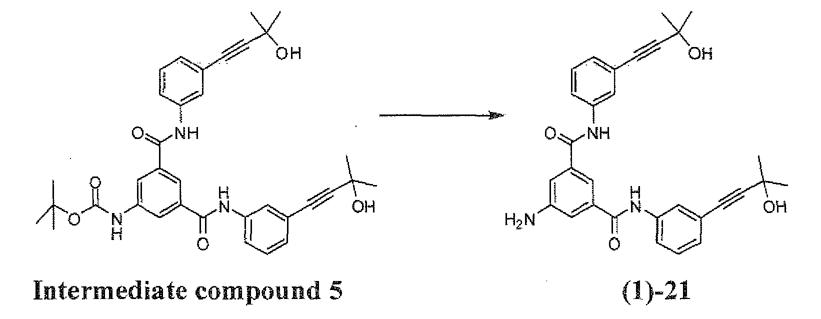

1.64 g of compound (1)-20 were obtained in a similar manner to the synthesis of compound (1)-3, except that intermediate compound 5 (5.52 mol) was used instead of intermediate compound 3 (5.52 mmol). The properties of the obtained compound were as follows.

¹H-NMR (400 MHz, DMSO-d₆): δ10.40 (s, 2H), 7.86 (s, 1H), 7.81 (s, 2H), 7.62 (d, 2H), 7.29 (t, 2H), 7.16 (d, 2H), 5.85 (s, 2H), 5.45 (s, 2H), 1.47 (s, 12H)
MS: M⁺ = 495.22

### (Example 12)

### Synthesis of compound (1)-1 in a consecutive method

Under a nitrogen flow, in a 1000-ml three-neck flask, 18.5 g (65.7 mmol) of 5-(t-buylcarboxy)aminoisophthalic acid, 200 g of N-methyl-2-pyrrolidone and 13.4 g (131.4 mmol) of triethylamine were added and stirred. To this reaction solution, 15 g (131.4 mmol) of methanesulfonyl chloride were added and stirred for 30 minutes while cooling with ice. To this reaction solution, a solution prepared by mixing 15.4 g (131.4 mmol) of 3-ethynylaniline and 13.4 g (131.4 mmol) of triethylamine in 30 ml of N-methyl-2-pyrrolidone was dropped. After confirming the disppearance of the raw materials by HPLC, 300 ml of toluene and 200 ml of water were added to separate the solution. To the obtained toluene layer, 22.1 g (230 mmol) of methanesulfonic acid were added and stirred at room temperature. After confirrming the disppearance of the raw materials, the toluene layer was neutralized with a sodium bicarbonate aqueous solution and washed with distilled water. The obtained organic layer was condensed using an evaporator, thereby obtaining 8.47 g of compound (1)-1 (yield: 34%). The properties of the obtained compound were the same as that of the compound obtained in Example 3.

### (Example 13) (Synthesis of compound 1-6)

Compound 1-6 was synthesized in a similar manner to Examples 1 to 3, except that 26.9 mmol of 2-(t-butylcarboxy)aminoterephthalic acid was used instead of 5-(t-butylcarboxy)aminoisophthalic acid. The properties of the obtained compound were as follows.
¹H-NMR (400 MHz, DMSO-d₆): δ10.25 (s, 2H), 7.88 (s, 1H), 7.81 (s, 2H), 7.62 (d, 2H), 7.54 (d, 1H), 7.33 (s, 1H), 7.29 (t, 2H), 7.14 (d, 2H), 5.85 (s, 2H), 4.08 (s, 2H)
MS: M⁺ = 379.13

### (Example 14) (Synthesis of compound 1-14)

In a 200-ml three-neck flask, 10 mmol of N-t-butoxycarbonyl-3,5-difluorobenzene, 20 mmol of potassium carbonate and 100 ml of NMP were placed and sirred. To this solution, 20 mmol of 4-ethynylphenol were added, and the solution was heated to 150°C and allowed to react for 10 hours.
After cooling the reaction solution to room temperature, 20 ml of distilled water was added thereto, and 30 ml of condensed hydrocyloric water were added thereto and stirred for several hours at room temperature. After confirming the disappearance of the raw materials, a sodium bicarbonate aqueous solution was added to the reaction solution, and ethyl acetate was added to separate the solution and washed with distilled water. The obtained ethyl acetate layer was dried with anhydrous magnesium sulfate and condensed using a rotary evaporator. The intended compound (1)-14 was obtained by collecting a solid precipitaed in the ethyl acetate layer by filtering (yield: 25%). The properties of the obtained compound were as follows.

¹H-NMR (400 MHz, DMSO-d₆): δ7.38 (d, 4H), 6.89 (d, 4H), 6.01 (s, 1H), 5.85 (s, 2H), 5.84 (s, 2H), 3.06 (s, 2H)
MS: M⁺ = 325.11

### (Example 15) (Synthesis of compound 1-57)

Compound 1-57 was synthesized in a similar manner to Example 1 to 3, except that 2-heptyn-1-ol (56.8 mmol) was used instead of 3-ethynylaniline. The properties of the obtained compound were as follows.

¹H-NMR (400 MHz, DMSO-d₆): δ7.64 (s, 1H),7.38 (s, 2H), 5.85 (s, 2H), 4.94 (s, 4H), 1.98 (t, 4H), 1.46 (q, 4H), 1.33 (q, 4H), 0.96 (t, 6H)
MS: M⁺ = 313.13

### (Example 16) (Synthesis of compound 1-94)

Compound 1-94 was synthesized in a similar manner to Examples 1 to 3, except that 2-(t-butylcarboxy)aminoterephthalic acid (26.9 mmol) was used instead of 5-(t-butylcarboxy)aminoisophthalic acid, and 2,2,8,8-tetramethyl-3,6-nonadin-5-ol (56.8 mmol) was used instead of 3-ethynylaniline. The properties of the obtained compound were as follows.

¹H-NMR (400 MHz, DMSO-d₆): δ7.71 (d, 1H), 7.28 (s, 1H), 7.19 (d, 1H), 6.05 (s, 2H), 5.85 (s, 2H), 1.28 (s, 12H)
MS: M⁺ = 529.32

### (Example 17)

Exemplary compound (2)-1 was synthesized in accordance with the following reaction scheme.

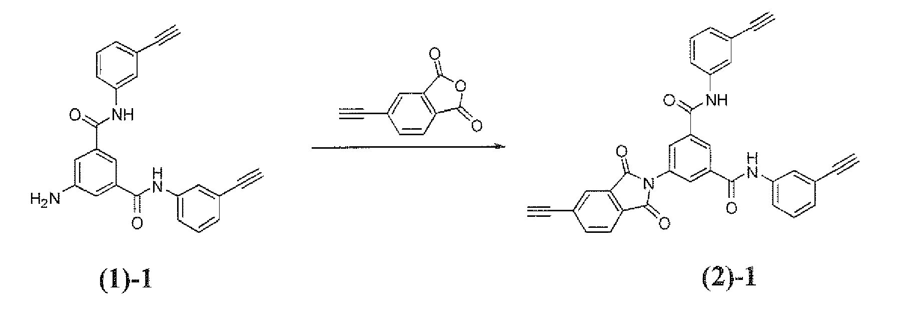

In a 100-ml four-neck flask, 4.5 g (11.9 mmol) of compound (1)-1 and 20 g of NMP were placed and the compound was dissolved under a nitrogen flow. 2.05 g (11.9 mmol) of 4-ethynylphthalic anhydride were added in multiple steps, and the solution was allowed to react for 4 hours at room temperature, thereby synthesizing an amic acid solution. Subsequently, 0.95 g (1.19 mmol) of pyridine and 3.66 g (35.8 mmol) of acetic anhydride were dropped to the reaction solution. The reaction solution was stirred for 5 hours at room temperature, and a crystal precipitated therein was collected by filtering and dried. 4.44 g of compound (2)-1 was obtained (yield: 70%). The properties of the obtained compound were as follows.

¹H-NMR (400 MHz, DMSO-d₆): δ10.25 (s, 1H), 8.29 (s, 2H), 8.27 (s, 1H), 8.10 (d, 2H), 8.07 (s, 2H), 7.85 (d, 2H), 7.81 (s, 1H), 7.62 (d, 1H), 7.29 (t, 1H), 7.14 (d, 1H), 4.08 (s, 1H), 3.08 (s, 2H)
MS: M⁺ = 533.53

### (Example 18) Preparation of polyimide composition containing compound (1)-1

In a 200-ml three-neck flask purged with an inert gas, 0.023 mol of bis(4-aminophenyl) ether and 0.004 mol of compound (1)-1 were placed, and 110 ml of N-methyl-2-pyrrolidone were added to dissolve the compound. While stirring this reaction solution at room temperature, 0.025 mol of 4,4'-(2,2-hexafluoroisopropylidene)diphthalic dianhydride in the form of a solid were added thereto, and the reaction solution was stirred for 2 hours at room temperature. Thereafter, 0.05 mol of acetic anhydride and 0.005 mol of pyridine were added to the reaction solution and stirred for 1 hour at room temperature, and subsequently heated to 60°C and stirred for 3 hours. A solution of polymide was thus obtained.
The obtained solution was dropped in 300 ml of acetonitrile, and a deposition precipitated therein was collected by filtering and dried. A powder of polyimide having compound (1)-1 at both terminals thereof was thus obtained. 10 g of this powder were dissolved in 50 ml of N-methyl-2-pyrrolidone, thereby obtaining a solution of polyimide having compound (1)-1 at both terminals thereof.

This solution was applied on a silica glass plate with a blade, dried and heated to cure at 300°C. Thereafter, the tension modulus of elasticity and the glass transition temperatuire of the polyimide film formed on the silica glass substrate were measured. The measurement of the tension modulus of elasticity was carried out by using a tensile tester (STROGRAPH V1-C, trade name, manufactuted by Toyo Seiki Seisaku-Sho, Ltd.). The mechanical properties at 23°C were measured in accordance with a method of ASTM D822. The results are shown in Table 2.

### (Example 19) Preparation of polyimide composition containing compound (1)-2

A film of a polyimide having compound (1)-2 at terminals thereof was obtained in a similar manner to the prearation of Example 18, except that compound (1)-2 was used instead of compound (1)-1 in Example 18. The tensile strength and the modulus of elasticity of the obtained polyimide film were measured by the method similar to that in Example 18. The results are shown in Table 2.

### (Example 20) Preparation of polyimide composition containing compound (1)-14

A film of a polyimide having compound (1)-14 at terminals thereof was obtained in a similar manner to the prearation of Example 18, except that compound (1)-14 was used instead of compound (1)-1 in Example 18. The tensile strength and the modulus of elasticity of the obtained polyimide film were measured by the method similar to that in Example 18. The results are shown in Table 2.

### (Example 21) Preparation of polyimide composition containing compound (1)-1

A film of a polyimide having compound (1)-1 at terminals thereof was obtained in a similar manner to the prearation of Example 18, except that bis(3-aminophenyl) ether was used instead of bis(4-aminophenyl) ether in Example 18. The tensile strength and the modulus of elasticity of the obtained polyimide film were measured by the method similar to that in Example 18. The results are shown in Table 2.

### (Example 22) Preparation of polyurea composition containing compound (1)-1

In a 300-ml three-neck flask thoroughly purged with an inert gas, 0.023 mol of 3,4'-diaminodiphenyl ether and 0.004 mol of compound (1)-1 were placed. Then, 4,4'-diphenylmethanediisocyanate (0.023 mol) was dissolved in 30 ml of NMP, and this was dropped in the flask while stirring the solution. After allowing the solution to react at 130°C for 3 hours, this was dropped in water to allow a polymer to precipitate, and this polymer was dried. A powder of a polyurethane having compound (1)-1 at terminals thereof was thus obtained. 10 g of this powder were dissolved in 50 ml of N-methyl-2-pyrrolidone, and this solution was applied on a silica glass plate with a blade, dried and heated to cure at 300°C. The tensile strength and the tensile modulus of elasticity of the obtained polyurea film were measured by the method as described in Example 18. The results are shown in Table 2.

### (Example 23) Preparation of polyurethane composition containing compound (1)-1

A film of a polyurethane having compound (1)-1 at terminals thereof was obtained in a similar manner to the prearation of Example 22, except that 4,4'-diaminodiphenyl ether was used instead of 3,4'-diaminodiphenyl ether in Example 22. The tensile strength and the modulus of elasticity of the obtained polyurethane film were measured by the method similar to that in Example 18. The results are shown in Table 2.

### (Example 24) Preparation of polyamide composition containing compound (1)-1

A film of a polyamide having compound (1)-1 at tenninals thereof was obtained in a similar manner to the prearation of Example 18, except that 0.025 mol of 2,6-naphthalenedicarboxyloyl chloride was used instead of 0.025 mol of 4,4'-(2,2-hexafluoroisopropylidene)diphthalic dianhydride in Example 18. The tensile strength and the modulus of elasticity of the obtained polyurethane film were measured by the method similar to that in Example 18. The results are shown in Table 2.

### (Comparative Example 1) Preparation of polyimide composition containing 3-ethynylaniline

A solution of a polyimide having 3-ethynylaniline at terminals thereof was obtained in a similar manner to Example 18, except that 3-ethynylaniline was used instead of compound (1)-1. The tensile modulus of elasticity and the glass transision temperature were measured by the method as described in Example 18. The tensile strength and the modulus of elasticity were measured by the method as described in Example 18. The results are shown in Table 2.

### (Comparative Example 2) Preparation of polyimide composition without any ethynyl group at a terminal

A film of polymer was obtained in a similar manner to the prearation of Example 18, except that the compound (1)-1 in Example 18 was not used. The measurement results of tensile strength and modulus of elasticity of the obtained polymer film are shown in Table 2.

### (Comparative Example 3) Preparation of polyurea composition containing 3-ethynylaniline group at a terminal

A film of polymer was obtained in a similar manner to the prearation of Example 22, except that 3-ethynylaniline was used instead of compound (1)-1 in Example 22. The measurement results of tensile strength and modulus of elasticity of the obtained polymer film are shown in Table 2.

### (Comparative Example 4) Preparation of polyurethane composition containing 4-ethynylaniline group at a terminal

A film of polymer was obtained in a similar manner to the prearation of Example 23, except that 4-ethynylaniline was used instead of compound (1)-1 in Example 23. The measurement results of tensile strength and modulus of elasticity of the obtained polymer film are shown in Table 2.

### (Comparative Example 5) Preparation of polyamide composition containing 3-ethynylaniline group at a terminal

A film of polymer was obtained in a similar manner to the prearation of Example 24, except that 3-ethynylaniline was used instead of compound (1)-1 in Example 24. The measurement results of tensile strength and modulus of elasticity of the obtained polymer film are shown in Table 2.

**Table 2**

| Polymer | Tensile Strength [MPa] | Modulus of Elasticity |
|---|---|---|
| Example 18 | 139.1 | 3.9 |
| Example 19 | 150.3 | 4.2 |
| Example 20 | 140.2 | 3.9 |
| Example 21 | 137.2 | 3.7 |
| Example 22 | 139.1 | 1.5 |
| Example 23 | 80.5 | 1.6 |
| Example 24 | 108.2 | 2.9 |
| Comparative Example 1 | 122.2 | 3 |
| Comparative Example 2 | 112.5 | 2.1 |
| Comparative Example 3 | 132.5 | 1.3 |
| Comparative Example 4 | 75.1 | 1.4 |
| Comparative Example 5 | 99.7 | 2.4 |

As is clear from Tables 1 and 2, the films formed from a polymer in which the acetylene compound obtained by the invention is introduced exhibit superior properties, i.e., higher tensile strength and modulus of elasticity as compared with the films formed from a polymer in which a conventionally known acetylene compound is introduced.

### Industrial Applicability

The acetylene compound provided by the invention makes it possible to produce a polymer or an oligomer which can be cured by heat and can exhibit improved mechanical strength, heat resistance or chemical resistance, by introducing the acetytlene compound to the polymer and curing the same.

The foregoing description of the exemplary embodiments of the present invention has been provided for the purposes of illustration and description. It is not intended to be exhaustive or to limit the invention to the precise forms disclosed. The embodiments were chosen and described in order to best explain the principles of the invention and its practical applications, thereby enabling others skilled in the art to understand the invention for various embodiments and with the various modifications as are suited to the particular use contemplated.

All publications, patent applications, and technical standards mentioned in this specification are herein incorporated by reference to the same extent as if such individual publication, patent application, or technical standard was specifically and individually indicated to be incorporated by reference. It will be obvious to those having skill in the art that many changes may be made in the above-described details of the preferred embodiments of the present invention. It is intended that the scope of the invention be defined by the following claims and their equivalents.

## Claims

1. An acetylene compound represented by the following Formula (1) and a salt thereof: 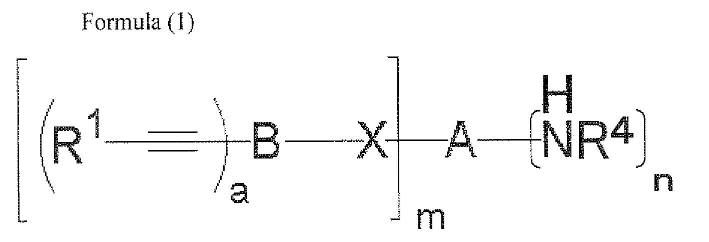 wherein in Formula (1), X represents a single bond or a divalent linking group; A represents a hydrocarbon group, a heteroaromatic ring or a heteroalicyclic compound; B represents a hydrocarbon group, a heteroaromatic ring, a heteroalicyclic compound or a single bond; R¹ represents a hydrogen atom, a hydrocarbon group, a heteroaromatic ring, a heteroalicyclic compound or a silyl group; R4 represents a hydrogen atom or a group that can be a substituent of the amino group; m, n and a each independently represent an integer of 1 or greater; and m and n are not 1 at the same time.

2. The acetylene compound and the salt thereof according to claim 1, wherein in Formula (1), X is selected from the group consisting of -OCO-, -NRCO-, -NRCONR'-, -NRCOO-, -OCONR-, -OCOO-, -OCS-, -NRCS-, -NRCSNR'-, -OCSO-, -S-, -O-, -SO-, -SO₂-, -NR-, -CO-, -CS- and a single bond; R and R' each represent a hydrogen atom, a hydrocarbon group or a heterocyclic group; and R⁴ is a hydrogen atom.

3. The acetylene compound and the salt thereof according to claim 2, wherein in Formula (1), n is 1; m is an integer of 2 or greater; R¹ represents one selected from the group consisting of a hydrogen atom, a hydrocarbon group, a heterocyclic group, a heteroalicyclic compound group and a silyl group.

4. The acetylene compound and the salt thereof according to claims 3, wherein in Formula (1), A and B each independently represent an aryl group.

5. The acetylene compound and the salt thereof according to claim 4, wherein in Formula (1), X is -OCO- or -NHCO-.

6. The acetylene compound and the salt thereof according to claim 5, wherein in Formula (1), m is 2.

7. The acetylene compound and the salt thereof according to claim 2, wherein in Formula (1), n represents an integer of 2 or greater; m is 1; and R¹ represents one selected from the group consisting of a hydrogen atom, an alkyl group, an alkenyl group, a heterocyclic group and an alkylsilyl group.

8. The acetylene compound and the salt thereof according to claim 7, wherein in Formula (1), A and B each independently represent an aryl group or a heteroaryl group.

9. The acetylene compound and the salt thereof according to claim 8, wherein in Formula (1), X is -OCO- or -NHCO-.

10. The acetylene compound and the salt thereof according to claim 9, wherein in Formula (1), n is 2.

11. A condensate obtained by condensing at least one acetylene compound according to any one of claims 1 to 10.

12. A composition at least including the acetylene compound according to any one of claims 1 to 10, and/or the condensate according to claim 11.
